# EUROPEAN PATENT SPECIFICATION

(11) **EP 1 032 668 B1**
(45) Date of publication and mention of the grant of the patent: **28.05.2008**
(21) Application number: 98961795.6
(22) Date of filing: 25.11.1998
(51) Int. Cl.: C12N 15/12, C07K 14/50, C12N 15/62, C07K 16/22

(54) **FIBROBLAST GROWTH FACTOR-19**
FIBROBLASTEN-WACHSTUMSFAKTOR-19
FACTEUR DE CROISSANCE DES FIBROBLASTES 19

(30) Priority: 25.11.1997 US 66840 P; 21.09.1998 US 158342
(43) Date of publication of application: 06.09.2000
(73) Proprietor: GENENTECH, INC., South San Francisco, CA 94080-4990 (US)
(72) Inventor: BOTSTEIN, David, Belmont, CA 94402 (US); GODDARD, Audrey, San Francisco, CA 94131 (US); GURNEY, Austin, L., Belmont, CA 94002 (US); HILLAN, Kenneth, J., San Francisco, CA 94114 (US); LAWRENCE, David, A., San Francisco, CA 94122 (US); ROY, Margaret, Ann, San Francisco, CA 94123 (US)
(74) Representative: Kiddle, Simon John
(86) International application number: PCT/US1998/025190
(87) International publication number: WO 1999/027100

(56) References cited:
- WO-A2-00/15666
- WO-A2-00/15796
- WO-A2-99/14327
- WO-A2-99/14328
- DATABASE EMBL - EMEST11 Entry Hs1139632, Acc.no. AA220994, 14 February 1997 HILLIER, L. ET A.: "zr01g05.r1 Stratagene NT2 neuronal precursor 937230 Homo sapiens cDNA clone 650264 5'." XP002099436
- NISHIMURA, T. ET AL: "Structure and expression of a novel human FGF, FGF-19, expressed in fetal brain." BIOCHIMICA ET BIOPHYSICA ACTA, vol. 1444, 18 January 1999, pages 148-51, XP002099435
- DATABASE EMBL - EMGSS Entry Hsb7673, Acc.no. B03767, 16 July 1996 EVANS, G.A. ET AL.: "cSRL-186H12-u cSRL flow sorted Chromosome 11 specific cosmid Homo sapiens genomic clone cSRL-186H12." XP002099437
- DATABASE EMBL - EMEST6 Entry/Acc.no. Ai076490, 10 August 1998 STRAUSBERG, R.: "oz28c05.x1 Soares_total_fetus_Nb2HF8_9w Homo sapiens cDNA clone IMAGE:1676648 3', mRNA sequence." XP002099438
- MING-HONG XIE ET AL: 'FGF-19, a novel fibroblast growth factor with unique specificity for FGFR4' CYTOKINE vol. 11, no. 10, October 1999, pages 729 - 735
- HARMER N.J. ET AL: 'the crystal structure of fibroblast growth factor (FGF) 19 reveals novel features of the FGF family and offers a structural basis for its unusual receptor affinity' BIOCHEMISTRY vol. 43, 2004, pages 629 - 640

### Sequence Listing

<110> Genentech Inc.
   Botstein, David A.
   Goddard, Audrey
   Gurney, Austin L.
   Hillan, Kenneth J.
   Lawrence, David A.
   Roy, Margaret Ann
<120> Fibroblast Growth Factor-19
<130> P1219 R1
<140> PCT/US98/25190
   <141> 1998-11-25
<150> US 60/066,840
   <151> 1997-11-25
<150> US 09/158,342
   <151> 1998-09-21
<160> 24
<210> 1
   <211> 216
   <212> PRT
   <213> Homo sapiens
<400> 2
<210> 2
   <211> 2137
   <212> DNA
   <213> Homo sapiens
<400> 2
   gctcccagcc aagaacctcg gggccgctgc gcggtgggga ggagttcccc 50
   gaaacccggc cgctaagcga ggcctcctcc tcccgcagat ccgaacggcc 100
   tgggcggggt caccccggct gggacaagaa gccgccgcct gcctgcccgg 150
   gcccggggag ggggctgggg ctggggccgg aggcggggtg tgagtgggtg 200
   tgtgcggggg gcggaggctt gatgcaatcc cgataagaaa tgctcgggtg 250
   tcttgggcac ctacccgtgg ggcccgtaag gcgctactat ataaggctgc 300
   cggcccggag ccgccgcgcc gtcagagcag gagcgctgcg tccaggatct 350
   agggccacga ccatcccaac ccggcactca cagccccgca gcgcatcccg 400
   gtcgccgccc agcctcccgc acccccatcg ccggagctgc gccgagagcc 450
   ccagggaggt gccatgcgga gcgggtgtgt ggtggtccac gtatggatcc 500
   tggccggcct ctggctggcc gtggccgggc gccccctcgc cttctcggac 550
   gcggggcccc acgtgcacta cggctggggc gaccccatcc gcctgcggca 600
   cctgtacacc tccggccccc acgggctctc cagctgcttc ctgcgcatcc 650
   gtgccgacgg cgtcgtggac tgcgcgcggg gccagagcgc gcacagtttg 700
   ctggagatca aggcagtcgc tctgcggacc gtggccatca agggcgtgca 750
   cagcgtgcgg tacctctgca tgggcgccga cggcaagatg caggggctgc 800
   ttcagtactc ggaggaagac tgtgctttcg aggaggagat ccgcccagat 850
   ggctacaatg tgtaccgatc cgagaagcac cgcctcccgg tctccctgag 900
   cagtgccaaa cagcggcagc tgtacaagaa cagaggcttt cttccactct 950
   ctcatttcct gcccatgctg cccatggtcc cagaggagcc tgaggacctc 1000
   aggggccact tggaatctga catgttctct tcgcccctgg agaccgacag 1050
   catggaccca tttgggcttg tcaccggact ggaggccgtg aggagtccca 1100
   gctttgagaa gtaactgaga ccatgcccgg gcctcttcac tgctgccagg 1150
   ggctgtggta cctgcagcgt gggggacgtg cttctacaag aacagtcctg 1200
   agtccacgtt ctgtttagct ttaggaagaa acatctagaa gttgtacata 1250
   ttcagagttt tccattggca gtgccagttt ctagccaata gacttgtctg 1300
   atcataacat tgtaagcctg tagcttgccc agctgctgcc tgggccccca 1350
   ttctgctccc tcgaggttgc tggacaagct gctgcactgt ctcagttctg 1400
   cttgaatacc tccatcgatg gggaactcac ttcctttgga aaaattctta 1450
   tgtcaagctg aaattctcta attttttctc atcacttccc caggagcagc 1500
   cagaagacag gcagtagttt taatttcagg aacaggtgat ccactctgta 1550
   aaacagcagg taaatttcac tcaaccccat gtgggaattg atctatatct 1600
   ctacttccag ggaccatttg cccttcccaa atccctccag gccagaactg 1650
   actggagcag gcatggccca ccaggcttca ggagtagggg aagcctggag 1700
   ccccactcca gccctgggac aacttgagaa ttccccctga ggccagttct 1750
   gtcatggatg ctgtcctgag aataacttgc tgtcccggtg tcacctgctt 1800
   ccatctccca gcccaccagc cctctgccca cctcacatgc ctccccatgg 1850
   attggggcct cccaggcccc ccaccttatg tcaacctgca cttcttgttc 1900
   aaaaatcagg aaaagaaaag atttgaagac cccaagtctt gtcaataact 1950
   tgctgtgtgg aagcagcggg ggaagaccta gaaccctttc cccagcactt 2000
   ggttttccaa catgatattt atgagtaatt tattttgata tgtacatctc 2050
   ttattttctt acattattta tgcccccaaa ttatatttat gtatgtaagt 2100
   gaggtttgtt ttgtatatta aaatggagtt tgtttgt 2137
<210> 3
   <211> 214
   <212> PRT
   <213> Homo sapiens
<400> 3
   Ser Gly Cys Val Val Val His Val Trp Ile Leu Ala Gly Leu Trp
   1               5                   10                  15
   Leu Ala Val Ala Gly Arg Pro Leu Ala Phe Ser Asp Ala Gly Pro
                   20                  25                  30
   His Val His Tyr Gly Trp Gly Asp Pro Ile Arg Leu Arg His Leu
                   35                  40                  45
   Tyr Thr Ser Gly Pro His Gly Leu Ser Ser Cys Phe Leu Arg Ile
                   50                  55                  60
   Arg Ala Asp Gly Val Val Asp Cys Ala Arg Gly Gln Ser Ala His
                   65                  70                  75
   Ser Leu Leu Glu Ile Lys Ala Val Ala Leu Arg Thr Val Ala Ile
                   80                  85                  90
   Lys Gly Val His Ser Val Arg Tyr Leu Cys Met Gly Ala Asp Gly
                   95                  100                 105
   Lys Met Gln Gly Leu Leu Gln Tyr Ser Glu Glu Asp Cys Ala Phe
                   110                 115                 120
   Glu Glu Glu Ile Arg Pro Asp Gly Tyr Asn Val Tyr Arg Ser Glu
                   125                 130                 135
   Lys His Arg Leu Pro Val Ser Leu Ser Ser Ala Lys Gln Arg Gln
                   140                 145                 150
   Leu Tyr Lys Asn Arg Gly Phe Leu Pro Leu Ser His Phe Leu Pro
                   155                 160                 165
   Met Leu Pro Met Val Pro Glu Glu Pro Glu Asp Leu Arg Gly His
                   170                 175                 180
   Leu Glu Ser Asp Met Phe Ser Ser Pro Leu Glu Thr Asp Ser Met
                   185                 190                 195
   Asp Pro Phe Gly Leu Val Thr Gly Leu Glu Ala Val Arg Ser Pro
                   200                 205                 210
   Ser Phe Glu Lys
               214
<210> 4
   <211> 213
   <212> PRT
   <213> Mus Musculus
<400> 4
   Asn Gly Arg Ala Val Ala Arg Ala Leu Val Leu Ala Thr Leu Trp
    1               5                  10                  15
   Leu Ala Val Ser Gly Arg Pro Leu Ala Gln Gln Ser Gln Ser Val
                   20                  25                  30
   Ser Asp Glu Asp Pro Leu Phe Leu Tyr Gly Trp Gly Lys Ile Thr
                   35                  40                  45
   Arg Leu Gln Tyr Leu Tyr Ser Ala Gly Pro Tyr Val Ser Asn Cys
                   50                  55                  60
   Phe Leu Arg Ile Arg Ser Asp Gly Ser Val Asp Cys Glu Glu Asp
                   65                  70                  75
   Gln Asn Glu Arg Asn Leu Leu Glu Phe Arg Ala Val Ala Leu Lys
                   80                  85                  90
   Thr Ile Ala Ile Lys Asp Val Ser Ser Val Arg Tyr Leu Cys Met
                   95                 100                 105
   Ser Ala Asp Gly Lys Ile Tyr Gly Leu Ile Arg Tyr Ser Glu Glu
                  110                 115                 120
   Asp Cys Thr Phe Arg Glu Glu Met Asp Cys Leu Gly Tyr Asn Gln
                  125                 130                 135
   Tyr Arg Ser Met Lys His His Leu His Ile Ile Phe Ile Gln Ala
                  140                 145                 150
   Lys Pro Arg Glu Gln Leu Gln Asp Gln Lys Pro Ser Asn Phe Ile
                  155                 160                 165
   Pro Val Phe His Arg Ser Phe Phe Glu Thr Gly Asp Gln Leu Arg
                  170                 175                 180
   Ser Lys Met Phe Ser Leu Pro Leu Glu Ser Asp Ser Met Asp Pro
                  185                 190                 195
   Phe Arg Met Val Glu Asp Val Asp His Leu Val Lys Ser Pro Ser
                  200                 205                 210
   Phe Gln Lys
           213
<210> 5
   <211> 752
   <212> DNA
   <213> Homo sapiens
<220>
   <221> unknown
   <222> 11, 14, 50, 403, 429, 442, 445, 466, 474, 487, 518, 525, 531, 540, 546, 561, 566, 572, 580, 582, 587, 637, 656, 671, 680, 690, 695, 722, 737, 740, 750
   <223> unknown base
<400> 5
   aaatctgccg nttngaaatg gcactgccag tcgaaaactc tgaatatgtn 50
   caacttctag atgtttcttc ctaaagctaa acagaacgtg gactcaggac 100
   tgttcttgta gaagcacgtc ccccacgctg caggtaccac agcccctggc 150
   agcagtgaag aggcccgggc atggtctcag ttacttctca aagctgggac 200
   tcctcacggc ctccagtccg gtgacaagcc caaatgggtc catgctgtcg 250
   gtctccaggg gcgaagagaa catgtcagat tccaagtggc ccctgaggtc 300
   ctcaggctcc tctgggacca tgggcagcat gggcaggaaa tgagagagtg 350
   gaagaaagcc tctgttcttg tacagctgcc gctgtttggc actgctcagg 400
   ganaccggga agcggtgctt ctcggatcng tacacattgt anccntctgg 450
   gcggatctcc tcctcnaaac acantctccc ccgagtnctg aaactgcaaa 500
   aaaaaaggca ctctgacnat ccccnttgga naggaaaacn ggtccncatg 550
   aactttgtct ncaaantgtc cnggtggccn cnacatnaac tcccataaac 600
   ccccttgcat ttttaaacat actcctgatg tcacccnttc ttttaattat 650
   aaaaanaacc taaatattta nttttaaaan aaatttcttn agggncgcca 700
   ttggtcccct ttttccccct tnggagccaa caatgtnccn gaaccgattn 750
   aa 752
<210> 6
   <211> 371
   <212> DNA
   <213> Homo sapiens
<400> 6
   ttcgaggagg agatccgccc agatggctac aatgtgtacc gatccgagaa 50
   gcaccgcctc ccggtctccc tgagcagtgc caaacagcgg cagtgtacaa 100
   gaacagaggc tttcttccac tctctcattt cctgcccatg ctgcccatgg 150
   tcccagagga gcctgaggac ctcaggggcc acttggaatc tgacatgttc 200
   tcttcgcccc tggagaccga cagcatggac ccatttgggc ttgtcaccgg 250
   actggaggcc gtgaggagtc ccagctttga gaagtaactg agaccatgcc 300
   cgggcctctt cactgctgcc aggggtgtgg tacctgcagc gtgggggacg 350
   tgcttctaca agaacagtcc t 371
<210> 7
   <211> 1824
   <212> DNA
   <213> Mus Musculus
<400> 7
   ctgtcggagc agtaactctg tgcgccccac gccacaagcg cccagttgct 50
   ttgtgggttg tgcctgccct gcgcctgcaa cttgagtccc cgccgcatcg 100
   cagtctccgc gccacctttg taacggcctt caggaccccg aggtgtcatg 150
   gcgagaaagt ggaacgggcg tgcggtggcc cgagccctgg tcctggccac 200
   tctgtggctg gctgtgtctg ggcgtcccct ggcccagcaa tcccagtctg 250
   tgtcagatga agatccactc tttctctacg gctggggcaa gattacccgc 300
   ctgcagtacc tgtactccgc tggtccctat gtctccaact gcttcctccg 350
   aatccggagc gacggctctg tggactgcga ggaggaccaa aacgaacgaa 400
   atttgttgga attccgcgcg gtcgctctga agacgattgc catcaaggac 450
   gtcagcagcg tgcggtacct ctgcatgagc gcggacggca agatatacgg 500
   gctgattcgc tactcggagg aagactgtac cttcagggag gaaatggact 550
   gtttaggcta caaccagtac agatccatga agcaccatct ccatatcatc 600
   ttcatccagg ccaagcccag agaacagctc caggaccaga aaccctcaaa 650
   ctttatcccc gtgtttcacc gctccttctt tgaaaccggg gaccagctga 700
   ggtctaaaat gttctccctg cccctggaga gtgacagcat ggatccgttc 750
   aggatggtgg aggatgtaga ccacctagtg aagagtccca gcttccagaa 800
   atgacaggat tccgacagga tggagaaaac cccaaggtcc cgtgaacttc 850
   ccccttagga agctgtagat attctaagtc tcacatggac cctgttgtgt 900
   tagtggctag acttgatcat gaacctaagt tgacaacctg cctggctgcc 950
   atcggagccc cactgacttt ggaggctgct gatatgtgcc taagttactc 1000
   cagttctgtt tgaatacctc cactaatagg gaacttactc ctgtgaaaca 1050
   ttcttagttt tgagccaaat ctgtgacttg gatggtttta gcgagcaagc 1100
   cagaaggtat gaagtcaaat gataaaattc atgtatagaa agtgggctct 1150
   aaaatatata ttccctatat ggatctcatg ggatcttagc ttgcccccca 1200
   aatgtctcct ggccagaact aactggggtt acaaacttgg aacaaaggac 1250
   agcctagaaa actttgggag ccttgaagga tggtcttagg attacgaatt 1300
   ccagctgact acgtagcttc ccccttttcc acttataaat gtcagatgga 1350
   agtgaccctt agctgagtgc atagccaagc tgccacttat gccccaggag 1400
   cttgtctctg tcccatgacc ccagatttcc aggacctgga tctgctcctc 1450
   tgacctttcc cagagttcac ctgggctctc caaccccaga gcaggtagct 1500
   tatgagccat ccagttgtgt ccccagctcc tggctctcag ttctggtcac 1550
   caaacattgt gaatcaacgt gtctgctgcc tgtgtcaacc tggatccctc 1600
   atttacaaac aagattagga agccccaaat tctccagtgg cagctgggga 1650
   actgtggagt cctttccccg gcacttacgt ggcagcatga tatttataag 1700
   taatttattg tgtgtgtgtc ttctattttc ttactttatt tatgccccag 1750
   atcatattta tgtacatgac ttgttttcta cattaaaaag gagttggttt 1800
   gtatcaaaaa aaaaaaaaaa aaaa 1824
<210> 8
   <211> 216
   <212> PRT
   <213> Homo sapiens
<400> 8
   Met Arg Ser Gly Cys Val Val Val His Val Trp Ile Leu Ala Gly
   1               5                   10                  15
   Leu Trp Leu Ala Val Ala Gly Arg Pro Leu Ala Phe Ser Asp Ala
                   20                  25                  30
   Gly Pro His Val His Tyr Gly Trp Gly Asp Pro Ile Arg Leu Arg
                   35                  40                  45
   His Leu Tyr Thr Ser Gly Pro His Gly Leu Ser Ser Cys Phe Leu
                   50                  55                  60
   Arg Ile Arg Ala Asp Gly Val Val Asp Cys Ala Arg Gly Gln Ser
                   65                  70                  75
   Ala His Ser Leu Leu Glu Ile Lys Ala Val Ala Leu Arg Thr Val
                   80                  85                  90
   Ala Ile Lys Gly Val His Ser Val Arg Tyr Leu Cys Met Gly Ala
                   95                  100                 105
   Asp Gly Lys Met Gln Gly Leu Leu Gln Tyr Ser Glu Glu Asp Cys
                   110                 115                 120
   Ala Phe Glu Glu Glu Ile Arg Pro Asp Gly Tyr Asn Val Tyr Arg
                   125                 130                 135
   Ser Glu Lys His Arg Leu Pro Val Ser Leu Ser Ser Ala Lys Gln
                   140                 145                 150
   Arg Gln Leu Tyr Lys Asn Arg Gly Phe Leu Pro Leu Ser His Phe
                   155                 160                 165
   Leu Pro Met Leu Pro Met Val Pro Glu Glu Pro Glu Asp Leu Arg
                   170                 175                 180
   Gly His Leu Glu Ser Asp Met Phe Ser Ser Pro Leu Glu Thr Asp
                   185                 190                 195
   Ser Met Asp Pro Phe Gly Leu Val Thr Gly Leu Glu Ala Val Arg
                   200                 205                 210
   Ser Pro Ser Phe Glu Lys
                   215 216
<210> 9
   <211> 155
   <212> PRT
   <213> Homo sapiens
<400> 9
   Met Ala Glu Gly Glu Ile Thr Thr Phe Thr Ala Leu Thr Glu Lys
   1               5                   10                  15
   Phe Asn Leu Pro Pro Gly Asn Tyr Lys Lys Pro Lys Leu Leu Tyr
                   20                  25                  30
   Cys Ser Asn Gly Gly His Phe Leu Arg Ile Leu Pro Asp Gly Thr
                   35                  40                  45
   Val Asp Gly Thr Arg Asp Arg Ser Asp Gln His Ile Gln Leu Gln
                   50                  55                  60
   Leu Ser Ala Glu Ser Val Gly Glu Val Tyr Ile Lys Ser Thr Glu
                   65                  70                  75
   Thr Gly Gln Tyr Leu Ala Met Asp Thr Asp Gly Leu Leu Tyr Gly
                   80                  85                  90
   Ser Gln Thr Pro Asn Glu Glu Cys Leu Phe Leu Glu Arg Leu Glu
                   95                  100                 105
   Glu Asn His Tyr Asn Thr Tyr Ile Ser Lys Lys His Ala Glu Lys
                   110                 115                 120
   Asn Trp Phe Val Gly Leu Lys Lys Asn Gly Ser Cys Lys Arg Gly
                   125                 130                 135
   Pro Arg Thr His Tyr Gly Gln Lys Ala Ile Leu Phe Leu Pro Leu
                   140                 145                 150
   Pro Val Ser Ser Asp
                   155
<210> 10
   <211> 268
   <212> PRT
   <213> Homo sapiens
<400> 10
   Met Ser Leu Ser Phe Leu Leu Leu Leu Phe Phe Ser His Leu Ile
   1               5                   10                  15
   Leu Ser Ala Trp Ala His Gly Glu Lys Arg Leu Ala Pro Lys Gly
                   20                  25                  30
   Gln Pro Gly Pro Ala Ala Thr Asp Arg Asn Pro Ile Gly Ser Ser
                   35                  40                  45
   Ser Arg Gln Ser Ser Ser Ser Ala Met Ser Ser Ser Ser Ala Ser
   50 55 60
   Ser Ser Pro Ala Ala Ser Leu Gly Ser Gln Gly Ser Gly Leu Glu
   65 70 75
   Gln Ser Ser Phe Gln Trp Ser Pro Ser Gly Arg Arg Thr Gly Ser
   80 85 90
   Leu Tyr Cys Arg Val Gly Ile Gly Phe His Leu Gln Ile Tyr Pro
   95 100 105
   Asp Gly Lys Val Asn Gly Ser His Glu Ala Asn Met Leu Ser Val
   110 115 120
   Leu Glu Ile Phe Ala Val Ser Gln Gly Ile Val Gly Ile Arg Gly
   125 130 135
   Val Phe Ser Asn Lys Phe Leu Ala Met Ser Lys Lys Gly Lys Leu
   140 145 150
   His Ala Ser Ala Lys Phe Thr Asp Asp Cys Lys Phe Arg Glu Arg
   155 160 165
   Phe Gln Glu Asn Ser Tyr Asn Thr Tyr Ala Ser Ala Ile His Arg
   170 175 180
   Thr Glu Lys Thr Gly Arg Glu Trp Tyr Val Ala Leu Asn Lys Arg
   185 190 195
   Gly Lys Ala Lys Arg Gly Cys Ser Pro Arg Val Lys Pro Gln His
   200 205 210
   Ile Ser Thr His Phe Leu Pro Arg Phe Lys Gln Ser Glu Gln Pro
   215 220 225
   Glu Leu Ser Phe Thr Val Thr Val Pro Glu Lys Lys Asn Pro Pro
   230 235 240
   Ser Pro Ile Lys Ser Lys Ile Pro Leu Ser Ala Pro Arg Lys Asn
   245 250 255
   Thr Asn Ser Val Lys Tyr Arg Leu Lys Phe Arg Phe Gly
   260 265 268
<210> 11
   <211> 194
   <212> PRT
   <213> Homo sapiens
<400> 11
   Met His Lys Trp Ile Leu Thr Trp Ile Leu Pro Thr Leu Leu Tyr
   1 5 10 15
   Arg Ser Cys Phe His Ile Ile Cys Leu Val Gly Thr Ile Ser Leu
   20 25 30
   Ala Cys Asn Asp Met Thr Pro Glu Gln Met Ala Thr Asn Val Asn
   35 40 45
   Cys Ser Ser Pro Glu Arg His Thr Arg Ser Tyr Asp Tyr Met Glu
   50 55 60
   Gly Gly Asp Ile Arg Val Arg Arg Leu Phe Cys Arg Thr Gln Trp
   65 70 75
   Tyr Leu Arg Ile Asp Lys Arg Gly Lys Val Lys Gly Thr Gln Glu
   80 85 90
   Met Lys Asn Asn Tyr Asn Ile Met Glu Ile Arg Thr Val Ala Val
   95 100 105
   Gly Ile Val Ala Ile Lys Gly Val Glu Ser Glu Phe Tyr Leu Ala
   110 115 120
   Met Asn Lys Glu Gly Lys Leu Tyr Ala Lys Lys Glu Cys Asn Glu
   125 130 135
   Asp Cys Asn Phe Lys Glu Leu Ile Leu Glu Asn His Tyr Asn Thr
   140 145 150
   Tyr Ala Ser Ala Lys Trp Thr His Asn Gly Gly Glu Met Phe Val
   155 160 165
   Ala Leu Asn Gln Lys Gly Ile Pro Val Arg Gly Lys Lys Thr Lys
   170 175 180
   Lys Glu Gln Lys Thr Ala His Phe Leu Pro Met Ala Ile Thr
   185 190 194
<210> 12
   <211> 231
   <212> PRT
   <213> Homo sapiens
<400> 12
   Met Gly Ser Pro Arg Ser Ala Leu Ser Cys Leu Leu Leu His Leu
   1               5                   10                  15
   Leu Val Leu Cys Leu Gln Ala Gln Glu Gly Pro Gly Arg Gly Pro
                   20                  25                  30
   Ala Leu Gly Arg Glu Leu Ala Ser Leu Phe Arg Ala Gly Arg Glu
                   35                  40                  45
   Pro Gln Gly Val Ser Gln Gln His Val Arg Glu Gln Ser Leu Val
                   50                  55                  60
   Thr Asp Gln Leu Ser Arg Arg Leu Ile Arg Thr Tyr Gln Leu Tyr
                   65                  70                  75
   Ser Arg Thr Ser Gly Lys His Val Gln Val Leu Ala Asn Lys Arg
                   80                  85                  90
   Ile Asn Met Ala Glu Asp Gly Asp Pro Phe Ala Lys Leu Ile Val
                   95                  100                 105
   Glu Thr Asp Thr Phe Gly Ser Arg Val Arg Val Arg Gly Ala Glu
                   110                 115                 120
   Thr Gly Leu Tyr Ile Cys Met Asn Lys Lys Gly Lys Leu Ile Ala
                   125                 130                 135
   Lys Ser Asn Gly Lys Gly Lys Asp Cys Val Phe Thr Glu Ile Val
                   140                 145                 150
   Leu Glu Asn Asn Tyr Thr Ala Leu Gln Asn Ala Lys Tyr Glu Gly
                   155                 160                 165
   Trp Tyr Met Ala Phe Thr Arg Lys Gly Arg Pro Arg Lys Gly Ser
                   170                 175                 180
   Lys Thr Arg Gln His Gln Arg Glu Val His Phe Met Lys Arg Leu
                   185                 190                 195
   Pro Arg Gly His His Thr Thr Glu Gln Ser Leu Phe Glu Phe Leu
                   200                 205                 210
   Asn Tyr Pro Pro Phe Thr Arg Ser Leu Arg Gly Ser Gln Arg Thr
                   215                 220                 225
   Trp Ala Pro Glu Pro Arg
                   230 231
<210> 13
   <211> 225
   <212> PRT
   <213> Homo sapiens
<400> 13
   Met Ala Ala Leu Ala Ser Ser Leu Ile Arg Gln Lys Arg Glu Val
   1               5                   10                  15
   Arg Glu Pro Gly Gly Ser Arg Pro Val Ser Ala Gln Arg Arg Val
                   20                  25                  30
   Cys Pro Arg Gly Thr Lys Ser Leu Cys Gln Lys Gln Leu Leu Ile
                   35                  40                  45
   Leu Leu Ser Lys Val Arg Leu Cys Gly Gly Arg Pro Ala Arg Pro
                   50                  55                  60
   Asp Arg Gly Pro Glu Pro Gln Leu Lys Gly Ile Val Thr Lys Leu
                   65                  70                  75
   Phe Cys Arg Gln Gly Phe Tyr Leu Gln Ala Asn Pro Asp Gly Ser
                   80                  85                  90
   Ile Gln Gly Thr Pro Glu Asp Thr Ser Ser Phe Thr His Phe Asn
                   95                  100                 105
   Leu Ile Pro Val Gly Leu Arg Val Val Thr Ile Gln Ser Ala Lys
                   110                 115                 120
   Leu Gly His Tyr Met Ala Met Asn Ala Glu Gly Leu Leu Tyr Ser
                   125                 130                 135
   Ser Pro His Phe Thr Ala Glu Cys Arg Phe Lys Glu Cys Val Phe
                   140                 145                 150
   Glu Asn Tyr Tyr Val Leu Tyr Ala Ser Ala Leu Tyr Arg Gln Arg
                   155                 160                 165
   Arg Ser Gly Arg Ala Trp Tyr Leu Gly Leu Asp Lys Glu Gly Gln
                   170                 175                 180
   Val Met Lys Gly Asn Arg Val Lys Lys Thr Lys Ala Ala Ala His
                   185                 190                 195
   Phe Leu Pro Lys Leu Leu Glu Val Ala Met Tyr Gln Glu Pro Ser
                   200                 205                 210
   Leu His Ser Val Pro Glu Ala Ser Pro Ser Ser Pro Pro Ala Pro
                   215                 220                 225
<210> 14
   <211> 252
   <212> PRT
   <213> Homo sapiens
<400> 14
   Met Val Lys Pro Val Pro Leu Phe Arg Arg Thr Asp Phe Lys Leu
    1               5                  10                  15
   Leu Leu Cys Asn His Lys Asp Leu Phe Phe Leu Arg Val Ser Lys
                   20                  25                  30
   Leu Leu Asp Cys Phe Ser Pro Lys Ser Met Trp Phe Leu Trp Asn
                   35                  40                  45
   Ile Phe Ser Lys Gly Thr His Met Leu Gln Cys Leu Cys Gly Lys
                   50                  55                  60
   Ser Leu Lys Lys Asn Lys Asn Pro Thr Asp Pro Gln Leu Lys Gly
                   65                  70                  75
   Ile Val Thr Arg Leu Tyr Cys Arg Gln Gly Tyr Tyr Leu Gln Met
                   80                  85                  90
   His Pro Asp Gly Ala Leu Asp Gly Thr Lys Gly Asp Ser Thr Asn
                   95                  100                 105
   Ser Thr Leu Phe Asn Leu Ile Pro Val Gly Leu Arg Val Val Ala
                   110                 115                 120
   Ile Gln Gly Val Lys Thr Gly Leu Tyr Ile Thr Met Asn Gly Glu
                   125                 130                 135
   Gly Tyr Leu Tyr Pro Ser Glu Leu Phe Thr Pro Glu Cys Lys Phe
                   140                 145                 150
   Lys Glu Ser Val Phe Glu Asn Tyr Tyr Val Ile Tyr Ser Ser Met
                   155                 160                 165
   Leu Tyr Arg Gln Gln Glu Ser Gly Arg Ala Trp Phe Leu Gly Leu
                   170                 175                 180
   Asn Lys Glu Gly Gln Ala Met Lys Gly Asn Arg Val Lys Lys Thr
                   185                 190                 195
   Lys Pro Ala Ala His Phe Leu Pro Lys Pro Leu Glu Val Ala Met
                   200                 205                 210
   Tyr Arg Glu Pro Ser Leu His Asp Val Gly Glu Thr Val Pro Lys
                   215                 220                 225
   Pro Gly Val Thr Pro Ser Lys Ser Thr Ser Ala Ser Ala Ile Met
                   230                 235                 240
   Asn Gly Gly Lys Pro Val Asn Lys Ser Lys Thr Thr
                   245                 250 252
<210> 15
   <211> 207
   <212> PRT
   <213> Homo sapiens
<400> 15
   Met Tyr Ser Ala Pro Ser Ala Cys Thr Cys Leu Cys Leu His Phe
   1               5                   10                  15
   Leu Leu Leu Cys Phe Gln Val Gln Val Leu Val Ala Glu Glu Asn
                   20                  25                  30
   Val Asp Phe Arg Ile His Val Glu Asn Gln Thr Arg Ala Arg Asp
                   35                  40                  45
   Asp Val Ser Arg Lys Gln Leu Arg Leu Tyr Gln Leu Tyr Ser Arg
                   50                  55                  60
   Thr Ser Gly Lys His Ile Gln Val Leu Gly Arg Arg Ile Ser Ala
                   65                  70                  75
   Arg Gly Glu Asp Gly Asp Lys Tyr Ala Gln Leu Leu Val Glu Thr
                   80                  85                  90
   Asp Thr Phe Gly Ser Gln Val Arg Ile Lys Gly Lys Glu Thr Glu
                   95                  100                 105
   Phe Tyr Leu Cys Met Asn Arg Lys Gly Lys Leu Val Gly Lys Pro
                   110                 115                 120
   Asp Gly Thr Ser Lys Glu Cys Val Phe Ile Glu Lys Val Leu Glu
                   125                 130                 135
   Asn Asn Tyr Thr Ala Leu Met Ser Ala Lys Tyr Ser Gly Trp Tyr
                   140                 145                 150
   Val Gly Phe Thr Lys Lys Gly Arg Pro Arg Lys Gly Pro Lys Thr
                   155                 160                 165
   Arg Glu Asn Gln Gln Asp Val His Phe Met Lys Arg Tyr Pro Lys
                   170                 175                 180
   Gly Gln Pro Glu Leu Gln Lys Pro Phe Lys Tyr Thr Thr Val Thr
                   185                 190                 195
   Lys Arg Ser Arg Arg Ile Arg Pro Thr His Pro Ala
                   200                 205     207
<210> 16
   <211> 26
   <212> DNA
   <213> Artificial
<220>
   <223> Artificial sequence
<400> 16
   atccgcccag atggctacaa tgtgta 26
<210> 17
   <211> 40
   <212> DNA
   <213> Artificial
<220>
   <223> Artificial sequence
<400> 17
   agaccgggag gcggtgcttc tcggatcggt acacattgta 40
<210> 18
   <211> 22
   <212> DNA
   <213> Artificial
<220>
   <223> Artificial sequence
<400> 18
   ccagtccggt gacaagccca aa 22
<210> 19
   <211> 46
   <212> DNA
   <213> Artificial
<220>
   <223> Artificial sequence
<400> 19
   ggattctaat acgactcact atagggcgga tcctggccgg cctcgg 46
<210> 20
   <211> 48
   <212> DNA
   <213> Artificial
<220>
   <223> Artificial sequence
<400> 20
   ctatgaaatt aaccctcact aaagggagcc cgggcatggt ctcagtta 48
<210> 21
   <211> 23
   <212> DNA
   <213> Artificial
<220>
   <223> Artificial sequence
<400> 21
   gggacgtgct tctacaagaa cag 23
<210> 22
   <211> 26
   <212> DNA
   <213> Artificial
<220>
   <223> Artificial sequence
<400> 22
   caggcttaca atgttatgat cagaca 26
<210> 23
   <211> 31
   <212> DNA
   <213> Artificial
<220>
   <223> Artificial sequence
<400> 23
   tattcagagt tttccattgg cagtgccagt t 31
<210> 24
   <211> 66
   <212> PRT
   <213> Artificial
<220>
   <223> Artificial sequence
<400> 24
   Met Gly Gly Ala Ala Ala Arg Leu Gly Ala Val Ile Leu Phe Val
    1               5                  10                  15
   Val Ile Val Gly Leu His Gly Val Arg Gly Lys Tyr Ala Leu Ala
                   20                  25                  30
   Asp Ala Ser Leu Lys Met Ala Asp Pro Asn Arg Phe Arg Gly Lys
                   35                  40                  45
   Asp Leu Pro Val Leu Asp Gln Leu Leu Glu Gly Gly Ala Ala His
                   50                  55                  60
   Tyr Ala Leu Leu Pro Gly
                   65  66

### Sequence Listing

<110> Genentech Inc.
   Botstein, David A.
   Goddard, Audrey
   Gurney, Austin L,
   Hillan, Kenneth J.
   Lawrence, David A.
   Roy, Margaret Ann
<120> Fibroblast Growth Factor-19
<130> P1219 R1
<140> PCT/US98/25190
   <141> 1998-11-25
<150> US 60/066,840
   <151> 1997-11-25
<150> US 09/158,342
   <151> 1998-09-21
<160> 24
<210> 1
   <211> 215
   <212> PRT
   <213> Homo sapiens
<400> 1
   Met Arg Ser Gly Cys Val Val Val His Val Trp Ile Leu Ala Gly
     1               5                  10                  15
   Leu Trp Leu Ala Val Ala Gly Arg Pro Leu Ala Phe Ser Asp Ala
                    20                  25                  30
   Gly Pro His Val His Tyr Gly Trp Gly Asp Pro Ile Arg Leu Arg
                    35                  40                  45
   His Leu Tyr Thr Ser Gly Pro His Gly Leu Ser Ser Cys Phe Leu
                    50                  55                  60
   Arg Ile Arg Ala Asp Gly Val Val Asp Cys Arg Gly Gln Ser Ala
                    65                  70                  75
   His Ser Leu Leu Glu Ile Lys Ala Val Ala Leu Arg Thr Val Ala
                    80                  85                  90
   Ile Lys Gly Val His Ser Val Arg Tyr Leu Cys Met Gly Ala Asp
                    95                 100                 105
   Gly Lys Met Gln Gly Leu Leu Gln Tyr Ser Glu Glu Asp Cys Ala
                   110                 115                 120
   Phe Glu Glu Glu Ile Arg Pro Asp Gly Tyr Asn Val Tyr Arg Ser
                   125                 130                 135
   Glu Lys His Arg Leu Pro Val Ser Leu Ser Ser Ala Lys Gln Arg
                   140                 145                 150
   Gln Leu Tyr Lys Asn Arg Gly Phe Leu Pro Leu Ser His Phe Leu
                   155                 160                 165
   Pro Met Leu Pro Met Val Pro Glu Glu Pro Glu Asp Leu Arg Gly
                   170                 175                 180
   His Leu Glu Ser Asp Met Phe Ser Ser Pro Leu Glu Thr Asp Ser
                   185                 190                 195
   Met Asp Pro Phe Gly Leu Val Thr Gly Leu Glu Ala Val Arg Ser
                   200                 205                 210
   Pro Ser Phe Glu Lys
                   215
<210> 2
   <211> 2137
   <212> DNA
   <213> Homo sapiens
<400> 2
   gctcccagcc aagaacctcg gggccgctgc gcggtgggga ggagttcccc 50
   gaaacccggc cgctaagcga ggcctcctcc tcccgcagat ccgaacggcc 100
   tgggcggggt caccccggct gggacaagaa gccgccgcct gcctgcccgg 150
   gcccggggag ggggctgggg ctggggccgg aggcggggtg tgagtgggtg 200
   tgtgcggggg gcggaggctt gatgcaatcc cgataagaaa tgctcgggtg 250
   tcttgggcac ctacccgtgg ggcccgtaag gcgctactat ataaggctgc 300
   cggcccggag ccgccgcgcc gtcagagcag gagcgctgcg tccaggatct 350
   agggccacga ccatcccaac ccggcactca cagccccgca gcgcatcccg 400
   gtcgccgccc agcctcccgc acccccatcg ccggagctgc gccgagagcc 450
   ccagggaggt gccatgcgga gcgggtgtgt ggtggtccac gtatggatcc 500
   tggccggcct ctggctggcc gtggccgggc gccccctcgc cttctcggac 550
   gcggggcccc acgtgcacta cggctggggc gaccccatcc gcctgcggca 600
   cctgtacacc tccggccccc acgggctctc cagctgcttc ctgcgcatcc 650
   gtgccgacgg cgtcgtggac tgcgcgcggg gccagagcgc gcacagtttg 700
   ctggagatca aggcagtcgc tctgcggacc gtggccatca agggcgtgca 750
   cagcgtgcgg tacctctgca tgggcgccga cggcaagatg caggggctgc 800
   ttcagtactc ggaggaagac tgtgctttcg aggaggagat ccgcccagat 850
   ggctacaatg tgtaccgatc cgagaagcac cgcctcccgg tctccctgag 900
   cagtgccaaa cagcggcagc tgtacaagaa cagaggcttt cttccactct 950
   ctcatttcct gcccatgctg cccatggtcc cagaggagcc tgaggacctc 1000
   aggggccact tggaatctga catgttctct tcgcccctgg agaccgacag 1050
   catggaccca tttgggcttg tcaccggact ggaggccgtg aggagtccca 1100
   gctttgagaa gtaactgaga ccatgcccgg gcctcttcac tgctgccagg 1150
   ggctgtggta cctgcagcgt gggggacgtg cttctacaag aacagtcctg 1200
   agtccacgtt ctgtttagct ttaggaagaa acatctagaa gttgtacata 1250
   ttcagagttt tccattggca gtgccagttt ctagccaata gacttgtctg 1300
   atcataacat tgtaagcctg tagcttgccc agctgctgcc tgggccccca 1350
   ttctgctccc tcgaggttgc tggacaagct gctgcactgt ctcagttctg 1400
   cttgaatacc tccatcgatg gggaactcac ttcctttgga aaaattctta 1450
   tgtcaagctg aaattctcta attttttctc atcacttccc caggagcagc 1500
   cagaagacag gcagtagttt taatttcagg aacaggtgat ccactctgta 1550
   aaacagcagg taaatttcac tcaaccccat gtgggaattg atctatatct 1600
   ctacttccag ggaccatttg cccttcccaa atccctccag gccagaactg 1650
   actggagcag gcatggccca ccaggcttca ggagtagggg aagcctggag 1700
   ccccactcca gccctgggac aacttgagaa ttccccctga ggccagttct 1750
   gtcatggatg ctgtcctgag aataacttgc tgtcccggtg tcacctgctt 1800
   ccatctccca gcccaccagc cctctgccca cctcacatgc ctccccatgg 1850
   attggggcct cccaggcccc ccaccttatg tcaacctgca cttcttgttc 1900
   aaaaatcagg aaaagaaaag atttgaagac cccaagtctt gtcaataact 1950
   tgctgtgtgg aagcagcggg ggaagaccta gaaccctttc cccagcactt 2000
   ggttttccaa catgatattt atgagtaatt tattttgata tgtacatctc 2050
   ttattttctt acattattta tgcccccaaa ttatatttat gtatgtaagt 2100
   gaggtttgtt ttgtatatta aaatggagtt tgtttgt 2137
<210> 3
   <211> 214
   <212> PRT
   <213> Homo sapiens
<400> 3
   Ser Gly Cys Val Val Val His Val Trp Ile Leu Ala Gly Leu Trp
    1               5                  10                  15
   Leu Ala Val Ala Gly Arg Pro Leu Ala Phe Ser Asp Ala Gly Pro
                   20                  25                  30
   His Val His Tyr Gly Trp Gly Asp Pro Ile Arg Leu Arg His Leu
                   35                  40                  45
   Tyr Thr Ser Gly Pro His Gly Leu Ser Ser Cys Phe Leu Arg Ile
                   50                  55                  60
   Arg Ala Asp Gly Val Val Asp Cys Ala Arg Gly Gln Ser Ala His
                   65                  70                  75
   Ser Leu Leu Glu Ile Lys Ala Val Ala Leu Arg Thr Val Ala Ile
                   80                  85                  90
   Lys Gly Val His Ser Val Arg Tyr Leu Cys Met Gly Ala Asp Gly
                   95                 100                 105
   Lys Met Gln Gly Leu Leu Gln Tyr Ser Glu Glu Asp Cys Ala Phe
                  110                 115                 120
   Glu Glu Glu Ile Arg Pro Asp Gly Tyr Asn Val Tyr Arg Ser Glu
                  125                 130                 135
   Lys His Arg Leu Pro Val Ser Leu Ser Ser Ala Lys Gln Arg Gln
                  140                 145                 150
   Leu Tyr Lys Asn Arg Gly Phe Leu Pro Leu Ser His Phe Leu Pro
                  155                 160                 165
   Met Leu Pro Met Val Pro Glu Glu Pro Glu Asp Leu Arg Gly His
                  170                 175                 180
   Leu Glu Ser Asp Met Phe Ser Ser Pro Leu Glu Thr Asp Ser Met
                  185                 190                 195
   Asp Pro Phe Gly Leu Val Thr Gly Leu Glu Ala Val Arg Ser Pro
                  200                 205                 210
   Ser Phe Glu Lys
              214
<210> 4
   <211> 213
   <212> PRT
   <213> Mus Musculus
<400> 4
   Asn Gly Arg Ala Val Ala Arg Ala Leu Val Leu Ala Thr Leu Trp
   1               5                   10                  15
   Leu Ala Val Ser Gly Arg Pro Leu Ala Gln Gln Ser Gln Ser Val
                   20                  25                  30
   Ser Asp Glu Asp Pro Leu Phe Leu Tyr Gly Trp Gly Lys Ile Thr
               35                  40                  45
   Arg Leu Gln Tyr Leu Tyr Ser Ala Gly Pro Tyr Val Ser Asn Cys
               50                  55                  60
   Phe Leu Arg Ile Arg Ser Asp Gly Ser Val Asp Cys Glu Glu Asp
               65                  70                  75
   Gln Asn Glu Arg Asn Leu Leu Glu Phe Arg Ala Val Ala Leu Lys
               80                  85                  90
   Thr Ile Ala Ile Lys Asp Val Ser Ser Val Arg Tyr Leu Cys Met
               95                  100                 105
   Ser Ala Asp Gly Lys Ile Tyr Gly Leu Ile Arg Tyr Ser Glu Glu
               110                 115                 120
   Asp Cys Thr Phe Arg Glu Glu Met Asp Cys Leu Gly Tyr Asn Gln
               125                 130                 135
   Tyr Arg Ser Met Lys His His Leu His Ile Ile Phe Ile Gln Ala
               140                 145                 150
   Lys Pro Arg Glu Gln Leu Gln Asp Gln Lys Pro Ser Asn Phe Ile
               155                 160                 165
   Pro Val Phe His Arg Ser Phe Phe Glu Thr Gly Asp Gln Leu Arg
               170                 175                 180
   Ser Lys Met Phe Ser Leu Pro Leu Glu Ser Asp Ser Met Asp Pro
               185                 190                 195
   Phe Arg Met Val Glu Asp Val Asp His Leu Val Lys Ser Pro Ser
               200                 205                 210
   Phe Gln Lys
           213
<210> 5
   <211> 752
   <212> DNA
   <213> Homo sapiens
<220>
   <221> unknown
   <222> 11, 14, 50, 403, 429, 442, 445, 466, 474, 487, 518, 525, 531, 540, 546, 561, 566, 572, 580, 582, 587, 637, 656, 671, 680, 690, 695, 722, 737, 740, 750
   <223> unknown base
<400> 5
   aaatctgccg nttngaaatg gcactgccag tcgaaaactc tgaatatgtn 50
   caacttctag atgtttcttc ctaaagctaa acagaacgtg gactcaggac 100
   tgttcttgta gaagcacgtc ccccacgctg caggtaccac agcccctggc 150
   agcagtgaag aggcccgggc atggtctcag ttacttctca aagctgggac 200
   tcctcacggc ctccagtccg gtgacaagcc caaatgggtc catgctgtcg 250
   gtctccaggg gcgaagagaa catgtcagat tccaagtggc ccctgaggtc 300
   ctcaggctcc tctgggacca tgggcagcat gggcaggaaa tgagagagtg 350
   gaagaaagcc tctgttcttg tacagctgcc gctgtttggc actgctcagg 400
   ganaccggga agcggtgctt ctcggatcng tacacattgt anccntctgg 450
   gcggatctcc tcctcnaaac acantctccc ccgagtnctg aaactgcaaa 500
   aaaaaaggca ctctgacnat ccccnttgga naggaaaacn ggtccncatg 550
   aactttgtct ncaaantgtc cnggtggccn cnacatnaac tcccataaac 600
   ccccttgcat ttttaaacat actcctgatg tcacccnttc ttttaattat 650
   aaaaanaacc taaatattta nttttaaaan aaatttcttn agggncgcca 700
   ttggtcccct ttttccccct tnggagccaa caatgtnccn gaaccgattn 750
   aa 752
<210> 6
   <211> 371
   <212> DNA
   <213> Homo sapiens
<400> 6
   ttcgaggagg agatccgccc agatggctac aatgtgtacc gatccgagaa 50
   gcaccgcctc ccggtctccc tgagcagtgc caaacagcgg cagtgtacaa 100
   gaacagaggc tttcttccac tctctcattt cctgcccatg ctgcccatgg 150
   tcccagagga gcctgaggac ctcaggggcc acttggaatc tgacatgttc 200
   tcttcgcccc tggagaccga cagcatggac ccatttgggc ttgtcaccgg 250
   actggaggcc gtgaggagtc ccagctttga gaagtaactg agaccatgcc 300
   cgggcctctt cactgctgcc aggggtgtgg tacctgcagc gtgggggacg 350
   tgcttctaca agaacagtcc t 371
<210> 7
   <211> 1824
   <212> DNA
   <213> Mus Musculus
<400> 7
   ctgtcggagc agtaactctg tgcgccccac gccacaagcg cccagttgct 50
   ttgtgggttg tgcctgccct gcgcctgcaa cttgagtccc cgccgcatcg 100
   cagtctccgc gccacctttg taacggcctt caggaccccg aggtgtcatg 150
   gcgagaaagt ggaacgggcg tgcggtggcc cgagccctgg tcctggccac 200
   tctgtggctg gctgtgtctg ggcgtcccct ggcccagcaa tcccagtctg 250
   tgtcagatga agatccactc tttctctacg gctggggcaa gattacccgc 300
   ctgcagtacc tgtactccgc tggtccctat gtctccaact gcttcctccg 350
   aatccggagc gacggctctg tggactgcga ggaggaccaa aacgaacgaa 400
   atttgttgga attccgcgcg gtcgctctga agacgattgc catcaaggac 450
   gtcagcagcg tgcggtacct ctgcatgagc gcggacggca agatatacgg 500
   gctgattcgc tactcggagg aagactgtac cttcagggag gaaatggact 550
   gtttaggcta caaccagtac agatccatga agcaccatct ccatatcatc 600
   ttcatccagg ccaagcccag agaacagctc caggaccaga aaccctcaaa 650
   ctttatcccc gtgtttcacc gctccttctt tgaaaccggg gaccagctga 700
   ggtctaaaat gttctccctg cccctggaga gtgacagcat ggatccgttc 750
   aggatggtgg aggatgtaga ccacctagtg aagagtccca gcttccagaa 800
   atgacaggat tccgacagga tggagaaaac cccaaggtcc cgtgaacttc 850
   ccccttagga agctgtagat attctaagtc tcacatggac cctgttgtgt 900
   tagtggctag acttgatcat gaacctaagt tgacaacctg cctggctgcc 950
   atcggagccc cactgacttt ggaggctgct gatatgtgcc taagttactc 1000
   cagttctgtt tgaatacctc cactaatagg gaacttactc ctgtgaaaca 1050
   ttcttagttt tgagccaaat ctgtgacttg gatggtttta gcgagcaagc 1100
   cagaaggtat gaagtcaaat gataaaattc atgtatagaa agtgggctct 1150
   aaaatatata ttccctatat ggatctcatg ggatcttagc ttgcccccca 1200
   aatgtctcct ggccagaact aactggggtt acaaacttgg aacaaaggac 1250
   agcctagaaa actttgggag ccttgaagga tggtcttagg attacgaatt 1300
   ccagctgact acgtagcttc ccccttttcc acttataaat gtcagatgga 1350
   agtgaccctt agctgagtgc atagccaagc tgccacttat gccccaggag 1400
   cttgtctctg tcccatgacc ccagatttcc aggacctgga tctgctcctc 1450
   tgacctttcc cagagttcac ctgggctctc caaccccaga gcaggtagct 1500
   tatgagccat ccagttgtgt ccccagctcc tggctctcag ttctggtcac 1550
   caaacattgt gaatcaacgt gtctgctgcc tgtgtcaacc tggatccctc 1600
   atttacaaac aagattagga agccccaaat tctccagtgg cagctgggga 1650
   actgtggagt cctttccccg gcacttacgt ggcagcatga tatttataag 1700
   taatttattg tgtgtgtgtc ttctattttc ttactttatt tatgccccag 1750
   atcatattta tgtacatgac ttgttttcta cattaaaaag gagttggttt 1800
   gtatcaaaaa aaaaaaaaaa aaaa 1824
<210> 8
   <211> 216
   <212> PRT
   <213> Homo sapiens
<400> 8
   Met Arg Ser Gly Cys Val Val Val His Val Trp Ile Leu Ala Gly
   1               5                   10                  15
   Leu Trp Leu Ala Val Ala Gly Arg Pro Leu Ala Phe Ser Asp Ala
                   20                  25                  30
   Gly Pro His Val His Tyr Gly Trp Gly Asp Pro Ile Arg Leu Arg
                   35                  40                  45
   His Leu Tyr Thr Ser Gly Pro His Gly Leu Ser Ser Cys Phe Leu
                   50                  55                  60
   Arg Ile Arg Ala Asp Gly Val Val Asp Cys Ala Arg Gly Gln Ser
                   65                  70                  75
   Ala His Ser Leu Leu Glu Ile Lys Ala Val Ala Leu Arg Thr Val
                   80                  85                  90
   Ala Ile Lys Gly Val His Ser Val Arg Tyr Leu Cys Met Gly Ala
                   95                  100                 105
   Asp Gly Lys Met Gln Gly Leu Leu Gln Tyr Ser Glu Glu Asp Cys
                   110                 115                 120
   Ala Phe Glu Glu Glu Ile Arg Pro Asp Gly Tyr Asn Val Tyr Arg
                   125                 130                 135
   Ser Glu Lys His Arg Leu Pro Val Ser Leu Ser Ser Ala Lys Gln
                   140                 145                 150
   Arg Gln Leu Tyr Lys Asn Arg Gly Phe Leu Pro Leu Ser His Phe
                   155                 160                 165
   Leu Pro Met Leu Pro Met Val Pro Glu Glu Pro Glu Asp Leu Arg
                   170                 175                 180
   Gly His Leu Glu Ser Asp Met Phe Ser Ser Pro Leu Glu Thr Asp
                   185                 190                 195
   Ser Met Asp Pro Phe Gly Leu Val Thr Gly Leu Glu Ala Val Arg
                   200                 205                 210
   Ser Pro Ser Phe Glu Lys
                   215 216
<210> 9
   <211> 155
   <212> PRT
   <213> Homo sapiens
<400> 9
   Met Ala Glu Gly Glu Ile Thr Thr Phe Thr Ala Leu Thr Glu Lys
     1               5                  10                  15
   Phe Asn Leu Pro Pro Gly Asn Tyr Lys Lys Pro Lys Leu Leu Tyr
                    20                  25                  30
   Cys Ser Asn Gly Gly His Phe Leu Arg Ile Leu Pro Asp Gly Thr
                    35                  40                  45
   Val Asp Gly Thr Arg Asp Arg Ser Asp Gln His Ile Gln Leu Gln
                    50                  55                  60
   Leu Ser Ala Glu Ser Val Gly Glu Val Tyr Ile Lys Ser Thr Glu
                    65                  70                  75
   Thr Gly Gln Tyr Leu Ala Met Asp Thr Asp Gly Leu Leu Tyr Gly
                    80                  85                  90
   Ser Gln Thr Pro Asn Glu Glu Cys Leu Phe Leu Glu Arg Leu Glu
                    95                 100                 105
   Glu Asn His Tyr Asn Thr Tyr Ile Ser Lys Lys His Ala Glu Lys
                   110                 115                 120
   Asn Trp Phe Val Gly Leu Lys Lys Asn Gly Ser Cys Lys Arg Gly
                   125                 130                 135
   Pro Arg Thr His Tyr Gly Gln Lys Ala Ile Leu Phe Leu Pro Leu
                   140                 145                 150
   Pro Val Ser Ser Asp
                   155
<210> 10
   <211> 268
   <212> PRT
   <213> Homo sapiens
<400> 10
   Met Ser Leu Ser Phe Leu Leu Leu Leu Phe Phe Ser His Leu Ile
     1               5                  10                  15
   Leu Ser Ala Trp Ala His Gly Glu Lys Arg Leu Ala Pro Lys Gly
                    20                  25                  30
   Gln Pro Gly Pro Ala Ala Thr Asp Arg Asn Pro Ile Gly Ser Ser
                    35                  40                  45
   Ser Arg Gln Ser Ser Ser Ser Ala Met Ser Ser Ser Ser Ala Ser
                   50                  55                  60
   Ser Ser Pro Ala Ala Ser Leu Gly Ser Gln Gly Ser Gly Leu Glu
                   65                  70                  75
   Gln Ser Ser Phe Gln Trp Ser Pro Ser Gly Arg Arg Thr Gly Ser
                   80                  85                  90
   Leu Tyr Cys Arg Val Gly Ile Gly Phe His Leu Gln Ile Tyr Pro
                   95                  100                 105
   Asp Gly Lys Val Asn Gly Ser His Glu Ala Asn Met Leu Ser Val
                   110                 115                 120
   Leu Glu Ile Phe Ala Val Ser Gln Gly Ile Val Gly Ile Arg Gly
                   125                 130                 135
   Val Phe Ser Asn Lys Phe Leu Ala Met Ser Lys Lys Gly Lys Leu
                   140                 145                 150
   His Ala Ser Ala Lys Phe Thr Asp Asp Cys Lys Phe Arg Glu Arg
                   155                 160                 165
   Phe Gln Glu Asn Ser Tyr Asn Thr Tyr Ala Ser Ala Ile His Arg
                   170                 175                 180
   Thr Glu Lys Thr Gly Arg Glu Trp Tyr Val Ala Leu Asn Lys Arg
                   185                 190                 195
   Gly Lys Ala Lys Arg Gly Cys Ser Pro Arg Val Lys Pro Gln His
                   200                 205                 210
   Ile Ser Thr His Phe Leu Pro Arg Phe Lys Gln Ser Glu Gln Pro
                   215                 220                 225
   Glu Leu Ser Phe Thr Val Thr Val Pro Glu Lys Lys Asn Pro Pro
                   230                 235                 240
   Ser Pro Ile Lys Ser Lys Ile Pro Leu Ser Ala Pro Arg Lys Asn
                   245                 250                 255
   Thr Asn Ser Val Lys Tyr Arg Leu Lys Phe Arg Phe Gly
                   260                 265         268
<210> 11
   <211> 194
   <212> PRT
   <213> Homo sapiens
<400> 11
   Met His Lys Trp Ile Leu Thr Trp Ile Leu Pro Thr Leu Leu Tyr
    1               5                  10                  15
   Arg Ser Cys Phe His Ile Ile Cys Leu Val Gly Thr Ile Ser Leu
                   20                  25                  30
   Ala Cys Asn Asp Met Thr Pro Glu Gln Met Ala Thr Asn Val Asn
                   35                  40                  45
   Cys Ser Ser Pro Glu Arg His Thr Arg Ser Tyr Asp Tyr Met Glu
                   50                  55                  60
   Gly Gly Asp Ile Arg Val Arg Arg Leu Phe Cys Arg Thr Gln Trp
                   65                  70                  75
   Tyr Leu Arg Ile Asp Lys Arg Gly Lys Val Lys Gly Thr Gln Glu
                   80                  85                  90
   Met Lys Asn Asn Tyr Asn Ile Met Glu Ile Arg Thr Val Ala Val
                   95                  100                 105
   Gly Ile Val Ala Ile Lys Gly Val Glu Ser Glu Phe Tyr Leu Ala
                   110                 115                 120
   Met Asn Lys Glu Gly Lys Leu Tyr Ala Lys Lys Glu Cys Asn Glu
                   125                 130                 135
   Asp Cys Asn Phe Lys Glu Leu Ile Leu Glu Asn His Tyr Asn Thr
                   140                 145                 150
   Tyr Ala Ser Ala Lys Trp Thr His Asn Gly Gly Glu Met Phe Val
                   155                 160                 165
   Ala Leu Asn Gln Lys Gly Ile Pro Val Arg Gly Lys Lys Thr Lys
                   170                 175                 180
   Lys Glu Gln Lys Thr Ala His Phe Leu Pro Met Ala Ile Thr
                   185                 190                 194
<210> 12
   <211> 231
   <212> PRT
   <213> Homo sapiens
<400> 12
   Met Gly Ser Pro Arg Ser Ala Leu Ser Cys Leu Leu Leu His Leu
    1               5                  10                  15
   Leu Val Leu Cys Leu Gln Ala Gln Glu Gly Pro Gly Arg Gly Pro
                   20                  25                  30
   Ala Leu Gly Arg Glu Leu Ala Ser Leu Phe Arg Ala Gly Arg Glu
                   35                  40                  45
   Pro Gln Gly Val Ser Gln Gln His Val Arg Glu Gln Ser Leu Val
                   50                  55                  60
   Thr Asp Gln Leu Ser Arg Arg Leu Ile Arg Thr Tyr Gln Leu Tyr
                   65                  70                  75
   Ser Arg Thr Ser Gly Lys His Val Gln Val Leu Ala Asn Lys Arg
                   80                  85                  90
   Ile Asn Met Ala Glu Asp Gly Asp Pro Phe Ala Lys Leu Ile Val
                   95                 100                 105
   Glu Thr Asp Thr Phe Gly Ser Arg Val Arg Val Arg Gly Ala Glu
                  110                 115                 120
   Thr Gly Leu Tyr Ile Cys Met Asn Lys Lys Gly Lys Leu Ile Ala
                  125                 130                 135
   Lys Ser Asn Gly Lys Gly Lys Asp Cys Val Phe Thr Glu Ile Val
                  140                 145                 150
   Leu Glu Asn Asn Tyr Thr Ala Leu Gln Asn Ala Lys Tyr Glu Gly
                  155                 160                 165
   Trp Tyr Met Ala Phe Thr Arg Lys Gly Arg Pro Arg Lys Gly Ser
                  170                 175                 180
   Lys Thr Arg Gln His Gln Arg Glu Val His Phe Met Lys Arg Leu
                  185                 190                 195
   Pro Arg Gly His His Thr Thr Glu Gln Ser Leu Phe Glu Phe Leu
                  200                 205                 210
   Asn Tyr Pro Pro Phe Thr Arg Ser Leu Arg Gly Ser Gln Arg Thr
                  215                 220                 225
   Trp Ala Pro Glu Pro Arg
                  230 231
<210> 13
   <211> 225
   <212> PRT
   <213> Homo sapiens
<400> 13
   Met Ala Ala Leu Ala Ser Ser Leu Ile Arg Gln Lys Arg Glu Val
   1               5                   10                  15
   Arg Glu Pro Gly Gly Ser Arg Pro Val Ser Ala Gln Arg Arg Val
                   20                  25                  30
   Cys Pro Arg Gly Thr Lys Ser Leu Cys Gln Lys Gln Leu Leu Ile
                   35                  40                  45
   Leu Leu Ser Lys Val Arg Leu Cys Gly Gly Arg Pro Ala Arg Pro
                   50                  55                  60
   Asp Arg Gly Pro Glu Pro Gln Leu Lys Gly Ile Val Thr Lys Leu
                   65                  70                  75
   Phe Cys Arg Gln Gly Phe Tyr Leu Gln Ala Asn Pro Asp Gly Ser
                   80                  85                  90
   Ile Gln Gly Thr Pro Glu Asp Thr Ser Ser Phe Thr His Phe Asn
                   95                  100                 105
   Leu Ile Pro Val Gly Leu Arg Val Val Thr Ile Gln Ser Ala Lys
                   110                 115                 120
   Leu Gly His Tyr Met Ala Met Asn Ala Glu Gly Leu Leu Tyr Ser
                   125                 130                 135
   Ser Pro His Phe Thr Ala Glu Cys Arg Phe Lys Glu Cys Val Phe
                   140                 145                 150
   Glu Asn Tyr Tyr Val Leu Tyr Ala Ser Ala Leu Tyr Arg Gln Arg
                   155                 160                 165
   Arg Ser Gly Arg Ala Trp Tyr Leu Gly Leu Asp Lys Glu Gly Gln
                   170                 175                 180
   Val Met Lys Gly Asn Arg Val Lys Lys Thr Lys Ala Ala Ala His
                   185                 190                 195
   Phe Leu Pro Lys Leu Leu Glu Val Ala Met Tyr Gln Glu Pro Ser
                   200                 205                 210
   Leu His Ser Val Pro Glu Ala Ser Pro Ser Ser Pro Pro Ala Pro
                   215                 220                 225
<210> 14
   <211> 252
   <212> PRT
   <213> Homo sapiens
<400> 14
   Met Val Lys Pro Val Pro Leu Phe Arg Arg Thr Asp Phe Lys Leu
    1               5                  10                  15
   Leu Leu Cys Asn His Lys Asp Leu Phe Phe Leu Arg Val Ser Lys
                   20                  25                  30
   Leu Leu Asp Cys Phe Ser Pro Lys Ser Met Trp Phe Leu Trp Asn
                   35                  40                  45
   Ile Phe Ser Lys Gly Thr His Met Leu Gln Cys Leu Cys Gly Lys
                   50                  55                  60
   Ser Leu Lys Lys Asn Lys Asn Pro Thr Asp Pro Gln Leu Lys Gly
                   65                  70                  75
   Ile Val Thr Arg Leu Tyr Cys Arg Gln Gly Tyr Tyr Leu Gln Met
                   80                  85                  90
   His Pro Asp Gly Ala Leu Asp Gly Thr Lys Gly Asp Ser Thr Asn
                   95                 100                 105
   Ser Thr Leu Phe Asn Leu Ile Pro Val Gly Leu Arg Val Val Ala
                  110                 115                 120
   Ile Gln Gly Val Lys Thr Gly Leu Tyr Ile Thr Met Asn Gly Glu
                  125                 130                 135
   Gly Tyr Leu Tyr Pro Ser Glu Leu Phe Thr Pro Glu Cys Lys Phe
                  140                 145                 150
   Lys Glu Ser Val Phe Glu Asn Tyr Tyr Val Ile Tyr Ser Ser Met
   155 160 165
   Leu Tyr Arg Gln Gln Glu Ser Gly Arg Ala Trp Phe Leu Gly Leu
   170 175 180
   Asn Lys Glu Gly Gln Ala Met Lys Gly Asn Arg Val Lys Lys Thr
   185 190 195
   Lys Pro Ala Ala His Phe Leu Pro Lys Pro Leu Glu Val Ala Met
   200 205 210
   Tyr Arg Glu Pro Ser Leu His Asp Val Gly Glu Thr Val Pro Lys
   215 220 225
   Pro Gly Val Thr Pro Ser Lys Ser Thr Ser Ala Ser Ala Ile Met
   230 235 240
   Asn Gly Gly Lys Pro Val Asn Lys Ser Lys Thr Thr
   245 250 252
<210> 15
   <211> 207
   <212> PRT
   <213> Homo sapiens
<400> 15
   Met Tyr Ser Ala Pro Ser Ala Cys Thr Cys Leu Cys Leu His Phe
    1               5                  10                  15
   Leu Leu Leu Cys Phe Gln Val Gln Val Leu Val Ala Glu Glu Asn
                   20                  25                  30
   Val Asp Phe Arg Ile His Val Glu Asn Gln Thr Arg Ala Arg Asp
                   35                  40                  45
   Asp Val Ser Arg Lys Gln Leu Arg Leu Tyr Gln Leu Tyr Ser Arg
                   50                  55                  60
   Thr Ser Gly Lys His Ile Gln Val Leu Gly Arg Arg Ile Ser Ala
                   65                  70                  75
   Arg Gly Glu Asp Gly Asp Lys Tyr Ala Gln Leu Leu Val Glu Thr
                   80                  85                  90
   Asp Thr Phe Gly Ser Gln Val Arg Ile Lys Gly Lys Glu Thr Glu
                   95                  100                 105
   Phe Tyr Leu Cys Met Asn Arg Lys Gly Lys Leu Val Gly Lys Pro
                   110                 115                 120
   Asp Gly Thr Ser Lys Glu Cys Val Phe Ile Glu Lys Val Leu Glu
                   125                 130                 135
   Asn Asn Tyr Thr Ala Leu Met Ser Ala Lys Tyr Ser Gly Trp Tyr
                   140                 145                 150
   Val Gly Phe Thr Lys Lys Gly Arg Pro Arg Lys Gly Pro Lys Thr
                   155                 160                 165
   Arg Glu Asn Gln Gln Asp Val His Phe Met Lys Arg Tyr Pro Lys
                   170                 175                 180
   Gly Gln Pro Glu Leu Gln Lys Pro Phe Lys Tyr Thr Thr Val Thr
                   185                 190                 195
   Lys Arg Ser Arg Arg Ile Arg Pro Thr His Pro Ala
                   200                 205     207
<210> 16
   <211> 26
   <212> DNA
   <213> Artificial
<220>
   <223> Artificial sequence
<400> 16
   atccgcccag atggctacaa tgtgta 26
<210> 17
   <211> 40
   <212> DNA
   <213> Artificial
<220>
   <223> Artificial sequence
<400> 17
   agaccgggag gcggtgcttc tcggatcggt acacattgta 40
<210> 18
   <211> 22
   <212> DNA
   <213> Artificial
<220>
   <223> Artificial sequence
<400> 18
   ccagtccggt gacaagccca aa 22
<210> 19
   <211> 46
   <212> DNA
   <213> Artificial
<220>
   <223> Artificial sequence
<400> 19
   ggattctaat acgactcact atagggcgga tcctggccgg cctcgg 46
<210> 20
   <211> 48
   <212> DNA
   <213> Artificial
<220>
   <223> Artificial sequence
<400> 20
   ctatgaaatt aaccctcact aaagggagcc cgggcatggt ctcagtta 48
<210> 21
   <211> 23
   <212> DNA
   <213> Artificial
<220>
   <223> Artificial sequence
<400> 21
   gggacgtgct tctacaagaa cag 23
<210> 22
   <211> 26
   <212> DNA
   <213> Artificial
<220>
   <223> Artificial sequence
<400> 22
   caggcttaca atgttatgat cagaca 26
<210> 23
   <211> 31
   <212> DNA
   <213> Artificial
<220>
   <223> Artificial sequence
<400> 23
   tattcagagt tttccattgg cagtgccagt t 31
<210> 24
   <211> 66
   <212> PRT
   <213> Artificial
<220>
   <223> Artificial sequence
<400> 24
   Met Gly Gly Ala Ala Ala Arg Leu Gly Ala Val Ile Leu Phe Val
     1             5                  10                  15
   Val Ile Val Gly Leu His Gly Val Arg Gly Lys Tyr Ala Leu Ala
                  20                  25                  30
   Asp Ala Ser Leu Lys Met Ala Asp Pro Asn Arg Phe Arg Gly Lys
                  35                  40                  45
   Asp Leu Pro Val Leu Asp Gln Leu Leu Glu Gly Gly Ala Ala His
                  50                  55                  60
   Tyr Ala Leu Leu Pro Gly
                  65  66

### Sequence Listing

<110> Genentech Inc.
   Botstein, David A.
   Goddard, Audrey
   Gurney, Austin L.
   Hillan, Kenneth J.
   Lawrence, David A.
   Roy, Margaret Ann
<120> Fibroblast Growth Factor-19
<130> P1219 R1
<140> PCT/US98/25190
   <141> 1998-11-25
<150> US 60/066,840
   <151> 1997-11-25
<150> US 09/158,342
   <151> 1998-09-21
<160> 24
<210> 1
   <211> 216
   <212> PRT
   <213> Homo sapiens
<400> 2
<210> 2
   <211> 2137
   <212> DNA
   <213> Homo sapiens
<400> 2

### Sequence Listing

<110> Genentech Inc.
   Botstein, David A.
   Goddard, Audrey
   Gurney, Austin L.
   Hillan, Kenneth J.
   Lawrence, David A.
   Roy, Margaret Ann
<120> Fibroblast Growth Factor-19
<130> P1219 R1
<140> PCT/US98/25190
   <141> 1998-11-25
<150> US 60/066,840
   <151> 1997-11-25
<150> US 09/158,342
   <151> 1998-09-21
<160> 24
<210> 1
   <211> 216
   <212> PRT
   <213> Homo sapiens
<400> 2
<210> 2
   <211> 2137
   <212> DNA
   <213> Homo sapiens
<400> 2

## Description

### FIELD OF THE INVENTION

The present invention relates generally to the identification and isolation of novel DNA, and to the recombinant production of novel polypeptides, which are characterized by having homology to fibroblast growth factors. Specifically, the present inventions relates to the identification, isolation, characterization and uses of a novel member of the fibroblast growth factor (FGF) family, designated herein as FGF-19 (PRO533). In particular, the invention relates to compositions and methods for the diagnosis and treatment of tumors and/or other conditions characterized by FGF-19 modulation.

### BACKGROUND OF THE INVENTION

Extracellular proteins play an important role in the formation, differentiation and maintenance of multicellular organisms. The fate of many individual cells, *e.g.*, proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including pharmaceuticals, diagnostics, biosensors and bioreactors. Most protein drugs available at present, such as thrombolytic agents, interferons, interleukins, erythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein et al., Proc. Natl. Acad. Sci., 93:7108-7113 (1996); U.S. Patent No. 5,536,637)].

Growth factors are molecular signals or mediators that enhance cell growth or proliferation, alone or in concert, by binding to specific cell surface receptors. However, there are other cellular reactions than only grow upon expression to growth factors. As a result, growth factors are better characterized as multifunctional and potent cellular regulators. Their biological effects include proliferation, chemotaxis and stimulation of extracellular matrix production. Growth factors can have both stimulatory and inhibitory effects. For example, transforming growth factors (TGF-β) is highly pleiotropic and can stimulate proliferation in some cells, especially connective tissues, while being a potent inhibitor of proliferation in others, such as lymphocytes and epithelial cells.

The physiological effect of growth stimulation or inhibition by growth factors depends upon the state of development and differentiation of the target tissue. The mechanism of local cellular regulation by classical endocrine molecules comprehends autocrine (same cell), juxtacrine (neighbor cell), and paracrine (adjacent cell) pathways. Peptide growth factors are elements of a complex biological language, providing the basis for intercellular communication. They permit cells to convey information between each other, mediate interaction between cells and change gene expression. The effect of these multifunctional and pluripotent factors is dependent on the presence or absence of other peptides.

Fibroblast growth factors (FGFs) are a family of heparin-binding, potent mitogens for both normal diploid fibroblasts and established cell lines, Godpodarowicz, D. et al. (1984), Proc. Natl. Acad. Sci. USA 81: 6983. The FGF family comprises acidic FGF (FGF-1), basic FGF (FGF-2), INT-2 (FGF-3), K-FGF/HST (FGF-4). FGF-5, FGF-6. KGF (FGF-7), AIGF (FGF-8), and FGF-9 through FGF-18 among others. All FGFs have two conserved cysteine residues and share 30-50% sequence homology at the amino acid level. These factors are mitogenic for a wide variety of normal diploid mesoderm-derived and neural crest-derived cells, inducing granulosa cells, adrenal cortical cells, chrondocytes, myoblasts, corneal and vascular endothelial cells (bovine or human), vascular smooth muscle cells, lens, retina and prostatic epithelial cells, oligodendrocytes, astrocytes, chrondocytes, myoblasts and osteoblasts.

Fibroblast growth factors can also stimulate a large number of cell types in a non-mitogenic manner. These activities include promotion of cell migration into a wound area (chemotaxis), initiation of new blood vessel formulation (angiogenesis), modulation of nerve regeneration and survival (neurotrophism), modulation of endocrine functions, and stimulation or suppression of specific cellular protein expression, extracellular matrix production and cell survival. Baird, A. & Bohlen, P., Handbook of Exp. Phrmacol. 95(1): 369-418 (1990). These properties provide a basis for using fibroblast growth factors in therapeutic approaches to accelerate wound healing, nerve repair, collateral blood vessel formation, and the like. For example, fibroblast growth factors, have been suggested to minimize myocardium damage in heart disease and surgery (U.S.P. 4,378.437).

The fibroblast growth factors constitute a large family of mitogenic cytokines. lnitial members of this family were identified as compounds which exhibited potent proliferative activity on 3T3 fibroblasts. FGF members have now been shown to have diverse activities on cells of mesodermal or neuroectodermal origin with roles including the capacity to promote or inhibit differentiated phenotypes during development, mediate angiogenic and neurotrophic efects, and modulate cell migration. Goldfarb, M., Cytokine Growth Factor Rev. 7: 311-25 (1996); Naski, M.C. and Ornitz, D.M., Front Biosci. 3: D781-94 (1998); and Slavin J., Cell Biol. Int. 19: 431-44 (1995). Biological specificity is thought to arise in part form the controlled expression of both the distinct FGFs and the FGF receptors (FGFR). Four highly related receptor tyrosine kinases have been identified which bind to members of the FGF family. Variant splice forms have been identified for three of the FGFRs. The individual FGF studied to date have, with one exception, been shown to interact with multiple FGFR isoforms, although differences in relative affinity are thought to contribute to selectivity. Mathieu, M. et al., J. Biol. Chem. 270:24197-203 (1995); Ornitz, D.M. and P. Leder, J. Biol. Chem. 267: 16305-11 (1992); Ornitz D.M. et al., J. Biol. Chem. 271: 15292-7 (1996) and Santos-Ocampo, S. et al., J Biol. Chem. 271: 1726-31 (1996). FGFs interact directly with the FGFRs. However, biological activity is found to require heparin or heparin sulfate proteoglycans such as syndican or perlecan which are believed to facilitate FGF dimerization, and present additional opportunity for control of function. Aviezer et al., Cell 79: 1005-13 (1994): Bashkin et al., Biochemistry 28: 1737-43 (1989); Folkman, J. et al., Am. J. Pathol. 130: 393-400 (1988): Herr et al., J. Biol. Chem. 272: 16382-89: Kiefer, M.C. et al.. A528-530. N.Y. Acad Sci. 638: 167-76 (1991): Mach. H. et al., Biochemistry 32: 5480-90 (1993); Moscatelli. D.. J. Cell Physiol. 131: 123-30: Ornitz, D.M. et al.. Mol. Cell Biol. 12: 240-47 (1992); Saksela. O. et al.. J. Cell Biol. 107: 743-51 (1988).

Alteration of gene expression is intimately related to the uncontrolled cell growth and de-differentiation which are a common feature of all cancers. The genomes of certain well studied tumors have been found to show decreased expression of recessive genes, usually referred to as tumor suppression genes, which would normally function to prevent malignant cell growth, and/or overexpression of certain dominant genes, such as oncogenes, that act to promote malignant growth. Each of these genetic changes appears to be responsible for importing some of the traits that, in aggregate, represent the full neoplastic phenotype (Hunter, Cell 64, 1129 [1991]; Bishop, Cell 64, 235-248 [1991]).

A well known mechanism of gene (e.g. oncogene) overexpression in cancer cells is gene amplification. This is a process where in the chromosome of the ancestral cell multiple copies of a particular gene are produced. The process involves unscheduled replication of the region of chromosome comprising the gene, followed by recombination of the replicated segments back into the chromosome (Alitalo et al., Adv. Cancer Res. 47, 235-281 [1986]). It is believed that the overexpression of the gene parallels gene amplification, *i.e.* is proportionate to the number of copies made.

Proto-oncogenes that encode growth factors and growth factor receptors have been identified to play important roles in the pathogenesis of various human malignancies, including breast cancer. For example, it has been found that the human ErbB2 gene (*erb*B2, also known as *her2*, or *c-erbB-2*), which encodes a 185-kd transmembrane glycoprotein receptor (p185^{HER2}; HER2) related to the epidermal growth factor receptor (EGFR), is overexpressed in about 25% to 30% of human breast cancer (Slamon et al., Science 235: 177-182 [1987]; Slamon et al., Science 244: 707-712 [1989]). It has been reported that gene amplification of a protooncogen is an event typically involved in the more malignant forms of cancer, and could act as a predictor of clinical outcome (Schwab et al., Genes Chromosomes Cancer 1, 181-193 [1990]; Alitalo *et al*., *supra).* Thus, *erb*B2 overexpression is commonly regarded as a predictor of a poor prognosis, especially in patients with primary disease that involves axillary lymph nodes (Slamon *et al*., [1987] and [1989], *supra*: Ravdin and Chamness. Gene 159: 19-27 [1995]; and Hynes and Stem, Biochim Biophys Acta 1198: 165-184 [1994]), and has been linked to sensitivity and/or resistance to hormone therapy and chemotherapeutic regimens, including CMF (cyclophosphamide, methotrexate, and fluoruracil) and anthracyclines (Baselga et al., Oncology 11 (3 Suppl 1): 43-48 [1997]). However, despite the association of *erb*B2 overexpression with poor prognosis, the odds of HER2-positive patients responding clinically to treatment with taxanes were greater than three times those of HER2-negative patients (*Ibid*). A recombinant humanized anti-ErbB2 (anti-HER2) monoclonal antibody (a humanized version of the murine anti-ErbB2 antibody 4D5, referred to as rhuMAb HER2 or Herceptin^{®}) has been clinically active in patients with ErbB2-overexpressing metastatic breast cancers that had received extensive prior anticancer therapy. (Baselga et al., J. Clin. Oncol. 14: 737-744 [1996]).

### SUMMARY OF THE INVENTION

A cDNA clone (DNA49435) has been identified, which has homology to fibroblast growth factor, designated in the present application as "PRO533." This novel FGF has also been termed FGF-19. A DNA encoding PR0533 (DNA49435) has been identified as a gene that is amplified in the genome of certain tumor cells. Such amplification is expected to be associated with the overexpression of the gene product and to contribute to tumorigenesis and/or autocrine signaling. Accordingly, PRO533 is believed to be a useful target for the dianogsis and/or treatment (including prevention) of certain cancers, and may act as predictors of the prognosis of tumor treatment. Other proposed effects of DNA49435 include possible roles in cartilage or bone development and osteoporosis pseudoglioma.

In one embodiment as defined in the claims the invention provides an isolated nucleic acid molecule comprising DNA encoding a PRO533 polypeptide.

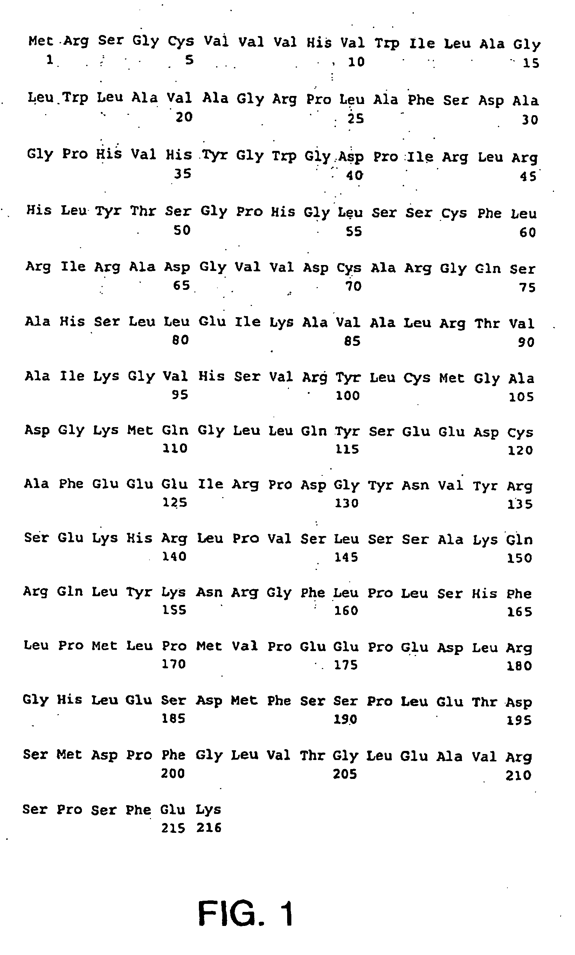

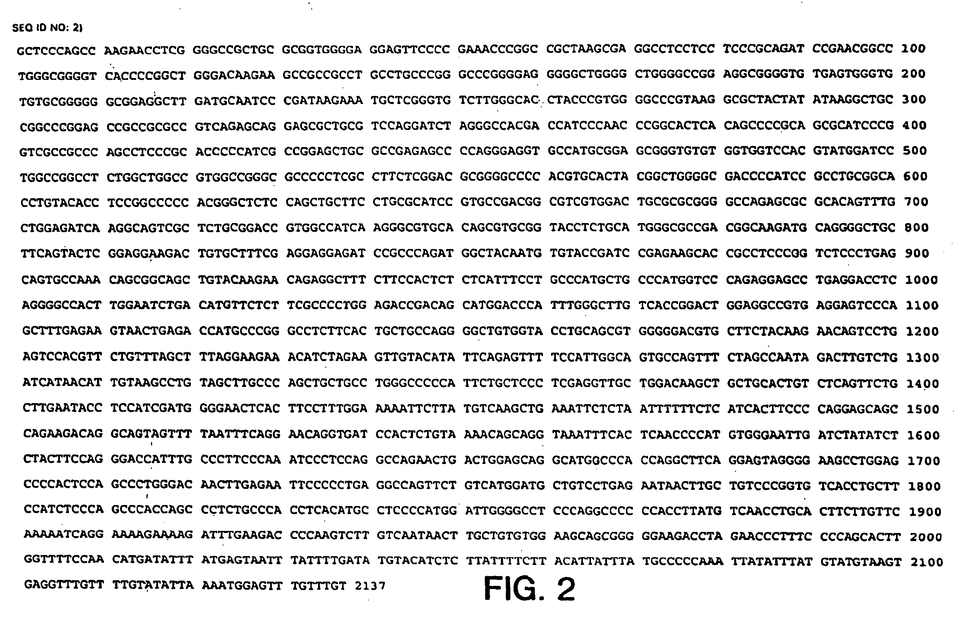

In another aspect as defined in the claims the invention concerns an isolated nucleic acid molecule encoding a PRO533 polypeptide having amino acid residues 1 to 216 of Fig. 1 (SEQ ID NO: 1), is complementary to such encoding nucleic acid sequence, and remains stably bound to it under at least moderate, and optionally, under high stringency conditions. Alternatively, an isolated nucleic acid molecule as defined in the claims encoding a PRO533 polypeptide comprising DNA hybridizing to the complement of the nucleic acid between about residues 464-466 and about 1109-1111, inclusive, of Figure 2 (SEQ ID NO: 2). Hybridization occurs under stringent hybridization and wash conditions.

In a further aspect as defined in the claims the invention concerns an isolated nucleic acid molecule comprising (a) a DNA molecule encoding a polypeptide having at least about 80% sequence identity, preferably at least about 85% sequence identity, more preferably at least about 90% sequence identity, most preferably at least about 95% sequence identity to the same mature polypeptide encoded by the human protein cDNA in ATCC Deposit No. 209480 (DNA49435-1219), or (b) the complement of the DNA molecule of (a). In a preferred embodiment, the nucleic acid comprises a DNA encoding the same mature polypeptide encoded by the human protein cDNA in ATCC Deposit No. 209480 (DNA49435-1219).

In a still further aspect as defined in the claims the invention concerns an isolated nucleic acid molecule comprising (a) DNA encoding a polypeptide having at least about 80% sequence identity, preferably at least about 85% sequence identity, more preferably at least about 90% sequence identity, most preferably at least about 95% sequence identity to the sequence of amino acid residues from about 23 to about 216, inclusive of Figure 1 (SEQ ID NO:1), or the complement of the DNA of (a).

In a further aspect as defined in the claims the invention concerns an isolated nucleic acid molecule having at least about 20-80 nucleotides and produced by hybridizing a test DNA molecule under stringent conditions with (a) a DNA molecule encoding a PRO533 polypeptide fragment having the sequence of nucleic acid residues from 1 to about 826 and about 1199 to 2137, inclusive of Figure 2 (SEQ ID NO: 2), or (b) the complement of the DNA molecule of (a), and, if the DNA molecule has at least about an 80 % sequence identity, prefereably at least about an 85% sequence identity, more preferably at least about a 90% sequence identity, most preferably at least about a 95% sequence identity to (a) or (b), isolating the test DNA molecule. Such nucleic acid molecules can act as antisense molecules of the amplified genes, or as antisense primers in the amplification reactions. Furthermore, such sequences can be used as part of ribozyme and/or triple helix sequences, which in turn, may be used in the regulation of PRO533 expression.

In a specific aspect, the invention provides an isolated nucleic acid molecule comprising DNA encoding a PRO533 polypeptide, with or without the N-terminal signal sequence and/or the initiating methionine, and its soluble, *i.e.* transmembrane domain deleted or inactivated variants, or is complementary to such encoding nucleic acid molecule. The signal peptide has been tentatively identified as extending from amino acid position 1 to about amino acid position 22 in the sequence of Figure 1 (SEQ ID NO: 1). N-myristolylation sites have been tentatively identified as being present at amino acid residues G15, G54, G66 and G201, while a prokaryotic membrane lipoprotein lipid attachment siteis believed to exist at amino acid residues Y48 to C58.

In another aspect as defined in the claims the invention concerns an isolated nucleic acid molecule comprising (a) DNA encoding a polypeptide scoring at least about 80% positives, preferably at least about 85% positives, more preferably at least about 90% positives, most preferably at least about 95% positives when compared with the amino acid sequence of residues 23 to about 216, inclusive of Figure 1 (SEQ ID NO: 1), or (b) the complement of the DNA of (a).

In another embodiment as defined in the claims the invention provides a vector comprising DNA encoding PRO533 or its variants. The vector may comprise any of the isolated nucleic acid molecules hereinabove defined. A host cell comprising such a vector is also provided. By way of example, the host cells may be CHO cells, *E. coli,* or yeast. A process for producing PR0533 polypeptides is further provided and comprises culturing host cells under conditions suitable for expression of PRO533 and recovering PRO533 from the cell culture.

In another embodiment as defined in the claims the invention provides isolated PRO533 polypeptide encoded by any of the isolated nucleic acid sequences hereinabove defined. In a specific aspect, the invention provides isolated native sequence PRO533 polypeptide, which in one embodiment, includes an amino acid sequence comprising residues 23 to 216 of Figure 1 (SEQ ID NO:1). Native PRO533 polypeptides with or without the native signal sequence (amino acids 1 to 22 in Figure 1), and with or without the iniating methionine are specifically included. Alternatively, the invention provides a PR0533 polypeptide encoded by the nucleic acid deposited under accession number ATCC209480.

In another aspect, the invention concerns an isolated PRO533 polypeptide, comprising an amino acid sequence having at least about 80% sequence identity, preferably at least about 85% sequence identity, more preferably at least about 90% sequence identity, most preferably at least about 95% sequence identity to the sequence of amino acid residues 23 to about 216, inclusive of Figure 1 (SEQ ID NO:1).

In a further aspect, the invention concerns an isolated PRO533 polypeptide, comprising an amino acid sequence scoring at least about 80% positives, preferably at least about 85% positives, more preferably at least about 90% positives, most preferably at least about 95% positives when compared with the amino acid sequence of residues 23 to 216 of Figure 1 (SEQ ID NO: 1).

In yet another aspect as defined in the claims the invention concerns an isolated PRO533 polypeptide, comprising the sequence of amino acid residues 23 to about 216, inclusive of Figure 1 (SEQ ID NO: 1), or a fragment thereof sufficient to provide a binding site for an anti-PR0533 antibody. Preferably, the PRO533 fragment retains a qualitative biological activity of a native PRO533 polypeptide.

In a still further aspect as defined in the claims the invention provides a polypeptide produced by (i) hybridizing a test DNA molecule under stringent conditions with (a) a DNA molecule encoding a PRO533 polypeptide having the sequence of amino acid residues from about 23 to about 216, inclusive of Figure 1 (SEQ ID NO: 1), or (b) the complement of the DNA molecule of (a), and if the test DNA molecule has at least about an 80% sequence identity, preferably at least about an 85% sequence identity, more preferably at least about a 90% sequence identity, most preferably at least about a 95% sequence identity to (a) or (b), (ii) culturing a host cell comprising the test DNA molecule under conditions suitable for expression of the polypeptide, and (iii) recovering the polypeptide from the cell culture.

In another embodiment as defined in the claims the invention provides chimeric molecules comprising a PRO533 polypeptide fused to a heterologous polypeptide or amino acid sequence. An example of such a chimeric molecule comprises a PRO533 polypeptide fused to an epitope tag sequence or a Fc region of an immunoglobulin.

In another embodiment, as defined in the claims the invention provides an antibody which specifically binds to PRO533 polypeptide. Optionally, the antibody is a monoclonal antibody. In one aspect, the antibody induces death of a cell overexpressing a PRO533 polypeptide. In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a single-chain antibody, or an anti-idiotypic antibody.

Also disclosed is a method of identifying agonists or antagonists of a native PRO533 polypeptide, comprising contacting a candidate compound with PRO533 under conditions and for a time sufficient to allow these two components to interact. In a specific aspect, either the candidate compound or the PRO533 polypeptide is immobilized on a solid support. In another aspect, the non-immobilized component carries a detectable label.

In another embodiment as defined in the claims the present invention concerns a method for determining the presence of a PR0533 polypeptide comprising exposing a cell suspected of containing PRO533 to an anti-PRO533 antibody and determining binding of the antibody to the cell.

Also disclosed is a method of diagnosing tumor(s) in a mammal, comprising detecting the level of expression of a gene encoding PR0533: (a) in a test sample of tissue cells obtained from the mammal, and (b) in a control sample of known normal tissue cells of the same cell type, wherein a higher expression level in the test sample indicates the presence of tumor in the mammal from which the test tissue cells were obtained.

Also disclosed is a method of diagnosing tumor in a mammal, comprising (a) contacting an anti-PRO533 antibody with a test sample of tissue cells obtained from the mammal, and (b) detecting the formation of a complex between the anti-PRO533 antibody and the PRO533 polypeptide in the test sample. The detection may be qualitative or quantitative, and may be performed in comparison with monitoring the complex formation in a control sample of known normal tissue cells of the same cell type. The antibody preferably carried a label. Complex formation can be monitored, for example, by light microscopy, flow cytometry, fluorimetry, or other techniques known in the art.

Also disclosed is a cancer diagnostic kit, comprising an anti-PR0533 antibody and a carrier (*e.g.*, buffer) in suitable packaging. The kit preferably contains instructions for using the antibody to detect PRO533.

Also disclosed is a method for inhibiting the growth of tumor cells comprising exposing a cell which overexpresses a PRO533 polypeptide to an effective amount of an agent inhibiting the expression and/or activity or PR0533. The agent is preferably an anti-PRO533 antibody, a small organic and inorganic molcule, peptide, phosphopeptide, antisense or ribozyme molecule, or a triple helix molecule. In a specific aspect, the agent (e.g., anti-PRO533 antibody) induces cell death. In a further aspect, the tumor cells are further exposed to radiation treatment and/or a cytotoxic or chemotherapeutic agent.

Also disclosed is an article of manufacture, comprising:
a container;
a label on the container; and
a composition comprising an active agent contained within the container;
wherein the composition can be used for treating conditions characterized by overexpression of PRO533, and the active agent in the composition is an agent inhibiting the expression and/or activity of the PRO533 polypeptide. In a preferred aspect, the active agent is an anti-PRO533 antibody.

### BRIEF DESCRIPTION OF THE DRAWINGS

Figure 1 shows the derived amino acid sequence of a native sequence PRO533. The signal peptide has been tentatively identified as extending from amino acid position I to about amino acid position 22 in the sequence of Figure 1 (SEQ ID NO: 1). N-myristolylation sites have been tentatively identified as being present at amino acid residues G15. G54. G66 and G201, while a prokaryotic membrane lipoprotein lipid attachment siteis believed to exist at amino acid residues Y48 to C58.
Figure 2 shows the nucleotide sequence of a cDNA encoding native sequence PR0533 (DNA49435).
The start codon is believed to be present at nucleotide residues 464-466, with the stop codon at residues 1112-1114.

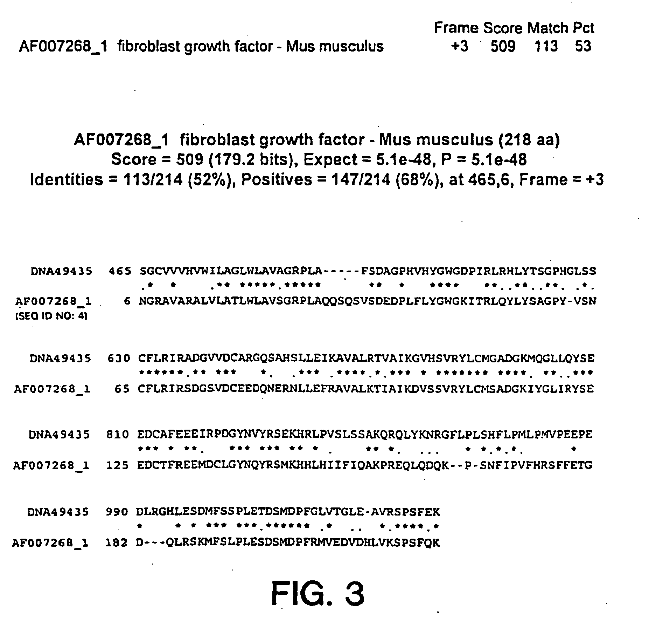

Figure 3 is an alignment describing the Blast-2 score, match and percent identity between amino acid residues 3 to 216 of a native sequence PRO533 protein encoded by DNA49435 with residues 6 to 218 of AF00728_1. a fibroblast growth factor sequence (FGF-15).

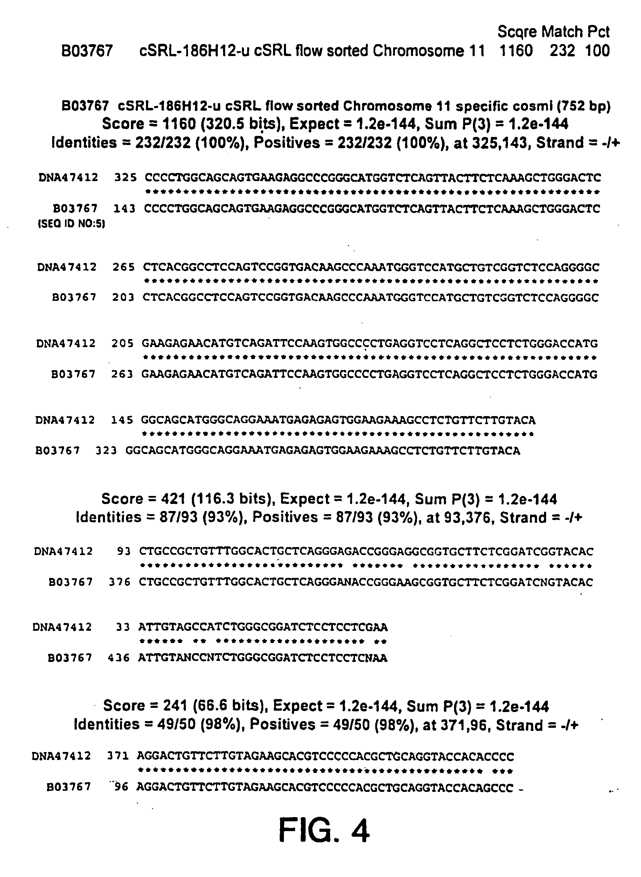

Figure 4 describes the Blast score, match and percent identity of DNA49435 with B03767. a genomic clone prepared from chromosome 11.
Figure 5 shows the double stranded fromDNA 47412 (GenBank AA220994) along with the nucleotide sequence and hybridization regions of the PCR oligos (FGF15.f, FGF15.p2, FGF15.r) which can be used to isolate DNA49435.
Figure 6 shows the entire sequence of AF007268, an FGF-15 EST sequence which was used to search various public sequence databases (*e.g*., GenBank. Dayhoff, etc.) in the process of isolating PR0533.
Figure 7 is a Northern blot of DNA49435 in various cancer cell lines. Shown in lanes 1-8 are polyA mRNA from the following cancer cell lines: (1) promyelocytic leukemia HL-60; (2) Hela S3; (3) chronic myelogenous leukemia K-562; (4) lymphoblastic leukemia MOLT-4; (5) Burkitt's lymphoma Raji; (6) colorectal adenocarcinoma SW480; (7) lung carcinoma A549; (8) melanoma G361.
Figure 8 indicates the results of *in situ* analysis of DNA49435 in human fetal (E12-E16 weeks) and adult tissues. Shown are corresponding bright and dark field images of: A.B- fetal low limb cartilage; C,D-fetal retina; E.F- fetal skin; G.H- adult gall bladder. Areas of expression are indicated by arrow.
Figure 9 is a Western blot indicating the binding of PRO533 to FGF receptor 4. FGF1(A) or PRO533 (FGF-19) expressed with either N-terminal gD epitope tag (B) or C-terminal His8 epitope tag (C) were tested for binding to receptor-Fc fusion proteins. Specific binding components are as indicated above lanes 1-8. Lane 9 contains FGF loaded directly onto the gel for comparison. Molecular weight markers are indicated on the left side of the gel for comparison.
Figure 10 is a Western blot indicating the dependence of PRO533 (FGF-19) binding on heparin. N-terminal gD-tagged PR0533 (FGF-19) was allowed to interact with FGFR4-Fc in the presence of the indicated concentrations of heparin.
Figure 11 is an alignment the PR0533 sequence encoded by DNA49435 (FGF-19) with other members of the FGF family.

### DETAILED DESCRIPTION OF THE PREFERRED EMBODIMENTS

### 1. Definitions

The phrases "gene amplification" and "gene duplication" are used interchangeably and refer to a process by which multiple copies of a gene or gene fragment are formed in a particular cell or cell line. The duplicated region (a stretch of amplified DNA) is often referred to as "amplicon." Usually, the amount of the messenger RNA (mRNA) produced. *i*.*e*. the level of gene expression, also increases in the proportion of the number of copies made of the particular gene expressed.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (*e.g*. cancer) treatment, a therapeutic agent may directly decrease the pathology of tumor cells, or render the tumor cells more susceptible to treatment by other therapeutic agents, *e*.*g*. radiation and/or chemotherapy.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, *etc*.

"Mammal" for purposes of treatment refers to any animal classified as a mammal, including humans, domestic and farm animals, and zoo, sports, or pet animals, such as dogs, horses, cats, cattle, *etc*. Preferably, the mammal is human.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids: antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitoLor sorbitol; salt-forming counterions such as sodium: and/or nonionic surfactants such as TWEEN^{™}, polyethylene glycol (PEG), and PLURONICS^{™}.

Administration "in combination with" one or more further therapeutic agents includes simultaneous (concurrent) and consecutive administration in any order.

The term "cytotoxic agent" as used herein refers to a substance that inhibits or prevents the function of cells and/or causes destruction of cells. The term is intended to include radioactive isotopes (*e*.*g*. I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g. paclitaxel (Taxol, Bristol-Myers Squibb Oncology, Princeton. NJ), and doxetaxel (e.g. Taxotere^{®}, Rhône-Poulenc Rorer. Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or in *vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G I arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G I also spill over into S-phase arrest, for example, DNA alkylating agents such as tamoxifen, prednisone, dacarbazine, mechlorethamine, cisplatin, methotrexate, 5-fluorouracil, and ara-C. Further information can be found in *The Molecular Basis of Cancer,* Mendelsohn and Israel, eds., Chapter 1, entitled "Cell cycle regulation, oncogens, and antineoplastic drugs" by Murakami *et al.* (WB Saunders: Philadelphia, 1995), especially p. 13.

"Doxorubicin" is an athracycline antibiotic. The full chemical name of doxorubicin is (8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5.12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone. N-methionyl human growth hormone, and bovine growth hormone; parathyroid hormone: thyroxine; insulin: proinsulin; relaxin; prorelaxin; glycoprotein hormones such as follicle stimulating hormone (FSH), thyroid stimulating hormone (TSH), and luteinizing hormone (LH); hepatic growth factor: fibroblast growth factor: prolactin; placental lactogen; tumor necrosis factor-α and -β; mullerian-inhibiting substance: mouse gonadotropin-associated peptide; inhibin; activin; vascular endothelial growth factor: integrin: thrombopoietin (TPO): nerve growth factors such as NGF-β; platelet-growth factor; transforming growth factors (TGFs) such as TGF-α and TGF-β: insulin-like growth factor-I and -II; erythropoietin (EPO): osteoinductive factors; interferons such as interferon-α, -β, and -γ; colony stimulating factors (CSFs) such as macrophaee-CSF (M-CSF); gmnulocvte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12; a tumor necrosis factor such as TNF-α or TNF-β: and other polypeptide factors including LIF and kit ligand (KL). As used herein, the term cytokine includes proteins from natural sources or from recombinant cell culture and biologically active equivalents of the native sequence cytokines.

The terms "PR0533 polypeptide". "PR0533 protein" and "PR0533" when used herein encompass native sequence PRO533 and PRO533 variants (which are further defined herein). The PRO533 may be isolated from a variety of sources, such as from human tissue types or from another source, or prepared by recombinant and/or synthetic methods.

A "native sequence PRO533" comprises a polypeptide having the same amino acid sequence as a PRO533 derived from nature. Such native sequence PR0533 can be isolated from nature or can be produced by recombinant and/or synthetic means. The term "native sequence PR0533" specifically encompasses naturally-occurring truncated or secreted forms (*e*.*g*., an extracellular domain sequence), naturally-occurring variant forms (*e.g.*, alternatively spliced forms) and naturally-occurring allelic variants of the PRO533. In one embodiment of the invention, the native sequence PR0533 is a mature or full-length native sequence PR0533 comprising amino acids 23 to 216 (alternatively 1 to 216) of Figure 1 (SEQ ID NO:1).

"PRO533 variant" means anything other than a native sequence PRO533 which is an active PRO533, as defined below, having at least about 80% amino acid sequence identity with the amino acid sequence of residues 23 to 216 of the PR0533 polypeptide having the deduced amino acid sequence shown in Figure 1 (SEQ ID NO:1). Such PRO533 variants include, for instance, PRO533 polypeptides wherein one or more amino acid residues are added, or deleted, at the N- or C-terminus, as well as within one or more internal domains, of the sequence of Figure 1 (SEQ ID NO:1). Ordinarily, a PR0533 variant will have at least about 80% amino acid sequence identity, more preferably at least about 85% amino acid sequence identity, even more preferably at least about 90% amino acid sequence identity, and most preferably at least about 95% sequence identity with the amino acid sequence of residues 23 to 216 of Figure 1 (SEQ ID NO:1).

"Percent (%) amino acid sequence identity" with respect to the PR0533 sequences identified herein is defined as the percentage of amino acid residues in a candidate sequence that are identical with the amino acid residues in the PRO533 sequence, after aligning the sequences and introducing gaps, if necessary, to achieve the maximum percent sequence identity, and not considering any conservative substitutions as part of the sequence identity. The % identity values used herein are generated by WU-BLAST-2 which was obtained from [Altschul et al., Methods in Enzymology, 266: 460-480 (1996); http://blast.wustl/edu/blast/README.html]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched: however, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

The term "positives", in the context of sequence comparison performed as described above, includes residues in the sequences compared that are not identical but have similar properties (e.g. as a result of conservative substitutions). The % value of positives is determined by the fraction of residues scoring a positive value in the BLOSUM 62 matrix divided by the total number of residues in the longer sequence, as defined above.

In a similar manner, "percent (%) nucleic acid sequence identity" with respect to the coding sequence of the PRO533 polypeptides identified herein is defined as the percentage of nucleotide residues in a candidate sequence that are identical with the nucleotide residues in the PRO533 coding sequence. The identity values used herein were generated by the BLASTN module of WU-BLAST-2 set to the default parameters, with overlap span and overlap fraction set to 1 and 0.125, respectively.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO533 natural environment will not be present. Ordinarily, however, isolated polypeptide will be prepared by at least one purification step.

An "isolated" nucleic acid molecule encoding a PRO533 polypeptide (*e*.*g*., DNA49435) is a nucleic acid molecule that is identified and separated from at least one contaminant nucleic acid molecule with which it is ordinarily associated in the natural source of the PRO533-encoding nucleic acid. An isolated PRO533-encoding nucleic acid molecule is other than in the form or setting in which it is found in nature. Isolated PRO533-encoding nucleic acid molecules (e.g., DNA49435) therefore are distinguished from the PRO533-encoding nucleic acid molecule as it exists in natural cells. However, an isolated nucleic acid molecule encoding a PR0533 polypeptide includes PRO533-encoding nucleic acid molecules contained in cells that ordinarily express PRO533 where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide: a promoter or enhancer is operably linked to a coding sequence if it affects the transcription of the sequence: or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers single anti-PRO533 monoclonal antibodies (including agonist, antagonist, and neutralizing antibodies) and anti-PRO533 antibody compositions with polyepitopic specificity. The term "monoclonal antibody" as used herein refers to an antibody obtained from a population of substantially homogeneous antibodies, *i.e.,* the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers. (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C: (2) employ during hybridization a denaturing agent, such as formamide, for example. 50% (v/v) formamide with 0.1% bovine serum albumin/0.1% Ficoll/0.1% polyvinylpyrrolidone/50mM sodium phosphate buffer at pH 6.5 with 750 mM sodium chloride. 75 mM sodium citrate at 42°C; or (3) employ 50% formamide. 5 x SSC (0.75 M NaCl. 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS. and 10% dextran sulfate at 42°C. with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (*e*.*g*., temperature, ionic strength and %SDS) less stringent that those described above. An example of moderately stringent conditions is overnight incubation at 37°C in a solution comprising: 20% formamide. 5 x SSC (150 mM NaCl, 15 mM trisodium citrate). 50 mM sodium phosphate (pH 7.6). 5 x Denhardt's solution. 10% dextran sulfate, and 20 mg/mL denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO533 polypeptide fused to a "tag polypeptide". The tag polypeptide has enough residues to provide an epitope against which an antibody can be made, yet is short enough such that it does not interfere with the activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or igG-4 subtypes, IgA (including IgA-1 and IgA-2), IgE, IgD or IgM.

"Active" or "activity" for the purposes herein refers to form(s) of PRO533 which retain the biologic and/or immunologic activities of native or naturally-occurring PRO533. Preferably, activity refers to the ability to bind with high affininty to fibroblast growth factor receptor 4. (FGFR4).

"Biological activity" in the context of an antibody or another molecule that can be identified by the screening assays disclosed herein (*e*.*g*., an organic or inorganic small molecule, peptide, etc.) is used to refer to the ability of such molecules to bind or complex with the polypeptides encoded by the amplified genes identified herein, or otherwise interfere with the interaction of a target tumor cell. Another preferred biological activity is cytotoxic activity resulting in the death of the target tumor cell.

The phrase "immunological property" means immunological cross-reactivity with at least one epitope of a PRO533 polypeptide. "Immunological cross-reactivity" as used herein means that the candidate polypeptide is capable of competively inhibiting the qualitative biological activity of a PRO533 polypeptide having this activity with the polyclonal antisera raised against the known active PR0533 polypeptide. Such antisera are prepared in conventional fashion by injecting goats or rabbits, for example, subcutaneously with the known active analogue in complete Freund's adjuvant, followed by booster intraperitoneal or subcutaneous injection in incomplete Freunds. The immunological cross-reactivity preferably is "specific", which means that the binding affinity of the immunologically cross-reactive molecule *(e.g.* antibody) identified, to the corresponding PRO533, polypeptide is significantly higher (preferably at least about 2-times, more preferably at least about 4-times, even more preferably at least about 8-times, most preferably at least about 8-times higher) than the binding affinity of that molecule to any other known native polypeptide.

The term "antagonist" is used in the broadest sense, and includes any molecule that partially or fully blocks, inhibits, or neutralizes a biological activity of a native PR0533 polypeptide disclosed herein. In a similar manner, the term "agonist" is used in the broadest sense and includes any molecule that mimics a biological activity of a native PR0533 polypeptide disclosed herein. Suitable agonist or antagonist molecules specifically include agonist or antagonist antibodies or antibody fragments, fragments or amino acid sequence variants of native PR0533 polypeptides, peptides, small organic molecules, *etc*.

A "small molecule" is defined herein to have a molecular weight below about 500 daltons.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (*e.g.* bispecific antibodies) formed from at least two intact antibodies, and antibody fragments so long as they exhibit the desired biological activity.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al., NIH Publ. No.91-3242, Vol. I. pages 647-669 (1991)). The constant domains are not involved directly in binding an antibody to an antigen, but exhibit various effector functions, such as participation of the antibody in antibody-dependent cellular toxicity.

"Antibody fragments" comprise a portion of an intact antibody, preferably the antigen binding or variable region of the intact antibody. Examples of antibody fragments include Fab, Fab', F(ab')₂, and Fv fragments: diabodies; linear antibodies (Zapata et al., Protein Eng, 8(10):1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linkine antigen.

"Fv" is the minimum antibody fragment which contains a complete antigen-recognition and -binding site. This region consists of a dimer of one heavy- and one tight-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CHI) of the heavy chain. Fab fragments differ from Fab fragments by the addition of a few residues at the carboxy terminus of the heavy chain CHI domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE. IgG, and IgM, and several of these may be further divided into subclasses (isotypes), *e.g.,* IgG 1, IgG2, IgG3, IgG4, IgA, and IgA2. The heavy-chain constant domains that correspond to the different classes of immunoglobulins are called α, δ, ∈, γ, and µ, respectively. The subunit structures and three-dimensional configurations of different classes of immunoglobulins are well known.

The term "monoclonal antibody" as used herein refers to an antibody obtained from a population of substantially homogeneous antibodies. *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 [1975], or may be made by recombinant DNA methods (see. *e*.*g*., U.S. Patent No. 4.816,567). The "monoclonal antibodies" may also be isolated from phage antibody libraries using the techniques described in Clackson et al., Nature,352:624-628 [1991] and Marks et al., J. Mol. Biol.,222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 [1984]).

"Humanized" forms of non-human (*e*.*g*., murine) antibodies are chimeric immunoglobulins, immunoglobulin chains or fragments thereof (such as Fv, Fab, Fab'. F(ab')₂ or other antigen-binding subsequences of antibodies) which contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 [1988]; and Presta, Curr. Op. Struct. Biol., 2:593-596 (1992). The humanized antibody includes a PRIMATIZED^{™}antibody wherein the antigen-binding region of the antibody is derived from an antibody produced by immunizing macaque monkeys with the antigen of interest.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv see Pluckthun in The Pharmacology of Monoclonal Antibodies. vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example. EP 404.097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzyme, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (*e*.*g*. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (*e*.*g*., controlled pore glass), polysaccharides (*e*.*g*., agarose), polyacrylamides, polystyrene, polyvinyl alcohol and silicones. In certain embodiments, depending on the context, the solid phase can comprise the well of an assay plate: in others it is a purification column (*e*.*g*., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (*e*.*g*., PRO533 polypeptide or an antibody thereto and optionally a chemotherapeutic agent) to a mammal. The components of the liposome are commonly arranged in a bilayer formation, similar to the lipid arrangement of biological membranes.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i*.*e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2. IgG-3. or IgG-4 subtypes. IgA (including IgA-1 and IgA-2), IgE, IgD or IgM.

### 2. Compositions and Methods of the Invention

### a. Full-length PRO533

The present invention provides newly identified and isolated nucleotide sequences encoding polypeptides referred to in the present application as PRO533 (UNQ334). In particular, cDNA encoding a PRO533 polypeptide has been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by DNA49435 as well as all further native homologues and variants included in the foregoing definition of PRO533, will be referred to as "PRO533", regardless of their origin or mode of preparation.

Using WU-BLAST2 sequence alignment computer programs, it has been found that a full-length native sequence PRO533 (shown in Figure 1 and SEQ ID NO:1) has about a 53% amino acid sequence identity with murine fibroblast growth factor - 15. Accordingly, it is presently believed that PRO533 disclosed in the present application is a newly identified member of the fibroblast growth factor family and may possess activitity typical of such polypeptides. Preferably, such activity includes the ability to bind with high affinity selectively to FGFR4.

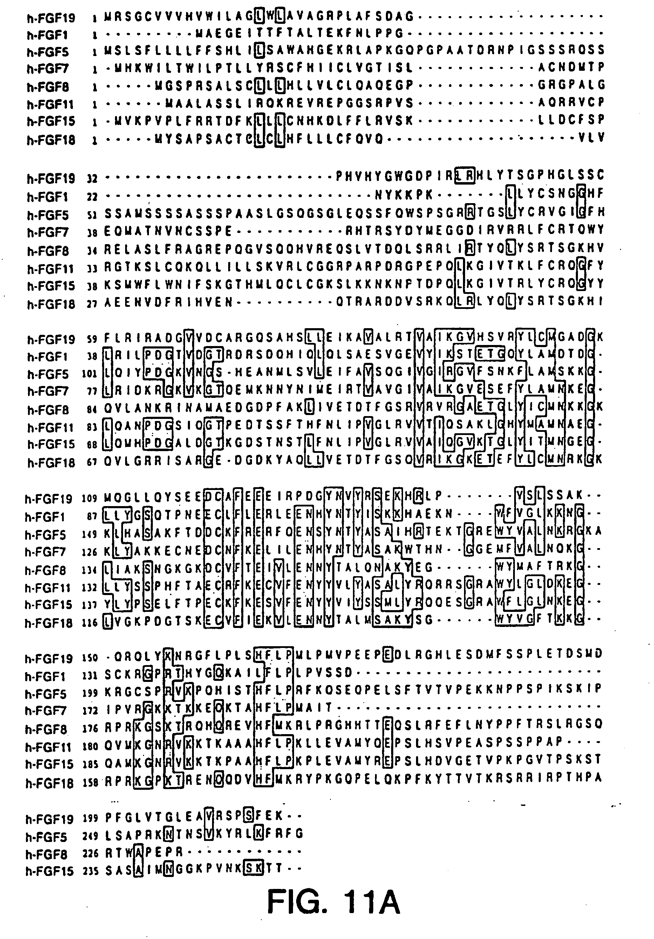

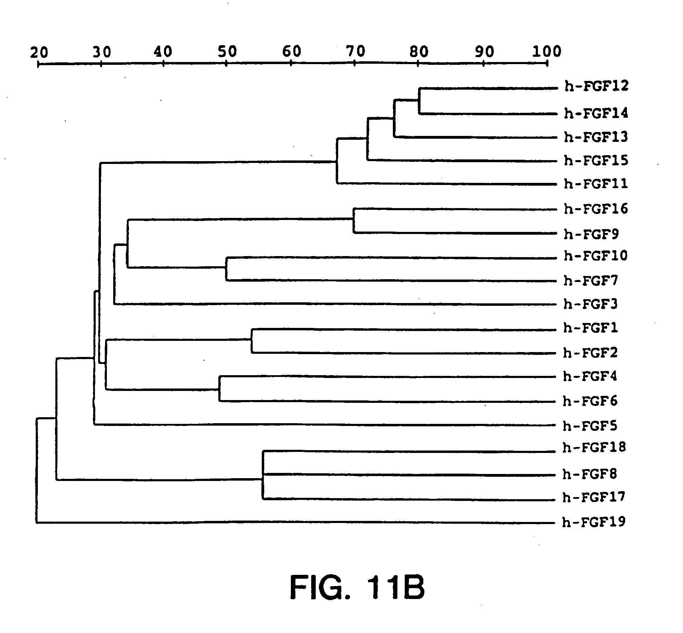

DNA49435 was isolated from a human fetal retina library. The cDNA encoding PRO533 depicted in Fie. 2 is 2137 base pairs in length and contains a predicted open reading frame of 216 amino acids. Blast comparisons with GenBank indicated that this represents a novel protein, and that it has significant homology to other known members of the FGF family. Alignment with other members of the FGF family indicates that this new member is somewhat distinctly related to other members of the family (=20% identity, see Fig. 11), although it possesses some homology to other FGF along the length of the predicted protein. While several members of the FGF family lack classical signal sequences. DNA49435, however, does possess such a sequence from about amino acid residues 1-22.

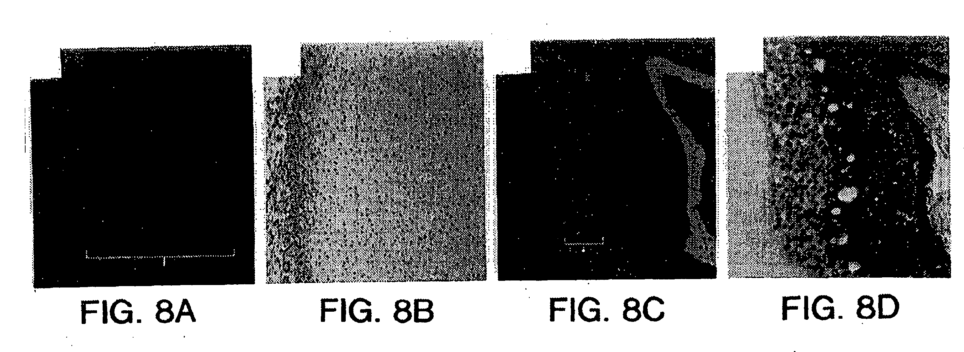

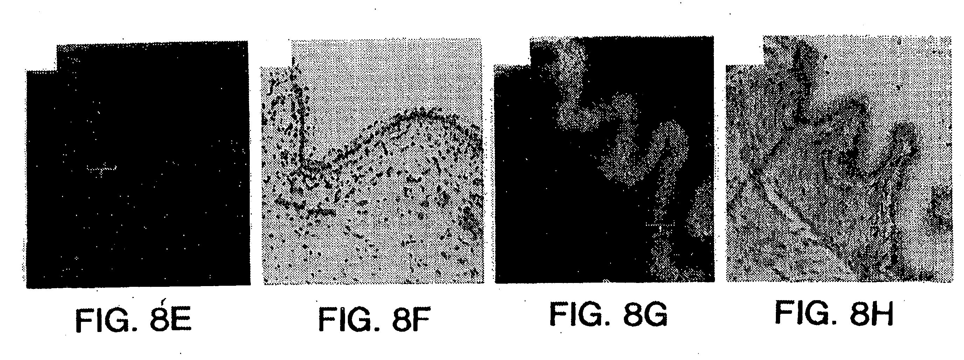

The chromosomal location was determined by radiation hybrid mapping to be chromosome 11 q13.1. *In situ* analysis showed expression over fetal skin, cartilage, the inner aspect of the fetal retina, and adult gall bladder epithilium (Figure 8) as well as fetal small intestine, placental villi and umbilical cord (not shown). DNA49435 was not clearly detectable by multiple tissue northern blot analysis but was detectable by RT-PCR in several tissues (not shown). Interestingly, a survey of several cancer cell lines revealed that colon adenocarcinoma line SW480 displayed markedly elevated levels of DNA49435 message

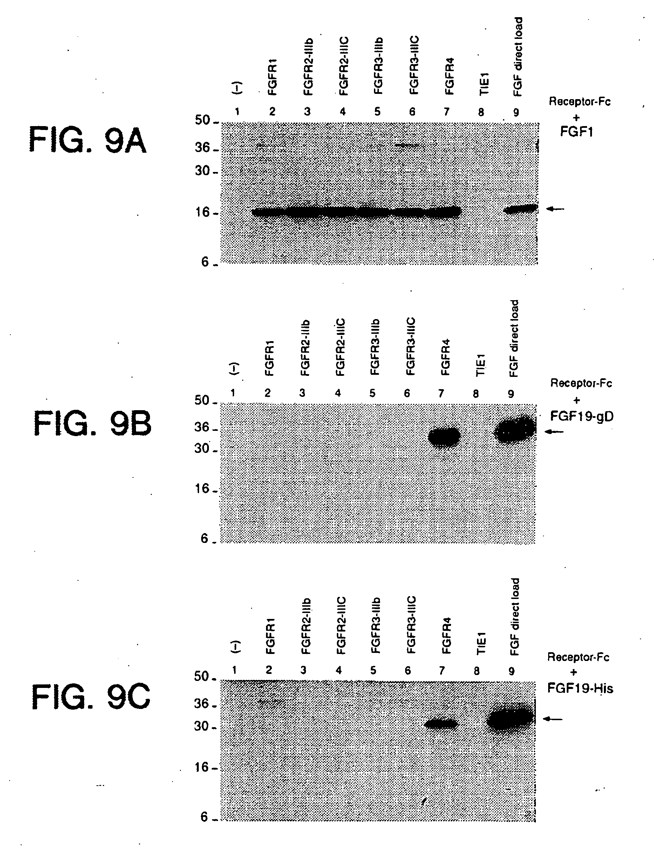

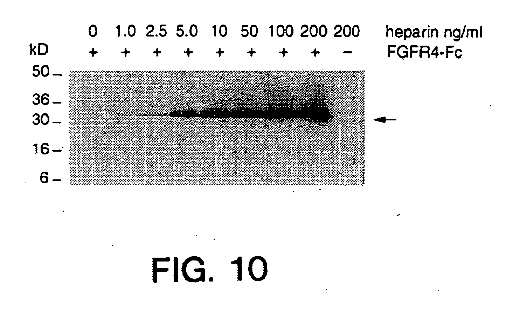

In order to determine if DNA49435 was in fact a ligand for the known FGF receptors, binding studies were conducted. DNA49435 was produced as a recombinant protein with either N-terminal or C-terminal epitope tags. The C-terminal His tagged protein was secreted from baculovirus infected insect cells using the native N-terminous indicating that the protein does in fact contain a functional signal sequence. The N-terminal sequence of purified DNA49435 begins with residue 25, two residues C-terminal of the predicted cleavage location. The extracellular domains (ECD) of the four known FGF receptors (IIIc splice form) were expressed as IgG Fc fusions. DNA49435 bound to FGF4R, but not to the other FGF receptors tested (Figure 9 A-C). N-terminal and C-terminal epitope tagged forms gave similar results with binding only observed with FGFR4. Alternative splice forms differing in the C terminal end of domain 3 of the ECD have been described for FGF receptors 1-3 (IIIb splice forms), Dell, K.R. & Williams, L.T., J. Biol. Chem. 267: 21225-29 (1992); Johnson, D.E. et al., Mol. Cell Biol. 11: 4627-34 (1991); Murgue, B.S. et al., Cancer Res. 54: 5206-11 (1994); Perez-Castro, A.V. et al., Genomics 30: 157-62 (1995). DNA49435 did not appear to bind to either FGFR3 (IIIb) or FGFR2 (IIIb). Binding to FGFR4 was heparin dependent with maximal binding occurring in the presence of 100 ng/ml heparin (Fig. 10). DNA49435 binding could be competed with 100 fold excess FGF-1. known to bind with high affinity to FGF4 (200 pM), but only poorly competed with FGF-2, which binds FGFR4 with lower (2 nM) affinity (not shown). Ornitz. D.M. et al., J. Biol. Chem. 271: 15292-97 (1996). The effect of DNA49435 on cell proliferation was examined with several cell lines including K563. an erythroleukemia cell line previously reported to express FGFR4 [Armstrong, E. et al., Cancer Res., 52: 2004-07 (1992): Partanen, J. et al., Embo. J. 10: 1347-54 (1991), NIH 3T3 fibroblasts, and primary human foreskin fibroblasts. In contrast to FGF-1 and several other FGFs tested, DNA49435 demonstrated little mitogenic activity (not shown).

DNA49435 (FGF-19) is a new member of the FGF family of growth factors. Like the other members for which analysis has been reported. DNA49435 (FGF-19) is a ligand for a member of the FGFR family. The binding specificity of several of the recently described members of the FGF family has yet to be determined. However, for the many members where binding has been examined, binding has not been found to be specific to one FGFR. The sole reported exception is FGF-7 (keratinocyte growth factor. KGF) which appears to solely bind KGFR, a IIIb splice variant of FGFR2. Numerous known FGF members are capable of binding FGFR4 [Ornitz. D.M. et al., J. Biol. Chem. 271: 15292-97; Ron. D. et al., J. Biol Chem. 268: 5388-94 (1993); Vainikka. S. et al., Embo. J. 11: 4273-80 (1992)]; however, each of these FGF also display binding to other FGFR. Binding is of high affinity and requires the presence of heparin. Interestingly, several cell lines including 3T3 fibroblast cell lines and primary human foreskin fibroblasts that have been extensively studied for responsiveness to other FGF members do not display a mitogenic response to FGF-19, underscoring the unique specificity of DNA49435 for FGF4. This result is an agreement with several reports that indicate the signal transduction events elicited by the individual FGFR differ, and suggests that FGFR4 signaling is much less mitogenic. Shaoul, E. et al.. Oncogene 10: 1553-61 (1995); Vainikka, S. et al., J. Biol. Chem. 271: 1270-73 (1996); Vainikka, S. et al.. J Biol. Chem. 269: 18320-26 (1994); Wang, J. et al., Mol. Cell Biol. 14: 181-88 (1994). This relative lack of mitogenicity appears dependent on the intracellular domain as chimeric receptors comprised of the extracellular domain of FGF4 and the intracellular domain of FGFR I induce survival and growth in BaF3 cells whereas FGFR4 does not. Ornitz, D.M. et al., J. Biol. Chem. 271: 15292-97 (1996); Wang, J.K. et al., Mol. Cell Biol. 14: 181-88 (1994). These reports have relied on overexpression of individual receptors in cell lines thought to otherwise lack FGFR as to date there has not been a ligand specific to FGFR4. By comparison, DNA49435 may serve as a novel reagent to enable analysis of FGFR4 function in complex primary cell systems and animal models. Despite the apparent lack of mitogenic activity, there have been numerous reports correlating upregulation or amplification of FGFR4 and a variety of human cancer, particularly breast cancer. Abass, S.A. et al., J Clin. Endocrinol. Metab. 82: 1160-66 (1997), Johnston, C.L. et al., Biochem. J. 306: 609-16 (1995), McLeskey, S.W. et al., Cancer Res. 54: 523-30 (1994), Penault-Llorca, F. et al., Int. J. Cancer 61: 170-76 (1995), Ron, D. et al., J. Biol. Chem. 268: 5388-94 (1993). It has been shown that FGFR4, but not FGFR1-3 is able to mediate a membrane ruffling response that may be relevant to cancer cell motility. Johnston, C.L. et al., Biochem. J. 306: 609-16 (1995). The very high level of DNA49435 message in SW480 colon adenocarcinoma cells reflect involvement of FGFR4 in autocrine signaling.

It is proposed that while relatively specific roles exist for some individual FGF ligands, broader roles are played by.the FGFRs. Mice deficient for FGFR1, or FGFR2 suffer from embryonic lethality. Arman, E. et al., Proc. Natl. Acad Sci. USA 95: 5082-87 (1998); Ciruna, B. et al., Development 124: 2829-41 (1997); Deng, C. et al., Genes Dev. 8: 3045-57 (1994). Mice deficient in FGFR3 display severe defects in skeletal growth as well as inner ear defects and deafness. Colvin, J.S. et al., Nat. Genet. 12: 390-97 (1996): Deng, C. et al., Cell 84: 911-21 (1996). The phenotype of FGFR4 deficient mice has not yet been reported. In contrast, for several FGFs, the null phenotype is mild. FGF-5 deficient mice have long hair, and the rat angora (go) mutation has been shown to be due to a defective FGF-5 allele. Hebert, J. et al., Cell 78: 1017-25 (1994). FGF-6 deficient mice are healthy, but exhibit defects in muscle regeneration. Fiore. F. et al., Int. J. Dev. Biol. 41: 639-42 (1997): Floss. T. et al., Genes Dev. 11: 2040-51 (1997). Even disruption of FGF-7/KGF, a growth factor thought to play a major role in epidermal cell growth and wound heating, resulted in deficient mice which displayed only a minor "matted" hair phenotype resulting from a hair follicle defect. Guo. L. et al., Genes Dev. 10: 165-75 (1996). Understanding the function of the individual FGFs is clearly complicated by the ability to elicit non physiological responses in *in vitro* systems and by the possibility of compensatory effects of other FGF family members in gene disruption experiments. The FGFR seem to have a particularly important role in skeletal development. Human genetic disorders of skeletal or cranial development have been linked to mutations that cause constitutive activation of FGFR1-3. Webster, M.K. & Donoghue, D.J.. Trends Genet. 13: 178-182 (1997), Wilkie, A.O.. Human Mol. Genet. 6: 1647-56 (1997). The expression of DNA49435. a specific ligand for FGFR4. in fetal cartillage suggests a possible role for FGFR4 in cartilage or bone development as well. The chromosomal mapping of FGF-19 to I 1 q13 as well as the exprssion of DNA49435 in cartilage and fetal retina suggests that it is a candidate gene for osteoporosis-pseudoglioma syndrome, a rare disorder defined by osteoporosis, vitreoretinal dysplasia, muscular hypotonia, and ligamentous laxity. Frontali, M. et al., Am. J. Med. Genet. 22: 35-47 (1985); Gong, Y.M. et al. Am. J. Hum. Genet. 59: 146-51 (1996). Johnson. M.L. et al., Am. J. Hum. Genet. 60: 1326-32 (1997), Somer, H.. J. Med. Genet. 25: 543-49 (1988). Two other known members of the FGF family, FGF-3 and FGF-4, also map to I 1 q13, but do not appear to be responsible for this disorder. However, it does appear that 11 q13 is a locus containing a cluster of FGF members.

DNA49435 is most similar (53% identity) to recently described murine FGF-15. McWhiner. J.R. et al., Development 124: 3221-32 (1997). This degree of relatedness is substantially less than the generally observed relatedness between mouse/human FGF ortholog (81%-99% for FGFI-8) but is in general agreement with the relatedness between members of subgroups within the FGF family such as the emerging FGF8/17/18 subfamily (54-63%) or the FGF 11/12/13/14/15 subfamily (66-72%). Murine FGF-15 was identified as a downstream target of the homeostatic selector (Hox) transcription factor Pbx 1 and likely has a role in neural development. As a novel member of the FGF family with expression in several fetal tissues and unusual receptor speicficity, DNA49435 likely has roles in directing developmental patterning, thereby possessing unique therapeutic potential.

### b. PRO533 Variants

In addition to the full-length native sequence PRO533 polypeptides described herein, it is contemplated that PRO533 variants can be prepared. PRO533 variants can be prepared by introducing appropriate nucleotide changes into the PRO533 DNA. and/or by synthesis of the desired PR0533 polypeptide. Those skilled in the art will appreciate that amino acid changes may alter post-translational processes of the PRO533, such as changing the number or position of glycosylation sites or altering the membrane anchoring characteristics.

Variations in the native full-length sequence PR0533 or in various domains of the PR0533 described herein, can be made, for example, using any of the techniques and guidelines for conservative and non-conservative mutations set forth, for instance, in U.S. Patent No. 5,364.934. Variations may be a substitution, deletion or insertion of one or more codons encoding the PR0533 that results in a change in the amino acid sequence of the PR0533 as compared with the native sequence PR0533. Optionally the variation is by substitution of at least one amino acid with any other amino acid in one or more of the domains of the PRO533. Guidance in determining which amino acid residue may be inserted, substituted or deleted without adversely affecting the desired activity may be found by comparing the sequence of the PRO533 with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of 1 to 5 amino acids. The variation allowed may be determined by systematically making insertions, deletions or substitutions of amino acids in the sequence and testing the resulting variants for activity in the *in vitro* assay described in the Examples below.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PR0533 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co.. N.Y.); Chothia, J. Mol. Biol., 150:1 (1976)]. If alanine substitution does not yield adequate amounts of variant, an isoteric amino acid can be used.

### c. Modifications of PRO533

Covalent modifications of PRO533 are included within the scope of this invention. One type of covalent modification includes reacting targeted amino acid residues of a PR0533 polypeptide with an organic derivatizing agent that is capable of reacting with selected side chains or the N- or C- terminal residues of the PRO533. Derivatization with bifunctional agents is useful, for instance, for crosslinking PR0533 to a water-insoluble support matrix or surface for use in the method for purifying anti-PRO533 antibodies, and vice-versa. Commonly used crosslinking agents include, *e.g.,* 1,1-bis(diazoacetyl)-2-phenylethane, glutaraldehyde. N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1.8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton. Proteins: Structure and Molecular Properties, W.H. Freeman & Co., San Francisco. pp. 79-86 (1983)], acetylation of the N-terminal amine, and amidation of any C-terminal carboxyl group.

Another type of covalent modification of the PRO533 polypeptide included within the scope of this invention comprises altering the native glycosylation pattern of the polypeptide. "Altering the native glycosylation pattern" is intended for purposes herein to mean deleting one or more carbohydrate moieties found in native sequence PRO533 (either by removing the underlying glycosylation site or by deleting the glycosylation by chemical and/or enzymatic means), and/or adding one or more glycosylation sites that are not present in the native sequence PRO533. In addition, the phrase includes qualitative changes in the glycosylation of the native proteins, involving a change in the nature and proportions of the various carbohydrate moieties present.

Addition of glycosylation sites to the PRO533 polypeptide may be accomplished by altering the amino acid sequence. The alteration may be made, for example, by the addition of, or substitution by, one or more serine or threonine residues to the native sequence PR0533 (for O-linked glycosylation sites). The PR0533 amino acid sequence may optionally be altered through changes at the DNA level, particularly by mutating the DNA encoding the PRO533 polypeptide at preselected bases such that codons are generated that will translate into the desired amino acids.

Another means of increasing the number of carbohydrate moieties on the PRO533 polypeptide is by chemical or enzymatic coupling of glycosides to the polypeptide. Such methods are described in the art. e.g., in WO 87/05330 published 11 September 1987, and in Aplin and Wriston, CRC Crit. Rev. Biochem., pp. 259-306(1981).

Removal of carbohydrate moieties present on the PRO533 polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PR0533 comprises linking the PR0533 polypeptide to one of a variety of nonproteinaceous polymers. *e*.*g*., polyethylene glycol (PEG), polypropylene glycol, or polyoxyalkylenes, in the manner set forth in U.S. Patent Nos. 4,640.835: 4,496,689: 4,301,144; 4,670,417; 4,791,192 or 4,179,337.

The PRO533 of the present invention may also be modified in a way to form a chimeric molecule comprising PRO533 fused to another, heterologous polypeptide or amino acid sequence.

In one embodiment, such a chimeric molecule comprises a fusion of the PRO533 with a tag polypeptide which provides an epitope to which an anti-tag antibody can selectively bind. The epitope tag is generally placed at the amino- or carboxyl- terminus of the PRO533. The presence of such epitope-tagged forms of the PRO533 can be detected using an antibody against the tag polypeptide. Also, provision of the epitope tag enables the PR0533 to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO533 with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PR0533 polypeptide in place of at least one variable region within an Ig molecule. In a particularly preferred embodiment, the immunoglobulin fusion includes the hinge, CH2 and CH3, or the hinge. CH1, CH2 and CH3 regions of an IgG1 molecule. For the production of immunoglobulin fusions see also US Patent No. 5,428.130 issued June 27. 1995.

### D. Preparation of PRO533

The description below relates primarily to production of PRO533 by culturing cells transformed or transfected with a vector containing PR0533 nucleic acid. It is, of course, contemplated that alternative methods, which are well known in the art, may be employed to prepare PRO533. For instance, the PR0533 sequence, or portions thereof, may be produced by direct peptide synthesis using solid-phase techniques [see, *e.g.,* Stewart et al., Solid-Phase Peptide Synthesis, W.H. Freeman Co.. San Francisco. CA (1969): Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO533 may be chemically synthesized separately and combined using chemical or enzymatic methods to produce the full-length PRO533.

### i. Isolation of DNA Encoding PRO533

DNA encoding PRO533 may be obtained from a cDNA library prepared from tissue believed to possess the PR0533 mRNA and to express it at a detectable level. Accordingly, human PR0533 DNA can be conveniently obtained from a cDNA library prepared from human tissue, such as described in the Examples. The PRO533-encoding gene may also be obtained from a genomic library or by oligonucleotide synthesis.

Libraries can be screened with probes (such as antibodies to the PR0533 or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO533 is to use PCR methodology [Sambrook *et al*., supra; Dieffenbach et al., PCR Primer: A Laboratory Manual (Cold Spring Harbor Laboratory Press. 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined through sequence alignment using computer software programs such as BLAST, BLAST2, ALIGN. DNAstar, and INHERIT which employ various algorithms to measure homology.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### ii. Selection and Transformation of Host Cells

Host cells are transfected or transformed with expression or cloning vectors described herein for PR0533 production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: A Practical Approach, M. Butler, ed. (IRL Press. 1991) and Sambrook *et al*., supra.

Methods of transfection are known to the ordinarily skilled artisan, for example, CaPO₄ and electroporation. Depending on the host cell used, transformation is performed using standard techniques appropriate to such cells. The calcium treatment employing calcium chloride, as described in Sambrook et al., supra, or electroporation is generally used for prokaryotes or other cells that contain substantial cell-wall barriers. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb. Virology, 52:456-457 (1978) can be employed. General aspects of mammalian cell host system transformations have been described in U.S. Patent No. 4.399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g.,* polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31.537): *E. coli* strain W3110 (ATCC 27.325) and K5 772 (ATCC 53,635).

In addition to prokaryotes, eukaryotic microbes such as filamentous fungi or yeast are suitable cloning or expression hosts for PRO533-encoding vectors. *Saccharomyces cerevisiae* is a commonly used lower eukaryotic host microorganism.

Suitable host cells for the expression of glycosylated PR0533 are derived from multicellular organisms. Examples of invertebrate cells include insect cells such as Drosophila S2 and Spodoptera Sf9. as well as plant cells. Examples of useful mammalian host cell lines include Chinese hamster ovary (CHO) and COS cells. More specific examples include monkey kidney CV1 line transformed by SV40 (COS-7. ATCC CRL 1651): human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)): mouse sertoli cells (TM4, Mather. Biol. Reprod., 23:243-251 (1980)): human lung cells (W138, ATCC CCL 75); human liver cells (Hep G2, HB 8065): and mouse mammary tumor (MMT 060562. ATCC CCL51). The selection of the appropriate host cell is deemed to be within the skill in the art.

### iii. Selection and Use of a Replicable Vector

The nucleic acid *(e.g..* cDNA or genomic DNA) encoding PR0533 may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general. DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PR0533 may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO533-encoding DNA that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, *e.g.,* the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus. VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins. *e.g.,* ampicillin, neomycin, methotrexate, or tetracycline. (b) complement auxotrophic deficiencies, or (c) supply critical nutrients not available from complex media, *e.g.,* the gene encoding D-alanine racemase for *Bacilli.*

An example of suitable selectable markers for mammalian cells are those that enable the identification of cells competent to take up the PRO533-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl, Acad. Sci. USA, 77:4216 (1980). A suitable selection gene for use in yeast is the *trp*I gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*l gene provides a selection marker for a mutant strain of yeast lacking the ability to grow in tryptophan, for example, ATCC No. 44076 or PEP4-1 (Jones, Genetics, 85:12 (1977)].

Expression and cloning vectors usually contain a promoter operably linked to the PRO533-encoding nucleic acid sequence to direct mRNA synthesis. Promoters recognized by a variety of potential host cells are well know. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980): EP 36,776], and hybrid promoters such as the tac promoter [deBoer et al., Proc. Natl. Acad. Sci USA, 80:21-25 (1983)]. Promoters for use in bacterial systems also will contain a Shine-Dalgarno (S.D.) sequence operably linked to the DNA encoding PRO533.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968): Holland. Biochemistry, 17:4900 (1978)], such as enolase, glyceraldehyde-3-phosphate dehydrogenase, hexokinase, pyruvate decarboxylase, phosphofructokinase, glucose-6-phosphate isomerase. 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2. isocytochrome C. acid phosphatase, degradative enzymes associated with nitrogen metabolism, metallothionein, glyeeraldehyde-3-phosphate dehydrogenase, and enzymes responsible for maltose and galactose utilization. Suitable vectors and promoters for use in yeast expression are further described in EP 73.657.

PRO533 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters. *e*.*g*., the actin promoter or an immunoglobulin promoter, and from heat-shock promoters, provided such promoters are compatible with the host cell systems.

Transcription of a DNA encoding the PR0533 by higher eukaryotes may be increased by inserting an enhancer sequence into the vector. Enhancers are cis-acting elements of DNA. usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO533 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO533.

Still other methods, vectors, and host cells suitable for adaptation to the synthesis of PRO533 in recombinant vertebrate cell culture are described in Gething et al., Nature, 293:620-625 (1981); Mantei et al., Nature, 281:40-46 (1979); EP 117,060; and EP 117,058.

### iv. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes. RNA duplexes, and DNA-RNA hybrid duplexes or DNA-protein duplexes. The antibodies in tum may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO533 polypeptide or against a synthetic peptide based on the DNA sequences provided herein or against exogenous sequence fused to PRO533 DNA and encoding a specific antibody epitope.

### v. Purification of Polypeptide

Forms of PRO533 may be recovered from culture medium or from host cell lysates. If membrane-bound, it can be released from the membrane using a suitable detergent solution (e.g. Triton-X 100) or by enzymatic cleavage. Cells employed in expression of PRO533 can be disrupted by various physical or chemical means, such as freeze-thaw cycling, sonication, mechanical disruption, or cell lysing agents.

It may be desired to purify PR0533 from recombinant cell proteins or polypeptides. The following procedures are exemplary of suitable purification procedures: by fractionation on an ion-exchange column; ethanol precipitation: reverse phase HPLC: chromatography on silica or on a cation-exchange resin such as DEAE: chromatofocusing; SDS-PAGE: ammonium sulfate precipitation; gel filtration using, for example, Sephadex G-75; protein A Sepharose columns to remove contaminants such as IgG; and metal chelating columns to bind epitope-tagged forms of the PRO533. Various methods of protein purification may be employed and such methods are known in the art and described for example in Deutscher, Methods in Enzymology, 182(1990); Scopes, Protein Purification: Principles and Practice, Springer-Verlag, New York (1982). The purification step(s) selected will depend, for example, on the nature of the production process used and the particular PRO533 produced.

### E. Uses for PRO533 and/or anti-PRO533 antibodies

### I. General Uses for PRO533

Nucleotide sequences (or their complement) encoding PRO533 have various applications in the art or molecular biology, including uses as hybridization probes, in chromosome and gene mapping and in the generation of anti-sense RNA and DNA. PRO533 nucleic acid will also be useful for the preparation of PRO533 polypeptides by the recombinant techniques described herein.

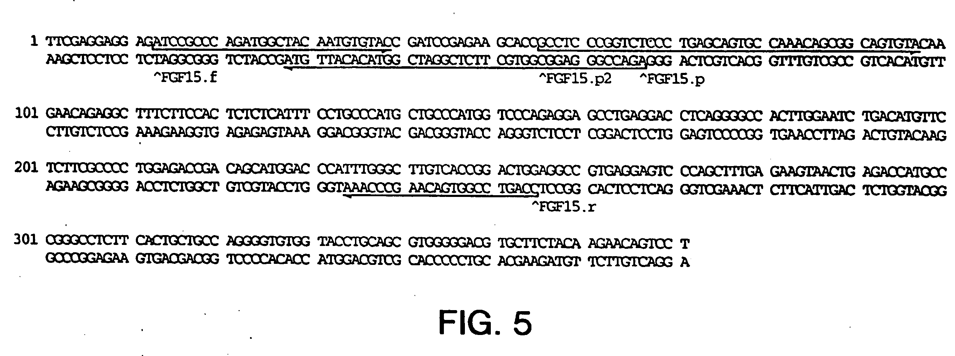

The full-length native PRO533 (SEQ ID NO:1) gene, or portions thereof, may be used as hybridization probes for a cDNAlibrary to isolate the full-length gene or to isolate still other genes (for instance, those encoding naturally-occurring variants of PR0533 or PRO533 from other species) which have a desired sequence identity to the PRO533 disclosed in Fig. 1 (SEQ ID NO: 1). Optionally, the length of the probes will be about 20 to about 80 bases. Preferably the length is from about 20 to about 50 bases. The hybridization probes may be derived from the nucleotide sequence of SEQ ID NO: 1 or from genomic sequences including promoters, enhancer elements and introns of native sequence PRO533. By way of example, a screening method will comprise isolating the coding region of the PRO533 gene using the known DNA sequence to synthesize a selected probe of about 40 bases. Additionally, the probes described in Fig. 5 may also be used as hybridization probes. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the PRO533 gene of the present invention can be used to screen libraries of human cDNA, genomic DNA or mRNA to determine to which members of such libraries the probe hybridizes. Hybridization techniques are described in further detail in the Examples below.

The probes may also be employed in PCR techniques to generate a pool of sequences for identification of closely related PRO533 sequences.

Nucleotide sequences encoding a PR0533 can also be used to construct hybridization probes for mapping the gene which encodes PRO533 and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

As PR0533 has been shown to bind the FGF4 receptor (FGFR4), it can be used in assays to identify other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Screening assays can be designed to find lead compounds that mimic the biological activity of a native PR0533 or a receptor for PRO533. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode PRO533 or its modified forms can also be used to generate either transgenic animals or "knock out" animals which, in turn, are useful in the development and screening of therapeutically useful reagents. A. transgenic animal (*e*.*g*., a mouse or rat) is an animal having cells that contain a transgene, which transgene was introduced into the animal or an ancestor or the animals at a prenatal, *e.g*., an embryonic stage. A transgene is a DNA which is integrated into the genome of a cell from which a trangenic animal develops. In one embodiment, cDNA encoding PRO533 can be used to clone genomic DNA encoding PRO533 in accordance with established techniques and the genomic sequences used to generate transgenic animals that contain cells which express DNA encoding PRO533. Methods for generating transgenic animals, particularly animals such as mice or rats, have become conventional in the art and are described, for example, in U.S. patent nos. 4,736,866 and 4,870,009. Typically, particular cells would be targeted for PR0533 transgene incorporation with tissue-specific enhancers. Transgenic animals that include a copy of a transgene encoding PR0533 introduced into the germ line of the animal at an embryonic stage can be used to examine the effect of increased expression of DNA encoding PR0533. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Nucleic acid encoding the PR0533 polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes in *vivo*. It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83, 4143-4146 [1986]). The oligonucleotides can be modified to enhance their uptake, *e.g.* by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, *etc.* The currently preferred *in vivo* gene transfer techniques include transfection with viral (typically retroviral) vectors and viral coat protein-liposome mediated transfection (Dzau *et al*., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, *etc.* Where liposomes are employed, proteins which bind to a cell surface membrane protein associated with endocytosis may be used for targeting and/or to facilitate uptake, *e*.*g*. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem, 262, 4429-4432 (1987); and Wagner et al., Proc. Natl. Acad. Sci. USA 87, 3410-3414 (1990). For review of gene marking and gene therapy protocols see Anderson et al., Science 256, 808-813 (1992).

The anti-PRO533 antibodies of the invention have various utilities. For example, anti-PRO533 antibodies may be used in diagnostic assays for PRO533, *e.g.*, detecting its expression in specific cells, tissues, or serum. Various diagnostic assay techniques known in the an may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974): Pain et al., J. Immunol, Meth., 40:219 (1981); and Nygren, J. Histochem. and Cytochem., 30:407 (1982).

Anti-PRO533 antibodies also are useful for the affinity purification of PRO533 from recombinant cell culture or natural sources. In this process, the antibodies against PR0533 are immobilized on a suitable support, such a Sephadex resin or filter paper, using methods well known in the art. The immobilized antibody then is contacted with a sample containing the PRO533 to be purified, and thereafter the support is washed with a suitable solvent that will remove substantially all the material in the sample except the PRO533, which is bound to the immobilized antibody. Finally, the support is washed with another suitable solvent that will release the PRO533 from the antibody.

FGFs can act upon cells in both a mitogenic and nonmitogenic manner. These factors are mitogenic for a wide variety of normal diploid mesoderm-derived and neural crest-derived cells, inducing granulosa cells, adrenal cortical cells, chrondorcytes, myoblasts, corneal and vascular endothelial cells (bovine or human), vascular smooth muscle cells, lens, retina and prostatic epithelial cells, oligodendrocytes, astrocytes and osteoblasts.

Non-mitogenic actions of FGF include promotion of cell migration into a wound area (chemotaxis), initiation of new blood vessel formation (angiogenesis), modulation of nerve regeneration and survival (neurotrophism), modulation of endocrine functions, and stimulation or suppression of specific cellular protein expression, extracellular matrix production and cell survival. Baird. A. Bohlen. P., Handbook of Exp. Pharmacol. 95(1): 369-418 (1990). These properties provide a basis for using FGFs in therapeutic approaches to accelerate wound healing, nerve repair, collateral blood vessel formation, and the like. For example. FGFs have been suggested to minimize myocardium damage in heart disease and surgery (U.S.P. 4.378.437).

FGF members have been shown to have diverse activities on cells of mesodermal or neuroectodermal origin with roles including the capcacity to promote or inhibit differentiated phenotypes during development, mediate angiogenic and neurotrophic effects, and modulate cell migration. Goldfarb. M.. Cytokine Growth Factor Rev. 7: 311-25 (1996); Naski, M.C. and Ornitz, D.M., Front. Biosci. 3: D781-94 (1998); Slavin. J. Cell Biol. Int. 19: 431-44 (1995). *In situ* analysis of DNA49435 shows expression in fetal skin. cartilage. the inner aspect of the fetal retina, and adult gall bladder epithelium (Fig. 8) as well as fetal small intestine, placental villi and umbilical cord (not shown). DNA49435 was not clearly detectable by RT-PCR in several tissues (not shown). It is further evident that DNA49435 shows elevated levels in colon adenocarcinoma cell line SW480, thereby indicating that antagonists of PR0533 could have therapeutic effect in the treatment of colon cancer.

PR0533 encoded by DNA49435 shows specificity for uniquely binding fibroblast growth factor receptor-4 (FGFR4), a property unique in the known FGF family. FGFR-4 signaling is proposed to be virtually non-mitogenic. Because of its unique binding characteristics. PR0533 could be used as a reagent to examine and analyze FGFR4 function in complex primary cell systems and animal models. Upregulation or amplification of FGFR4 has been associated with a variety of human cancers, particularly breast cancer. Abass, S.A., et al. J. Clin. Endocrinol. Metal. 82: 1160-66 (1997); Johnston, C.L. et al., Biochem. J. 306: 609-16 (1995): McLeskey, S.W. et al., Cancer Res. 54: 523-30 (1994); Penault-Llorca. F. et al., Int. J. Cancer 61: 170-76 (1995); Ron. D.R., J. Biol. Chem. 268: 5388-94 (1993). It has been shown that FGFR4. but not FGFR1-3 can mediate a membrane ruffling response that may be relevant to cancer cell motility. Johnston, C.L.. Biochem. J. 306: 609-16 (1995). The expression at high levels of DNA49435 in SW480 colon adenocarcinoma reflects the modulatory effects of FGFR4 in autocrine signaling.

The expression of PRO533. a specific FGFR4-ligand in fetal cartilage suggests a possible role for FGF19 in cartillage or bone development as well. The chromosomal mapping of DNA49435 to 11 q13 in conjunction with its expression in cartilage and the fetal retina suggests that PRO533-encoding DNA is a candidate gene for osteoporosis-pseudoglioma syndrome, a rare disorder defined by osteoporosis, vitreoretinal dysplasia, muscular hypotonia, and ligamentous laxity. Frontali, M. et al., Am. J. Med. Genet. 22: 35-47 (1985): Gong, Y. et al., Am. J. Hum. Genet. 59: 146-51 (1996); Johnson. M.L. et al., Am. J. Hum. Genet. 60: 1326-32 (1997); Somer, J. et al., J. Med. Genet. 25: 543-49 (1988).

### 2. Amplification of Genes Encoding PRO533 polypeptides in Tumor Tissues and Cell Lines

The present invention is based in part on the finding that the gene encoding PRO533 is amplified in primary lung tumors.

The genome of prokaryotic and eukaryotic organisms is subjected to two seemingly conflicting requirements. One is the preservation and propagation of DNA as the genetic information in its original form, to guarantee stable inheritance through multiple generations. On the other hand, cells or organisms must be able to adapt to lasting environmental changes. The adaptive mechanisms can include qualitative or quantitative modifications of the genetic material. Qualitative modifications include DNA mutations, in which coding sequences are altered resulting in a structurally and/or functionally different protein. Gene amplification is a quantitative modification, whereby the actual number of complete coding sequence. i.e. a gene, increases, leading to an increased number of available templates for transcription, an increased number of translatable transcripts, and, ultimately, to an increased abundance of the protein encoded by the amplified gene.

The phenomenon of gene amplification and its underlying mechanisms have been investigated *in vitro* in several prokaryotic and eukaryotic culture systems. The best-characterized example of gene amplification involves the culture of eukaryotic cells in medium containing variable concentrations of the cytotoxic drug methotrexate (MTX). MTX is a folic acid analogue and interferes with DNA synthesis by blocking the enzyme dihydrofolate reductase (DHFR). During the initial exposure to low concentrations of MTX most cells (>99.9%) will die. A small number of cells survive, and are capable of growing in increasing concentrations of MTX by producing large amounts of DHFR-RNA and protein. The basis of this overproduction is the amplification of the single DHFR gene. The additional copies of the gene are found as extrachromosomal copies in the form of small, supernumerary chromosomes (double minutes) or as integrated chromosomal copies.

Gene amplification is most commonly encountered in the development of resistance to cytotoxic drugs (antibiotics for bacteria and chemotherapeutic agents for eukaryotic cells) and neoplastic transformation. Transformation of a eukaryotic cell as a spontaneous event or due to a viral or chemical/environmental insult is typically associated with changes in the genetic material of that cell. One of the most common genetic changes observed in human malignancies are mutations of the p53 protein. p53 controls the transition of cells from the stationary (G1) to the replicative (S) phase and prevents this transition in the presence of DNA damage. In other words, one of the main consequences of disabling p53 mutations is the accumulation and propagation of DNA damage. *i*.*e*. genetic changes. Common types of genetic changes in neoplastic cells are, in addition to point mutations, amplifications and gross, structural alterations, such as translocations.

The amplification of DNA sequences may indicate specific functional requirement as illustrated in the DHFR experimental system. Therefore, the amplification of certain oncogenes in malignancies points toward a causative role of these genes in the process of malignant transformation and maintenance of the transformed phenotype. This hypothesis has gained support in recent studies. For example, the *bcl-2* protein was found to be amplified in certain types of non-Hodgkin's lymphoma. This protein inhibits apoptosis and leads to the progressive accumulation of neoplastic cells. Members of the gene family of growth factor receptors have been found to be amplified in various types of cancers suggesting that overexpression of these receptors may make neoplastic cells less susceptible to limiting amounts of available growth factor. Examples include the amplification of the androgen receptor in recurrent prostate cancer during androgen deprivation therapy and the amplification of the growth factor receptor homologue ERB2 in breast cancer. Lastly, genes involved in intracellular signaling and control of cell cycle progression can undergo amplification during malignant transformation. This is illustrated by the amplification of the *bcl-1* and *ras* genes in various epithelial and lymphoid neoplasms.

These earlier studies illustrate the feasibility of identifying amplified DNA sequences in neoplasms, because this approach can identify genes important for malignant transformation. The case of ERB2 also demonstrates the feasibility from a therapeutic standpoint, since transforming proteins may represent novel and specific targets for tumor therapy.

Several different techniques can be used to demonstrate amplified genomic sequences. Classical cytogenetic analysis of chromosome spreads prepared from cancer cells is adequate to identify gross structural alterations, such as translocations, deletions and inversions. Amplified genomic regions can only be visualized, if they involve large regions with high copy numbers or are present as extrachromosomal material. While cytogenetics was the first technique to demonstrate the consistent association of specific chromosomal changes with particular neoplasms, it is inadequate for the identification and isolation of manageable DNA sequences. The more recently developed technique of comparative genomic hybridization (CGH) has illustrated the widespread phenomenon of genomic amplification in neoplasms. Tumor and normal DNA are hybridized simultaneously onto metaphases of normal cells and the entire genome can be screened by image analysis for DNA sequences that are present in the tumor at an increased frequency. (WO 93/18.186: Gray et al., Radiation Res. 137, 275-289 [1994]). As a screening method, this type of analysis has revealed a large number of recurring amplicons (a stretch of amplified DNA) in a variety of human neoplasms. Although CGH is more sensitive than classical cytogenetic analysis in identifying amplified stretches of DNA, it does not allow a rapid identification and isolation of coding sequences within the amplicon by standard molecular genetic techniques.

The.most sensitive methods to detect gene amplification are polymerase chain reaction (PCR)-based assays. These assays utilize very small amount of tumor DNA as starting material, are exquisitely sensitive and provide DNA that is amenable to further analysis, such as sequencing and are suitable for high-volume throughput analysis.

The above-mentioned assays are not mutually exclusive, but are frequently used in combination to identify amplifications in neoplasms. While cytogenetic analysis and CGH represent screening methods to survey the entire genome for amplified regions. PCR-based assays are most suitable for the final identification of coding sequences. i.e. genes in amplified regions.

According to the present invention, such genes have been identified by quantitative PCR (S. Gelmini et al., Clin. Chem, 43, 752 [1997]), by comparing DNA from a variety of primary tumors, including breast, lung, colon, prostate, brain, liver, kidney, pancreas, spleen, thymus, testis, ovary, uterus, etc. tumor, or tumor cell lines, with pooled DNA from healthy donors. Quantitative PCR was performed using a TaqMan instrument (ABI). Gene-specific primers and fluorogenic probes were designed based upon the coding sequences of the DNAs.

Primary human lung tumor cells usually derive from adenocarcinomas, squamous cell carcinomas, large cell carcinomas, non-small cell carcinomas, small cell carcinomas, and broncho alveolar carcinomas, and include, for example, SRCC724 (squamous cell carcinoma abbreviated as "SqCCa"), SRCC725 (non-small cell carcinoma, abbreviated as "NSCCa"), SRCC726 (adenocarcinoma, abbreviated as "AdenoCa"), SRCC727 (adenocarcinoma), SRCC728 (squamous cell carcinoma), SRCC729 (adenocarcinoma). SRCC730 (adeno/squamous cell carcinoma), SRCC731 (adenocarcinoma), SRCC732 (squamous cell carcinoma), SRCC733 (adenocarcinoma), SRCC734 (adenocarcinoma), SRCC735 (broncho alveolar carcinoma, abbreviated as "BAC"). SRCC736 (squamous cell carcinoma), SRCC738 (squamous cell carcinoma), SRCC739 (squamous cell carcinoma), SRCC740 (squamous cell carcinoma), SRCC740 (lung cell carcinoma, abbreviated as "LCCa").

### 3. Tissue Distribution

The results of the gene amplification assays herein can be verified by further studies, such as, by determining mRNA expression in various human tissues.

As noted before, gene amplification and/or gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequencePR0533 polypeptide or against a synthetic peptide based on the DNA sequences provided herein or against exogenous sequence fused to PRO533 DNA and encoding a specific antibody epitope. General techniques for generating antibodies, and special protocols for Northern blotting and *in situ* hybridization are provided hereinbelow.

### 4. Chromosome Mapping

If the amplification of a given gene is functionally relevant, then that gene should be amplified more than neighboring genomic regions which are not important for tumor survival. To test this, the gene can be mapped to a particular chromosome, *e.g.* by radiation-hybrid analysis. The amplification level is then determined at the location identified, and compared with the level at neighboring genomic regions. Selective or preferential amplification at the genomic region to which to gene has been mapped is consistent with the possibility that the gene amplification observed promotes tumor growth or survival. Chromosome mapping includes both framework and epicenter mapping. For further details see e.g., Stewart et al., Genome Research 7,422-433 (1997).

### 5. Antibody Binding Studies

The results of the gene amplification study can be further verified by antibody binding studies, in which the ability of anti-PRO533 antibodies to inhibit the effect of the PRO533 polypeptides on tumor (cancer) cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein (encoded by a gene amplified in a tumor cell) in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.,* US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tumor sample may be fresh or frozen or may be embedded in paraffin and fixed with a preservative such as formalin, for example.

### 6. Cell-Based Tumor Assays

Cell-based assays and animal models for tumors (*e*.*g*. cancers) can be used to verify the findings of the gene amplification assay, and further understand the relationship between the genes identified herein and the development and pathogenesis of neoplastic cell growth. The role of gene products identified herein in the development and pathology of tumor or cancer can be tested by using primary tumor cells that have been identified to amplify the genes herein. Such cells include, for example, the lung cancer cells listed above.

In a different approach, cells of a cell type known to be involved in a particular tumor are transfected with the cDNAs herein, and the ability of these cDNAs to induce excessive growth is analyzed. Suitable cells include, for example, stable tumor cells lines such as, the B104-1-1 cell line (stable NIH-3T3 cell line transfected with the *neu* protooncogene) and *ras*-transfected NIH-3T3 cells, which can be transfected with the desired gene, and monitored for tumorogenic growth. Such transfected cell lines can then be used to test the ability of poly- or monoclonal antibodies or antibody compositions to inhibit tumorogenic cell growth by exerting cytostatic or cytotoxic activity on the growth of the transformed cells, or by mediating antibody-dependent cellular cytotoxicity (ADCC). Cells transfected with the coding sequences of the genes identified herein can further be used to identify drug candidates for the treatment of cancer.

In addition, primary cultures derived from tumors in transgenic animals (as described below) can be used in the cell-based assays herein, although stable cell lines are preferred. Techniques to derive continuous cell lines from transgenic animals are well known in the art (see, e.g. Small et al., Mol. Cell. Biol. 5, 642-648 [1985]).

### 7. Animal Models

A variety of well known animal models can be used to further understand the role of the genes identified herein in the development and pathogenesis of tumors, and to test the efficacy of candidate therapeutic agents, including antibodies, and other antagonists of the native polypeptides, including small molecule antagonists. The in *vivo* nature of such models makes them particularly predictive of responses in human patients. Animal models of tumors and cancers (*e*.*g*. breast cancer, colon cancer, prostate cancer, lung cancer, *etc*.) include both non-recombinant and recombinant (transgenic) animals. Non-recombinant animal models include, for example, rodent, *e*.*g*., murine models. Such models can be generated by introducing tumor cells into syngeneic mice using standard techniques, *e*.*g*. subcutaneous injection, tail vein injection, spleen implantation, intraperitoneal implantation, implantation under the renal capsule, or orthopin implantation, e.g. colon cancer cells implanted in colonic tissue. (See, *e*.*g*. PCT publication No. WO 97/33551. published September 18, 1997).

One of the most often used animal species in oncological studies are immunodeficient mice and, in particular, nude mice. The observation that the nude mouse with aplasia could successfully act as a host for human tumor xenografts has lead to its wide spread use for this purpose. The autosomal recessive nu gene has been introduced into a very large number of distinct congenic strains of nude mouse, including, for example. ASW, A/He, AKR, BALB/c. B10.LP, C17, C3H, C57BL. C57, CBA, DBA. DDD, I/st, NC, NFR, NFS, NFS/N. NZB, NZC, NZW. P. RIII and SJL. In addition, a wide variety of other animals with inherited immunological defects other than the nude mouse have been bred and used as recipients of tumor xenografts. For further details see, *e.g.* The Nude Mouse in Oncology Research. E. Boven and B. Winograd, eds.. CRC Press. Inc., 1991.

The cells introduced into such animals can be derived from known tumor/cancer cell lines, such as, any of the above-listed tumor cell lines, and, for example, the B104-1-1 cell line (stable NIH-3T3 cell line transfected with the neu protooncogene): ras-transfected NIH-3T3 cells; Caco-2 (ATCC HTB-37): a moderately well-differentiated grade II human colon adenocarcinoma cell line. HT-29 (ATCC HTB-38), or from tumors and cancers. Samples of tumor or cancer cells can be obtained from patients undergoing surgery, using standard conditions *(e.g.* freezing and storing in liquid nitrogen. Karmali et al., Br. J. Cancer 48, 689-696 [1983]).

Tumor cells can be introduced into animals, such as nude mice by a variety of procedures. The subcutaneous (s.c.) space in mice is very suitable for tumor implantation. Tumors can be transplanted s.c. as solid blocks, as needle biopsies by use of a trochar, or as cell suspensions. For solid block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue. Boven and Winograd, *supra*.

Animal models of breast cancer can be generated, for example, by implanting rat neuroblastoma cells (from which the *neu* oncogen was initially isolated), or *neu*-transformed NIH-3T3 cells into nude mice, essentially as described by Drebin et al. PNAS USA 83, 9129-9133 (1986).

Similarly, animal models of colon cancer can be generated by passaging colon cancer cells in animals, e.g. nude mice, leading to the appearance of tumors in these animals. An orthotopic transplant model of human colon cancer in nude mice has been described, for example, by Wang et al., Cancer Res. 54, 4726-4728 (1994) and Too et al., Cancer Res. 55, 681-684 (1995). This model is based on the so-called "METAMOUSE^{®}" sold by AntiCancer, Inc. (San Diego, California).

Tumors that arise in animals can be removed and cultured in vitro. Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A, CMS4, CMS5, CMS21, and WEHI-164 are chemically induced fibrosarcomas of BALB/c female mice (DeLeo et al., J. Exp. Med. 146, 720 [1977]), which provide a highly controllable model system for studying the anti-tumor activities of various agents (Palladino et al., J. Immunol. 138, 4023-4032 [1987]). Briefly, tumor cells are propagated in vitro in cell culture. Prior to injection to the animals, the cell lines are washed and suspended in buffer, at a cell density of about 10x10⁶ to 10x10⁷ cells/ml. The animals are then infected subcutaneously with 100 to 100 µl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small cell carcinoma of the lung (SCCL). This tumor can be introduced in normal mice upon injection of tumor fragments from an affected mouse or of cells maintained in culture (Zupi et al., Br. J. Cancer 41, suppl. 4, 309 [1980]), and evidence indicates that tumors can be started from injection of even a single cell and that a very high proportion of infected tumor cells survive. For further information about this tumor model see Zacharski, Haemostasis 16, 300-320 [1986]).

One way of evaluating the efficacy of a test compound in an animal model is implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen. Proc. 6th Int. Workshop on Immune-Deficient Animals, Wu and Sheng eds.. Basel, 1989, 301. It is noted, however, that necrosis and inflammatory responses following treatment may actually result in an increase in tumor size, at least initially. Therefore, these changes need to be carefully monitored, by a combination of a morphometric method and flow cytometric analysis.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g.* baboons, chimpanzees and monkeys. Techniques known in the art to introduce a transgene into such animals include pronucleic microinjection (Hoppe and Wanger, U.S. Patent No. 4,873,191): retrovirus-mediated gene transfer into germ lines (*e*.*g*., Van der Putten et al., Proc. Natl. Acad. Sci. USA 82, 6148-615 [1985]); gene targeting in embryonic stem cells (Thompson et al.. Cell 56, 313-321 [1989]); electroporation of embryos (Lo. Mol. Cell Biol. 3, 1803-1814 [1983]); sperm-mediated gene transfer (Lavitrano et al.. Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e*.*g*., head-to-head or head-to-tail tandems. Selective introduction of a transgene into a particular cell type is also possible by following, for example, the technique of Lasko et al., Proc. Natl. Acad Sci. USA 89, 6232-636 (1992).

The expression of the transgene in transgenic animals can be monitored by standard techniques. For example. Southern blot analysis or PCR amplification can be used to verify the integration of the transgene. The level of mRNA expression can then be analyzed using techniques such as in situ hybridization. Northern blot analysis. PCR. or immunocytochemistry. The animals are further examined for signs of tumor or cancer development.

Alternatively, "knock out" animals can be constructed which have a defective or altered gene encoding a PRO533 polypeptide identified herein, as a result of homologous recombination between the endogenous gene encoding the polypeptide and altered genomic DNA encoding the same polypeptide introduced into an embryonic cell of the animal. For example, cDNA encoding a particular PRO533 polypeptide can be used to clone genomic DNA encoding that polypeptide in accordance with established techniques. A portion of the genomic DNA encoding a particular PRO533 polypeptide can be deleted or replaced with another gene, such as a gene encoding a selectable marker which can be used to monitor integration. Typically, several kilobases of unaltered flanking DNA (both at the 5' and 3' ends) are included in the vector [see *e.g*., Thomas and Capecchi. Cell, 51: 503 (1987) for a description of homologous recombination vectors]. The vector is introduced into an embryonic stem cell line (*e*.*g*., by electroporation) and cells in which the introduced DNA has homologously recombined with the endogenous DNA are selected [see e.g., Li et al., Cell, 69: 915 (1992)]. The selected cells are then injected into a blastocyst of an animal (*e*.*g*., a mouse or rat) to form aggregation chimeras [see *e*.*g*., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the PRO533.

The efficacy of antibodies specifically binding the polypeptides identified herein and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination, biopsy, and is scanned by computed tomography (CT). Cats diagnosed with sublingual oral squamous cell tumors are excluded from the study. The tongue can become paralyzed as a result of such tumor, and even the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chrondroma, leiomyosarcoma of dogs, cats, and baboons can also be tested. Of these mammary adenocarcinoma in dogs and cats is a preferred model as its appearance and behavior are very similar to those in humans. However, the use of this model is limited by the rare occurrence of this type of tumor in animals.

### 8. Screening Assays for Drug Candidates

Screening assays for drug candidates are designed to identify compounds that bind or complex with the polypeptides encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides. (poly)peptide-immunoglobulin fusions, and, in particular, antibodies including, without limitation, poly- and monoclonal antibodies and antibody fragments. sinele-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art.

All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e*.*g*. on a microtiter plate, by covalent or non-covalent attachments. Non-covalent attachment generally is accomplished by coating the solid surface with a solution of the polypeptide and drying. Alternatively, an immobilized antibody, e.g. a monoclonal antibody, specific for the polypeptide to be immobilized can be used to anchor it to a solid surface. The assay is performed by adding the non-immobilized component, which may be labeled by a detectable label, to the immobilized component, *e*.*g*. the coated surface containing the anchored component. When the reaction is complete, the non-reacted components are removed. *e.g.* by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PRO533 protein encoded by a gene identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989): Chien et al.. Proc. Natl. Acad Sci. USA 88, 9578-9582 (1991)] as disclosed by Chevray and Nathans [Proc. Natl. Acad Sci. USA 89, 5789-5793 (1991)]. Many transcriptional activators, such as yeast GAL4. consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4. and another, in which candidate activating proteins are fused to the activation domain. The expression of a GAL I-IacZ reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a PRO533-encoding gene identified herein and other intra- or extracellular components can be tested as follows: usually a reaction mixture is prepared containing the product of the amplified gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

### 9. Compositions and Methods for the Treatment of Tumors

The compositions useful in the treatment of tumors associated with the amplification of the genes identified herein include, without limitation, antibodies, small organic and inorganic molecules, peptides, phosphopeptides, antisense and ribozyme molecules, triple helix molecules, etc. that inhibit the expression and/or activity of the target gene product.

For example, antisense RNA and RNA molecule act to directly block the translation of mRNA by hybridizing to targeted mRNA and preventing protein translation. When antisense DNA is used, oligodeoxyribonucleotides derived from the translation initiation site, e.g. between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.* Rossi. Curr. Biol. 4: 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizable stretches of purines or pyrimidines on one strand of a duplex. For further details see, e.g. PCT publication No. WO 97/33551. supra.

These molecules can be identified by any or any combination of the screening assays discussed hereinabove and/or by any other screening techniques well known for those skilled in the art.

### 10. Anti-PRO533 Antibodies

The present invention further provides anti-PRO533 antibodies. Exemplary antibodies include polyclonal, monoclonal, humanized, bispecific, and heteroconjugate antibodies. Promising drug candidates according to the present invention are antibodies and antibody fragments which may inhibit the production, or the gene product of the amplified genes identified herein and/or reduce the activity of the gene products.

### 10.1. Polyclonal Antibodies

The anti-PRO533 antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO533 polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin. bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 10.2. Monoclonal Antibodies

The anti-PRO533 antibodies may, alternatively, be monoclonal antibodies. Monoclonal antibodies may be prepared using hybridoma methods, such as those described by Kohler and Milstein. Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO533 polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press. (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Rockville. Maryland. Human myeloma and mouse-human heteromyeloma cell lines also have been described for the production of human monoclonal antibodies [Kozbor, J. Immunol., 133: 3001 (1984); Brodeur et al., Monoclonal Antibody Production Techniques and Applications, Marcel Dekker, Inc.. New York. (1987) pp. 51-63].

The culture medium in which the hybridoma cells are cultured can then be assayed for the presence of monoclonal antibodies directed against PR0533. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107: 220 (1980).

After the desired hybridoma cells are identified, the clones may be subcloned by limiting dilution procedures and grown by standard methods [Goding, supra]. Suitable culture media for this purpose include, for example. Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816.567. DNA encoding the monoclonal antibodies of the invention can be readily isolated and sequenced using conventional procedures (*e*.*g*., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison *et al*., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 10.3. Human and Humanized Antibodies

The anti-PRO533 antibodies of the invention may further comprise humanized antibodies or human antibodies. Humanized forms of non-human (*e.g.*, murine) antibodies are chimeric immunoglobulins, immunoglobulin chains or fragments thereof (such as Fv, Fab. Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332: 323-329 (1988); and Presta, Curr. Op. Struct. Biol., 2: 593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321: 522-525 (1986); Riechmann et al., Nature. 332: 323-327 (1988); Verhoeyen et al., Science. 239: 1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter. J. Mol. Biol., 227: 381 (1991); Marks et al., J. Mol Biol., 222: 581 (1991)]. The techniques of Cole *et al.* and Boerner *et al.* are also available for the preparation of human monoclonal antibodies (Cole et al., Monoclonal Antibodies and Cancer Therapy, Alan R. Liss, p. 77 (1985) and Boerner et al., J. Immunol., 147(1): 86-95 (1991)]. Similarly, human antibodies can be made by introducing of human immunoglobulin loci into transgenic animals, *e*.*g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5.545.807; 5,545,806; 5,569.825; 5,625,126; 5.633,425; 5,661.016, and in the following scientific publications: Marks et al., Bio/Technology 10, 779-783 (1992); Lonberg et al., Nature 368: 856-859 (1994); Morrison. Nature 368: 812-13 (1994); Fishwild et al., Nature Biotechnology 14: 845-51 (1996); Neuberger, Nature Biotechnology 14: 826 (1996); Lonberg and Huszar. Intern. Rev. Immunol. 13: 65-93 (1995).

### 10.4. Bispecific Antibodies

Bispecific antibodies are monoclonal, preferably human or humanized, antibodies that have binding specificities for at least two different antigens. In the present case, one of the binding specificities is for the PRO533, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature. 305: 537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829. published 13 May 1993, and in Traunecker et al., EMBO J., 10: 3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CHI) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121: 210 (1986).

### 10.5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360: WO 92/200373: EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 10.6. Effector Function Engineering

It may be desirable to modify the antibody of the invention with respect to effector function, so as to enhance the effectiveness of the antibody in treating cancer, for example. For example, cysteine residue(s) may be introduced in the Fc region, thereby allowing interchain disulfide bond formation in this region. The homodimeric antibody thus generated may have improved internalization capability and/or increased-complement-mediated cell killing and antibody-dependent cellular cytotoxicity (ADCC). See Caron et al., J. Exp. Med. 176: 1191-1195 (1992) and Shopes. B., J. Immunol. 148: 2918-2922 (1992). Homodimeric antibodies with enhanced anti-tumor activity may also be prepared using heterobifunctional cross-linkers as described in Wolff et al., Cancer Research 53: 2560-2565 (1993). Alternatively, an antibody can be engineered which has dual Fc regions and may thereby have enhanced complement lysis and ADCC capabilities. See Stevenson et al., Anti-Cancer Drug Design 3: 219-230 (1989).

### 10.7. Immunoconjugates

The invention also pertains to immunoconjugates comprising an antibody conjugated to a cytotoxic agent such as a chemotherapuetic agent, toxin, (*e*.*g*., an enzymatically active toxin of bacterial, fungal, plant or animal origin, or fragments thereof), or a radioactive isotope *(i.e.,* a radioconjugate).

Chemotherapeutic agents useful in the generation of such immunoconjugates have been described above. Enzymatically active toxins and fragments thereof which can be used include diphtheria A chain, nonbinding active fragments of diphtheria toxin, exotoxinA chain (from *Pseudomonas aeruginosa),* ricin A chain, abrin A chain, modeccin A chain, alpha-sarcin, *Aleurites fordii* proteins, dianthin proteins. *Phytolaca americana* proteins (PAPI, PAPII and PAP-S), momordica charantia inhibitor, curcin, crotin, saponaria officinalis inhibitor, gelonin, mitogellin, restrictocin, phenomycin, enomycin and the tricothecenes. A variety of radionuclides are available for the production of radioconjugated antibodies. Examples include ²¹²Bi, ¹³¹, ¹³¹In, ⁹⁰Y and ¹⁸⁶Re.

Conjugates of the antibody and cytotoxic agent are made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis-(p-azidobenzoyl)-hexanediamine), bis-diazonium derivatives (such as bis-(p-diazoniumbenzoyl)-ethylenediamine), diisocyanates (such as tolyl-2,6-diisocyanate), and bis-active fluorine compounds (such as 1,5-difluoro-2,4-dinitrobenzene). For example, a ricin immunotoxin can be prepared as described in Vitetta et al., Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (e.g. avidin) which is conjugated to a cytotoxic agent *(e.g.* a radionucleotide).

### 10.8. Immunoliposomes

The antibodies disclosed herein may also be formulated as immunoliposomes. Liposomes containing the antibody are prepared by methods known in the art, such as described in Epstein et al., Proc. Natl. Acad. Sci. USA. 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257: 286-288 (1982) via a disulfide interchange reaction. A chemotherapeutic agent (such as Doxorubicin) is optionally contained within the liposome. See Gabizon et al., J. National Cancer Inst. 81 (19): 1484 (1989).

### F. Pharmaceutical Compositions

Antibodies specifically binding PRO533 (FGF-19), as well as other molecules identified by the screening assays disclosed hereinbefore, can be administered for the treatment of tumors, including cancers, in the form of pharmaceutical compositions.

If the protein encoded by the amplified gene is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.* Marasco et al., Proc. Natl. Acad. Sci. USA 90: 7889-7893 [1993]).

Therapeutic formulations of the polypeptide or antibody are prepared for storage as lyophilized formulations or aqueous solutions by mixing the polypeptide having the desired degree of purity with optional "pharmaceutically-acceptable" or "physiologically-acceptable" carriers, excipients or stabilizers typically employed in the art (all of which are termed "excipients"). For example, buffering agents, stabilizing agents, preservatives, isotonifiers, non-ionic detergents, antioxidants and other miscellaneous additives. (See Remington's Pharmaceutical Sciences. 16th edition (or later), A. Osol, Ed. (1980)). Such additives must be nontoxic to the recipients at the dosages and concentrations employed.

Buffering agents help to maintain the pH in the range which approximates physiological conditions. They are preferably present at concentration ranging from about 2mM to about 50 mM. Suitable buffering agents for use with the present invention include both organic and inorganic acids and salts thereof. For example, citrate buffers (*e*.*g*., monosodium citrate-disodium citrate mixture, citric acid-trisodium citrate mixture, citric acid-monosodium citrate mixture, *etc*.), succinate buffers (*e*.*g*., succinic acid-monosodium succinate mixture, succinic acid-sodium hydroxide mixture, succinic acid-disodium succinate mixture, *etc*.), tartrate buffers (*e*.*g*., tartaric acid-sodium tartrate mixture, tartaric acid-potassium tartrate mixture, tartaric acid-sodium hydroxide mixture, *etc*.), fumarate buffers (*e.g.*, fumaric acid-monosodium fumarate mixture, *etc.),* fumarate buffers (*e*.*g*., fumaric acid-monosodium fumarate mixture, fumaric acid-disodium fumarate mixture, monosodium fumarate-disodium fumarate mixture, *etc*.), gluconate buffers (*e*.*g*., gluconic acid-sodium glyconate mixture, gluconic acid-sodium hydroxide mixture, gluconic acid-potassium gluconate mixture, *etc*.), oxalate buffer (*e*.*g*., oxalic acid-sodium oxalate mixture, oxalic acid-sodium hydroxide mixture, oxalic acid-potassium oxalate mixture, *etc*.), lactate buffers (*e*.*g*., lactic acid-sodium lactate mixture, lactic acid-sodium hydroxide mixture, lactic acid-potassium lactate mixture, *etc.)* and acetate buffers (e*.g.,* acetic acid-sodium acetate mixture, acetic acid-sodium hydroxide mixture, *etc.*). Additionally; phosphate buffers, histidine buffers and trimethylamine salts such as Tris may be employed.

Preservatives are added to retard microbial growth, and are added in amounts ranging from 0.2% - 1% (w/v). Suitable preservatives for use with the present invention include phenol, benzyl alcohol, meta-cresol, methyl paraben, propyl paraben, octadecyldimethylbenzyl ammonium chloride, benzalconium halides (e.g., chloride, bromide, iodide), hexamethonium chloride, alkyl parabens such as methyl or propyl paraben, catechol, resorcinol, cyclohexanol, and 3-pentanol.

Isotonicifiers sometimes known as "stabilizers" are present to ensure isotonicity of liquid compositions of the present invention and include polyhydric sugar alcohols, preferably trihydric or higher sugar alcohols, such as glycerin, erythritol, arabitol, xylitol, sorbitol and mannitol. Polyhydric alcohols can be present in an amount between 0.1% to 25% by weight, preferably 1% to 5% taking into account the relative amounts of the other ingredients.

Stabilizers refer to a broad category of excipients that can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol: amino acid polymers: sulfur containing reducing agents, such as urea, glutathione, thioctic acid, sodium thioglycolate, thioglycerol, a-monothioglycerol and sodium thio sulfate; low molecular weight polypeptides (*i*.*e*. < 10 residues); proteins such as human serum albumin, bovine serum albumin, gelatin or immunoglobulins; hydrophilic polymers, such as polyvinylpyrrolidone monosaccharides, such as xylose, mannose, fructose, glucose; disaccharides such as lactose, maltose, sucrose and trisaccacharides such as raffinose: polysaccharides such as dextran. Stabilizers can be present in the range from 0.1 to 10,000 weights per part of weight active protein.

Non-ionic surfactants or detergents (also known as "wetting agents") are present to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, etc.), polyoxamers (184. 188 etc.), Pluronic^{®} polyols, polyoxyethylene sorbitan monoethers (Tween ^{®}-20. Tween ^{®}-80, etc.). Non-ionic surfactants are present in a range of about 0.05 mg/ml to about 1.0 mg/ml, preferably about 0.07 mg/ml to about 0.2 mg/ml.

Additional miscellaneous excipients include bulking agents. (*e*.*g*. starch), chelating agents (*e*.*g*. EDTA), antioxidants (*e*.*g*., ascorbic acid, methionine, vitamin E), and cosolvents.

The formulations herein may also contain more than one active compound as necessary for the particular indication being treated, preferably those with complementary activities that do not adversely affect each other. For example, it may be desirable to further provide an immunosuppressive agent. Such molecules are suitably present in combination in amounts that are effective for the purpose intended.

The active ingredients may also be entrapped in microcapsule prepared, for example, by coascervation techniques or by interfacial polymerization, for example, hydroxymethylcellulose orgelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition (or later), A. Osal. Ed. (1980).

The formulations to be used for *in vivo* administration must be sterile. This is readily accomplished, for example, by filtration through sterile filtration membranes.

Sustained-release preparations may be prepared. Suitable examples of sustained-release preparations include semi-permeable matrices of solid hydrophobic polymers containing the antibody mutant, which matrices are in the form of shaped articles, *e*.*g*., films, or microcapsules. Examples of sustained-release matrices include polyesters, hydrogels (for example, poly(2-hydroxyethyl- methacrylate), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT^{®} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Non-antibody compounds identified by the screening assays of the present invention can be formulated in an analogous manner, using standard techniques well known in the art.

### G. Methods of Treatment

It is contemplated that the antibodies and other anti-tumor compounds of the present invention may be used to treat various conditions, including those characterized by overexpression and/or activation of the gene encoding PRO533. Exemplary conditions or disorders to be treated with such antibodies and other compounds, including, but not limited to, small organic and inorganic molecules, peptides, antisense molecules, *etc.* include benign or malignant tumors (*e.g*. renal, liver, kidney, bladder, breast, gastric, ovarian, colorectal, prostate, pancreatic, ling, vulval, thyroid, hepatic carcinomas: sarcomas: glioblastomas: and various head and neck tumors); leukemias and lymphoid malignancies; other disorders such as neuronal, glial, astrocytal, hypothalamic and other glandular, macrophagal, epithelial, stromal and blastocoelic disorders: and inflammatory, angiogenic and immunologic disorders.

The anti-tumor agents of the present invention, e.g. antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

Other therapeutic regimens may be combined with the administration of the anti-cancer agents, e.g. antibodies of the instant invention. For example, the patient to be treated with such anti-cancer agents may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins. Baltimore, MD (1992). The chemotherapeutic agent may precede, or follow administration of the anti-tumor agent, e.g. antibody, or may be given simultaneously therewith. The antibody may be combined with an anti-oestrogen compound such as tamoxifen or an anti-progesterone such as onapristone (see, EP 616812) in dosages known for such molecules.

It may be desirable to also administer antibodies against other tumor associated antigens, such as antibodies which bind to theErbB2, EGFR. ErbB3, ErbB4, or vascular endothelial factor (VEGF). Alternatively, or in addition, two or more antibodies binding the same or two or more different antigens disclosed herein may be co-administered to the patient. Sometimes, it may be beneficial to also administer one or more cytokines to the patient. In a preferred embodiment, the antibodies herein are co-administered with a growth inhibitory agent. For example, the growth inhibitory agent may be administered first, followed by an antibody of the present invention. However, simultaneous administration or administration of the antibody of the present invention first is also contemplated. Suitable dosages for the growth inhibitory agent are those presently used and may be lowered due to the combined action (synergy) of the growth inhibitory agent and the antibody herein.

For the prevention or treatment of disease, the appropriate dosage of an anti-tumor agent, e.g. an antibody herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments.

The amount of therapeutic polypeptide, antibody or fragment thereof which will be effective in the treatment of a particular disorder or condition will depend on the nature of the disorder or condition, and can be determined by standard clinical techniques. Where possible, it is desirable to determine the dose-response curve and the pharmaceutical compositions of the invention first in vitro, and then in useful animal model systems prior to testing in humans. However, based on common knowledge of the art, a pharmaceutical composition effective in promoting the survival of sensory neurons may provide a local therapeutic agent concentration of between about 5 and 20 ng/ml, and, preferably, between about 10 and 20 ng/ml.

The dosing schedule for subcutaneous administration may vary form once a month to daily depending on a number of clinical factors, including the type of disease, severity of disease, and the subject's sensitivity to the therapeutic agent.

For example, depending on the type and severity of the disease, about 500-ng/kg to 100 mg/kg (*i*.*e*. 0.0005- 100mg/kg) of antibody is an initial candidate dosage for administration to the patient, whether, for example, by one or more separate administrations, or by continuous infusion. A typical daily dosage might range from about 1 µg/kg to 20 mg/kg or more, depending on the factors mentioned above. For repeated administrations over several days or longer, depending on the condition, the treatment is sustained until a desired suppression of disease symptoms occurs. However, other dosage regimens may be useful. Conventional techniques and assays easily monitor the progress of this therapy.

As can be appreciated by one of ordinary skill, optimal dosages and desired drug concentrations of pharmaceutical compositions of the present invention may vary depending on the particular use envisioned. The determination of the appropriate dosage or route of administration is well within the skill of an artisan of ordinary skill in the art. Animal experiments provide reliable guidance for the determination of effective doses for human therapy. Interspecies scaling of effective doses can be performed following the principles laid down by Mordenti, J. and Chappell, W., "The use of interspecies scaling in toxicokinetics", Toxicokinetics and New Drug Development, Yacobi et al., Eds, Pergamon Press, New York 1989. pp. 42-96.

### H. Articles of Manufacture

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually an anti-tumor agent that is capable of interfering with the activity of a gene product identified herein. e.g. an antibody. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

### 1. Diagnosis and Prognosis of Tumors

While cell surface proteins, such as growth receptors overexpressed in certain tumors are excellent targets for drug candidates or tumor (e.g. cancer) treatment, the same proteins along with secreted proteins encoded by the genes amplified in tumor cells find additional use in the diagnosis and prognosis of tumors. For example, antibodies directed against the proteins products of genes amplified in tumor cells can be used as tumor diagnostics or prognostics.

For example, antibodies, including antibody fragments, can be used to qualitatively or quantitatively to detect the expression of proteins encoded by the amplified genes ("marker gene products"). The antibody preferably is equipped with a detectable, e.g. fluorescent label, and binding can be monitored by light microscopy, flow cytometry, fluorimetry, or other techniques known in the art. These techniques are particularly suitable, if the amplified gene encodes a cell surface protein, *e*.*g*. a growth factor. Such binding assays are performed essentially as described in section 5 above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE I

### Isolation of cDNA clones Encoding Human PRO533

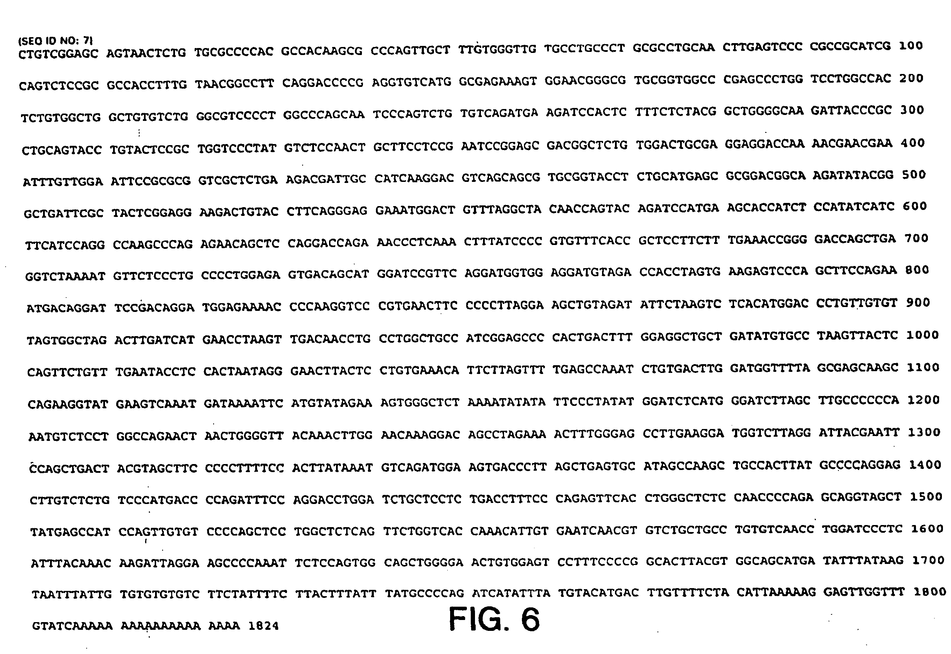

The EST sequence accession number AF007268, a murine fibroblast growth factor (FGF-15) was used to search various public EST databases (*e*.*g*., GenBank, Dayhoff, *etc*.). The search was performed using the computer program BLAST or BLAST2 [Altschul *et al., Methods in Enzymology*, 266: 460-480 (1996); http://blast.wustl/edu/blast/README.html] as a comparison of the ECD protein sequences to a 6 frame translation of the EST sequences. The search resulted in a hit with GenBank EST AA220994, which has been identified as stratagene NT2 neuronal precursor 937230. AA220994 (DNA47412) is identified in Fig. 6.

Based on this sequence, oligonucleotides were synthesized: 1) to identify by PCR a cDNA library that contained the sequence of interest, and 2) for use as probes to isolate a clone of the full-length coding sequence. Forward and reverse PCR primers (notated as *.f and *.r, respectively) may range from 20 to 30 nucleotides (typically about 24), and are designed to give a PCR product of 100-1000 bp in length. The probe sequences (notated as *.p) are typically 40-55 bp (typically about 50) in length. In order to screen several libraries for a source of a full-length clone, DNA from the libraries was screened by PCR amplification, as per Ausubel *et al., Current Protocols in Molecular Biology,* with the PCR primer pair. A positive library was then used to isolate clones encoding the gene of interest by the in vivo cloning procedure suing the probe oligonucleotide and one of the PCR primers.

In order to screen several libraries for a source of a full-length clone, DNA from the libraries was screened by PCR amplification with the PCR primer pair identified above. A positive library was then used to isolate clones encoding the PR0533 gene using the probe oligonucleotide and one of the PCR primers.

RNA for construction of the cDNA libraries was isolated from human fetal retina. The cDNA libraries used to isolated the cDNA clones were constructed by standard methods using commercially available reagents (*e.g.,* Invitrogen, San Diego, CA: Clontech, *etc.)* The cDNA was primed with oligo dT containing a Notl site, linked with blunt to Sall hemikinased adaptors, cleaved with Notl, sized appropriately by gel electrophoresis, and cloned in a defined orientation into a suitable cloning vector (such as pRKB or pRKD; pRK5B is a precursor of pRK5D that does not contain the Sfil site: see. Holmes et al., Science, 253:1278-1280 (1991)) in the unique Xhol and Notl sites.

A cDNA clone was sequenced in its entirety. The full length nucleotide sequence of PRO533 is shown in Figure 1 (SEQ ID NO: 1). Clone DNA49435 contains a single open reading frame with an apparent translational initiation site at nucleotide positions 459-461 (Fig. 2: SEQ ID NO: 2). The predicted polypeptide precursor is 216 amino acids long. Clone DNA49435-1219 has been deposited with ATCC and is assigned ATCC deposit no. 209480.

The extracellular domsins of Fibroblast Growth Factors 1-4 (i.e., amino acids 1-404, 1-408, 1-403 and 1-412, respetively) were isolated by PCR using Pfu polymerase (Stratagene) from fetal lung cDNA (according to the manufacturer instructions) and subcloned in frame with the Fc region of human IgG1 in the eukaryotic expression vector pRK5tkNEO, a derivative or pRK5.

Based on a BLAST-2 and FastA sequence alignment analysis of the full-length sequence. PRO533 shows amino acid sequence identity to murine fibroblast growth factor-15 (53%).

The oligonucleotide sequences used in the above procedure were the following:
FGF15.f: ATCCGCCCAGATGGCTACAATGTGTA (SEQ ID NO: 16)
FGF15.p2: AGACCGGGAGGCGGTGCTTCTCGGATCGGTACACATTGTA (SEQ ID NO: 17)
FGF15.r: CCAGTCCGGTGACAAGCCCAAA (SEQ ID NO: 18)

### EXAMPLE 2

### Northern Blot Analysis

Expression of PR0533 mRNA in human tissues was examined by Northern blot analysis. Multiple tissue human RNA blots were hybridized to a ³²P-labelled DNA probe of random primed DNA49435 cDNA according to the manufacturers (Clontech) instructions. Human fetal RNA blot MTN (Clontech) and human adult RNA blot MTN-II (Clontech) were incubated with the DNA probes. Blots were incubated with the probes in hybridization buffer (5X SSPE: 2X Denhardt's solution: 100 mg/mL denatured sheared salmon sperm DNA; 50% formamide: 2% SDS) for 60 hours at 42°C. The blots were washed several times in 2X SSC: 0.05% SDS for I hour at room temperature, followed by a 30 minute wash in 0.1X SSC: 0.1% SDS at 50°C. The blots were developed after exposure to X-omat (Kodak) for 72 hours.

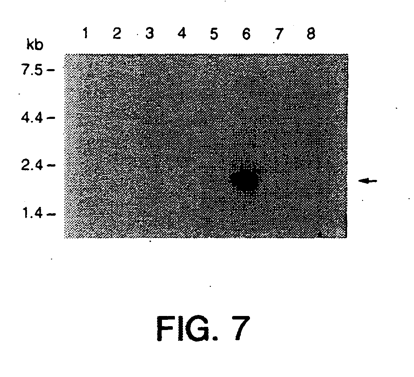

As shown in Fig. 7. PR0533 mRNA transcripts were detected. Strong expression was seen in colorectal adenocarcinoma SW480.

### EXAMPLE 3

### In situ Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization was performed following an optimized version of the protocol by Lu and Gillett. Cell Vision 1: 169-176 (1994), using PCR-generated ³³P-labeled riboprobes from a plasmid vector containing PRO533 encoding DNA. Briefly, formalin-fixed, paraffin-embedded human tissues were sectioned, deparaffinized, deproteinated in proteinase K (20 g/ml) for 15 minutes at 37°C. and further processed for *in situ* hybridization as described by Lu and Gillett, *supra.* A [³³-P]-UTP-labeled antisense riboprobe was generated from a PCR product and hybridized at 55°C overnight. The slides were dipped in Kodak NTB2 nuclear track emulsion and exposed for 4 weeks.

### ³³P-Riboprobe synthesis

6.0 µl (125 mCi) of "P-UTP (Amersham BF 1002, SA<2000 Ci/mmol) were speed vac dried. To each tube containing dried ³³P-UTP, the following ingredients were added:
2.0 µl 5x transcription buffer
1.0 µl DTT (100 mM)
2.0 µl NTP mix (2.5 mM : 10 µl; each of 10 mM GTP, CTP & ATP + 10 µl H₂O)
1.0 µl UTP (50 µM)
1.0 µl Rnasin
1.0 µl DNA template (1µg)
1.0 µl H₂O
1.0 µl RNA polymerase (for PCR products T3 = AS. T7 = S, usually)

The tubes were incubated at 37°C for one hour. 1.0 µl RQ1 DNase were added, followed by incubation at 37°C for 15 minutes. 90 µl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 µl TE were added, 1 µl of the final product was pipetted on DE81 paper and counted in 6 ml of Biofluor II.

The probe was run on a TBE/urea gel. 1-3 µl of the probe or 5 µl of RNA Mrk III were added to 3 µl of loading buffer. After heating on a 95°C heat block for three minutes, the gel was immediately placed on ice. The wells of gel were flushed, the sample loaded, and run at 180-250 volts for 45 minutes. The gel was wrapped in saran wrap and exposed to XAR film with an intensifying screen in -70°C freezer one hour to overnight.

### ³³P-Hybridization

*Pretreatment of frozen sections* The slides were removed from the freezer, placed on aluminium trays and thawed at room temperature for 5 minutes. The trays were placed in a 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNase-free RNAse buffer), the sections were washed in 0.5 x SSC for 10 minutes at room temperature. The sections were dehydrated in 70%, 95%, 100% ethanol, 2 minutes each.

*Pretreatment of paraffin-embedded sections* The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer: 37°C, 15 minutes) - human embryo, or 8 x proteinase K (100 µl in 250 ml Rnase buffer, 37°C, 30 minutes) - formalin tissues. Subsequent rinsing in 0.5 x SSC and dehydration were performed as described above.

*Prehybridization:* The slides were laid out in plastic box lined with Box buffer (4 x SSC, 50% formamide) - saturated filter paper. The tissue was covered with 50 µl of hybridization buffer (3.75g Dextran Sulfate + 6 ml SQ H₂O), vortexed and heated in the microwave for 2 minutes with the cap loosened. After cooling on ice, 18.75 ml formamide, 3.75 ml 20 x SSC and 9 ml SQ H₂O were added, the tissue was vortexed well, and incubated at 42°C for 1-4 hours.

*Hybridization:* 1.0 x 10⁶ cpm probe and 1.0 µl tRNA (50 mg/ml stock) per slide were heated at 95°C for 3 minutes. The slides were cooled on ice, and 48 µl hybridization buffer were added per slide. After vortexing. 50 µl ³³P mix were added to 50 µl prehybridization on slide. The slides were incubated overnight at 55°C.

*Washes:* Washing was done 2x10 minutes with 2xSSC, EDTA at room temperature (400 ml 20 x SSC + 16 ml 0.25M EDTA, V_{f}=4L), followed by RNaseA treatment at 37°C for 30 minutes (500 µl of 10 mg/ml in 250 ml Rnase buffer = 20 µg/ml), The slides were washed 2x10 minutes with 2 x SSC, EDTA at room temperature. The stringency wash conditions were as follows: 2 hours at 55°C. 0.1 x SSC, EDTA (20 ml 20 x SSC + 16 ml EDTA. V_{f}=4L).

### DNA49435 (FGF homologue, FGF receptor 3 ligand)

Moderate expression was observed over cortical neurons in the fetal brain. Expression was observed over the inner aspect of the fetal retina, and possibly in the developing lens. Expression was seen over fetal skin, cartilage, small intestine, placental villi and umbilical cord. It adult tissues, there was an extremely high level of expression over the gallbladder epithelium (see Figure 34). Moderate expression was seen over the adult kidney, gastric and colonic epithelia. These data are consistent with the potential role of this molecule in cartilage and bone growth.

### EXAMPLE 4

### Western Analysis of PRO533 and Fibroblast Growth Factor Receptor

The protein at interest was allowed to interact in binding buffer (DMEM medium, 10 mM Hepes, pH 7.4. 0.1% albumin, 200 ng/ml heparin) at room temperature for I hour. Protein A Sepharose (Pharmacia) was added (0.01 ml) and binding continued for 30 minutes. Protein A Sepharose beads were collected and washed twice in binding buffer. Samples were then resolved by SDS PAGE under reducing conditions. Western blot analysis was conducted with anti-His antibody (Quiagen), anti-gD antibody 5B6, or anti-acidic FGF (R&D systems) according to manufacturers instructions and revealed with ECL (Amersham).

Figure 9 is a Western blot indicating the binding of PR0533 to FGF receptor 4. FGFI(A) or PRO533 (FGF- 19) expressed with either N-terminal gD epitope tag (B) or C-terminal His8 epitope tag (C) were tested for binding to receptor-Fc fusion proteins. Specific binding components are as indicated above lanes 1-8. Lane 9 contains FGF loaded directly onto the gel for comparison. Molecular weight markers are indicated on the left side of the gel for comparison.

Figure 10 is a Western blot indicating the dependence of PR0533 (FGF-19) binding on heparin. N-terminal gD-tagged PR0533 (FGF-19) was allowed to interact with FGFR4-Fc in the presence of the indicated concentrations of heparin.

### EXAMPLE 5

### Gene Amplification

This example shows that the PRO533-encoding genes are amplified in the genome of certain human lung, cancers. Amplification is associated with overexpression of the gene product, indicating that the PRO533 proteins are useful targets for therapeutic intervention in certain cancers such as lung and other cancers. Therapeutic agents may take the form of antagonists of PRO533-encoding genes, for example, murine-human chimeric, humanized or human antibodies against a PR0533 polypeptide.

The starting material for the screen was genomic DNA isolated from a variety cancers. The DNA is quantitated precisely, *e*.*g*. fluorometrically. As a negative control. DNA was isolated from the cells of ten normal healthy individuals which was pooled and used as assay controls for the gene copy in healthy individuals (not shown). The 5' nuclease assay (for example, TaqMan^{™}) and real-time quantitative PCR (for example. ABI Prizm 7700 Sequence Detection System^{™} (Perkin Elmer. Applied Biosystems Division. Foster City. CA)), were used to find genes potentially amplified in certain cancers. The results were used to determine whether the DNA encoding PR0533 is over-represented in any of the primary lung or colon cancers or cancer cell lines or breast cancer cell lines that were screened. The primary lung cancers were obtained from individuals with tumors of the type and stage as indicated in Table 1. An explanation of the abbreviations used for the designation of the primary tumors listed in Table 1 and the primary tumors and cell lines referred to throughout this example has been given hereinbefore.

The results of the Taqman^{™} are reported in delta (Δ) CT units. One unit corresponds I PCR cycle or approximately a 2-fold amplification relative to normal, two units corresponds to 4-fold, 3 units to 8-fold amplification and so on. Quantitation was obtained using primers and a Taqman^{™} fluorescent prove derived from the PRO533- which are most likely to contain unique nucleic acid sequences and which are least likely to have spliced out introns are preferred for the primer and probe derivation, e.g. 3-untranslated region. The sequences for the primers and probes (forward, reverse and probe) used for the PRO533 gene amplification were as follows:
DNA49435.tm.f
5'GGGACGTGCTTCTACAAGAACAG-3' (SEQ ID NO: 21)
DNA49435.tm.r
5'-CAGGCTTACAATGTTATGATCAGACA-3' (SEQ ID NO: 22)
DNA49:135.tm.p
5'-TATTCAGAGTTTTCCATTGGCAGTGCCAGTT-3' (SEQ ID NO: 23)

The 5' nuclease assay reaction is a fluorescent PCR-based technique which makes use of the 5' exonuclease activity of Taq DNA polymerase enzyme to monitor amplification in real time. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the TAQ DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

The 5' nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5' Nuclease assay data are initially expressed as Ct, or the threshold cycle. This is defined as the cycle at which the reporter signal accumulates above the background level of fluorescence. The ΔCt values are used as quantitative measurement of the relative number of starting copies of a particular target sequence in a nucleic acid sample when comparing cancer DNA results to normal human DNA results.

Table I describes the stage, T stage and N stage of various primary tumors which were used to screen the PR0533 compounds of the invention.

**Table 1**

| Primary Lung and Colon Tumor Profiles | | | |
|---|---|---|---|
| Primary Tumor | Stage | T Stage | N Stage |
| Human lung tumor SqCCA (SRCC724) [LT1] | IB | T1 | N1 |
| Human lung tumor NSCCa (SRCC725) [LT1a] | IA | T3 | N0 |
| Human lung tumor AdenoCa (SRCC726) [LT2] | IB | T2 | N0 |
| Human lung tumor AdenoCa (SRCC727) [LT3] | IB | T1 | N2 |
| Human lung tumor SqCCa (SRCC728) [LT4] | IIB | T2 | N0 |
| Human lung tumor AdenoCa (SRCC729) [LT6] | IV | T1 | N0 |
| Human lung tumor Adeno/SqCCa (SRCC730) [LT7] | IB | T1 | N0 |
| Human lung tumor AdenoCa (SRCC731) [LT9] | IIB | T2 | N0 |
| Human lung tumor SqCCa (SRCC732) [LT10] | IA | T2 | N1 |
| Human lung tumor AdenoCa (SRCC733) [LT11] | IB | T1 | N1 |
| Human lung tumor AdenoCa (SRCC734) [LT12] | IIA | T2 | N0 |
| Human lung tumor BAC (SRCC735) [LT13] | IB | T2 | N0 |
| Human lung tumor SqCCa (SRCC736) [LT15] | IB | T2 | N0 |
| Human lung tumor SqCCa (SRCC737) [LT16] | IB | T2 | N0 |
| Human lung tumor SqCCa (SRCC738) [LT17] | IIB | T2 | N1 |
| Human lung tumor SqCCa (SRCC739) [LT18] | IB | T2 | N0 |
| Human lung tumor SqCCa (SRCC740) [LT19] | IB | T2 | N0 |
| Human lung tumor LCCa (SRCC741) [LT21] | IIB | T3 | N1 |

### DNA Preparation:

DNA was prepared from cultured cell lines, primary tumors, normal human blood. The isolation was performed using purification kit, buffer set and protease and all from Quiagen, according to the manufacturer's instructions and the description below.

### Cell culture lysis:

Cells were washed and trypsinized at a concentration of 7.5 x 10⁸ per tip and pelleted by centrifuging at 1000 rpm for 5 minutes at 4°C. followed by washing again with 1/2 volume of PBS recentrifugation. The pellets were washed a third time, the suspended cells collected and washed 2x with PBS. The cells were then suspended into 10 mL PBS. Buffer CI was equilibrated at 4°C. Quiagen protease =19155 was diluted into 6.25 ml cold ddH₂0 to a final concentration of 20 mg/ml and equilibrated at 4°C. 10 mL of G2 Buffer was prepared by diluting Quiagen RNAse A stock (100 mg/ml) to a final concentration of 200 µg/ml.

Buffer C1 (10 mL. 4°C) and ddH2O (40 mL. 4°C) were then added to the 10 mL of cell suspension, mixed by inverting and incubated on ice for 10 minutes. The cell nuclei were pelleted by centrifuging in a Beckman swinging bucket rotor at 2500 rpm at 4°C for 15 minutes. The supernatant was discarded and the nuclei were suspended with a vortex into 2 mL Buffer C 1 (at 4°C) and 6 mL ddH₂O, followed by a second 4°C centrifugation at 2500 rpm for 15 minutes. The nuclei were then resuspended into the residual buffer using 200 µl per tip. G2 buffer (10 ml) was added to the suspended nuclei while gentle vortexing was applied. Upon completion of buffer addition, vigorous vortexing was applied for 30 seconds. Quiagen protease (200 µl, prepared as indicated above) was added and incubated at 50°C for 60 minutes. The incubation and centrifugation was repeated until the lysates were clear (*e*.*g*., incubating additional 30-60 minutes, pelleting at 3000 x g for 10 min., 4°C).

### Solid human tumor sample preparation and lysis:

Tumor samples were weighed and placed into 50 ml conical tubes and held on ice. Processing was limited to no more than 250 mg tissue per preparation (1 tip/preparation). The protease solution was freshly prepared by diluting into 6.25 ml cold ddH₂O to a final concentration of 20 mg/ml and stored at 4°C. G2 buffer (20 ml) was prepared by diluting DNAse A to a final concentration of 200 mg/ml (from 100 mg/ml stock). The tumor tissue was homogenated in 19 ml G2 buffer for 60 seconds using the large tip of the polytron in a laminar-flow TC hood to order to avoid inhalation of aerosols, and held at room temperature. Between samples, the polytron was cleaned by spinning at 2 x 30 seconds each in 2L ddH₂O, followed by G2 buffer (50 ml). If tissue was still present on the generator tip, the apparatus was disassembled and cleaned.

Quiagen protease (prepared as indicated above. 1.0 ml) was added, followed by vortexing and incubation at 50°C for 3 hours. The incubation and centrifugation was repeated until the lysates were clear (*e*.*g*., incubating additional 30-60 minutes, pelleting at 3000 x g for 10 min., 4°C).

### Human blood preparation and lysis:

Blood was drawn from healthy volunteers using standard infectious agent protocols and citrated into 10 ml samples per tip. Quiagen protease was freshly prepared by dilution into 6.25 ml cold ddH₂O to a final concentration of 20 mg/ml and stored at 4°C. G2 buffer was prepared by diluting RNAse A to a final concentration of 200 µg/ml from 100 mg/ml stock. The blood (10 ml) was placed into a 50 ml conical tube and 10 ml C I buffer and 30 ml ddH₂O (both previously equilibrated to 4°C) were added, and the components mixed by inverting and held on ice for 10 minutes. The nuclei were pelleted with a Beckman swinging bucket rotor at 2500 rpm. 4°C for 15 minutes and the supernatant discarded. With a vortex, the nuclei were suspended into 2 ml C I buffer (4°C) and 6 ml ddH₂O (4°C). Vortexing was repeated until the pellet was white. The nuclei were then suspended into the residual buffer using a 200 µl tip. G2 buffer (10 ml) were added to the suspended nuclei while gently vortexing, followed by vigorous vortexing for 30 seconds. Quiagen protease was added (200 µl) and incubated at 50°C for 60 minutes. The incubation and centrifugation was repeated until the lysates were clear (*e*.*g*., incubating additional 30-60 minutes, pelleting at 3000 x g for 10 min., 4°C).

### Purification of cleared lysates:

### (1) Isolation of genomic DNA:

Genomic DNA was equilibrated (1 sample per maxi tip preparation) with 10 ml QBT buffer. QF elution buffer was equilibrated at 50°C. The samples were vortexed for 30 seconds, then loaded onto equilibrated tips and drained by gravity. The tips were washed with 2 x 15 ml QC buffer. The DNA was eluted into 30 ml silanized, autoclaved 30 ml Corex tubes with 15 ml QF buffer (50°C). Isopropanol (10.5 ml) was added to each sample, the tubes covered with parafin and mixed by repeated inversion until the DNA precipitated. Samples were pelleted by centrifugation in the SS-34 rotor at 15,000 rpm for 10 minutes at 4°C. The pellet location was marked, the supernatant discarded, and 10 ml 70% ethanol (4°C) was added. Samples were pelleted again by centrifugation on the SS-34 rotor at 10,000 rpm for 10 minutes at 4 °C. The pellet location was marked and the supernatant discarded. The tubes were then placed on their side in a drying rack and dried 10 minutes at 37°C, taking care not to overdry the samples.

After drying, the pellets were dissolved into 1.0 ml TE (pH 8.5) and placed at 50°C for 1-2 hours. Samples were held overnight at 4°C as dissolution continued. The DNA solution was then transferred to 1.5 ml tubes with a 26 gauge needle on a tuberculin syringe. The transfer was repeated 5x in order to shear the DNA. Samples were then placed at 50°C for 1-2 hours.

### Quantitation of genomic DNA and preparation for gene amplification assay:

The DNA levels in each tube were quantified by standard A260, A280 spectrophotometry on a 1:20 dilution (5 µl DNA + 95 µl ddH₂O) using the 0.1 ml quartz cuvetts in the Beckman DU640 spectrophotometer. A260/A280 ratios were in the range of 1.8-1.9. Each DNA samples was then diluted further to approximately 200 ng/ml in TE (pH 8.5). If the original material was highly concentrated (about 700 ng/µl), the material was placed at 50°C for several hours until resuspended.

Fluorometric DNA quantitation was then performed on the diluted material (20-600 ng/ml) using the manufacturer's guidelines as modified below. This was accomplished by allowing a Hoeffer DyNA Quant 200 fluorometer to warm-up for about 15 minutes. The Hoechst dye working solution (#H33258, 10 µl, prepared within 12 hours of use) was diluted into 100 ml 1 x TNE buffer. A 2 ml cuvette was filled with the fluorometer solution, placed into the machine, and the machine was zeroed, pGEM 3Zf(+) (2 µl, lot #360851026) was added to 2 ml of fluorometer solution and calibrated at 200 units. An additional 2 µl of pGEM 3Zf(+) DNA was then tested and the reading confirmed at 400 +/- 10 units. Each sample was then read at least in triplicate. When 3 samples were found to be within 10% of each other, their average was taken and this value was used as the quantification value.

The fluorometricly determined concentration was then used to dilute each sample to 10 ng/µl in ddH₂O. This was done simultaneously on all template samples for a single TaqMan plate assay, and with enough material to run 500-1000 assays. The samples were tested in triplicate with Taqman^{™} primers and probe both B-actin and GAPDH on a single plate with normal human DNA and no-template controls. The diluted samples were used provided that the CT value of normal human DNA subtracted from test DNA was +/- I CT. The diluted, lot-qualified genomic DNA was stored in 1.0 ml aliquots at -80°C. Aliquots which were subsequently to be used in the gene amplification assay were stored at 4°C. Each I ml aliquot is enough for 8-9 plates or 64 tests.

### Gene amplification assay:

The PR0533 compounds of the invention were screened in the following primary tumors and the resulting ΔCt values are reported in Table 2.

**Table 2**

| ΔCt value for various lung primary tumor models of DNA49435 | |
|---|---|
| Primary Tumor | ΔCt value |
| LT1 | -0.05 |
| LT1a | 1.02 |
| LT2 | -0.17 |
| LT3 | 0.78 |
| LT4 | 0.14 |
| LT6 | -0.02 |
| LT7 | 1.04 |
| LT9 | 0.80 |
| LT10 | 0.79 |
| LT11 | 1.09 |
| LT12 | 0.76 |
| LT13 | 0.91 |
| LT15 | 0.50 |
| LT16 | 1.66 |
| LT17 | 1.32 |
| LT18 | 0.34 |
| LT19 | 1.67 |
| LT21 | 0.92 |

### Discussion and Conclusion:

The ΔCt values for DNA49435 (PRO533) in a variety of lung tumors are reported in Table 2. A ΔCt value of > 1 was typically used as the threshold value for amplification scoring, as this represents a doubling of the gene copy. Table 2 indicates that amplification of DNA49435 occurred in primary lung tumors LT1a, LT7. LT11, LT16, LT17 and LT19. The ΔCt values in these tumors were 1.02, 1.04, 1.09, 1.66, 1.32 and 1.67. This represents approximately a 2.0, 2.1, 2.1, 3.2. 2.5 and 3.2, respectively, fold increase in gene copy relative to normal tissue. Because amplification of DNA49435 (PRO533) occurs in various tumors, it is likely associated with tumor formation or growth. As a result, antagonists (*e*.*g*., antibodies) directed against the protein encoded by DNA49435 (PRO533) would be expected to be useful in cancer therapy.

### EXAMPLE 6

### Use of PR0533 as a hybridization probe

The following method describes use of a nucleotide sequence encoding PRO533 as a hybridization probe.

DNA comprising the coding sequence of full-length or mature PR0533 (as shown in Figure 1, SEQ ID NO: 1) is employed as a probe to screen for homologous DNAs (such as those encoding naturally-occurring variants of PR0533) in human tissue cDNA libraries or human tissue genomic libraries.

Hybridization and washing of filters containing either library DNAs is performed under the following high stringency conditions. Hybridization of radiolabeled PR0533-derived probe to the filters is performed in a solution of 50% formamide. 5x SSC, 0.1% SDS. 0.1% sodium pyrophosphate. 50 mM sodium phosphate, pH 6.8. 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO533 can then be identified using standard techniques known in the art.

### EXAMPLE 7

### Expression of PRO533 in E. coli

This example illustrates preparation of an unglycosylated form of PRO533 by recombinant expression in *E. coli.*

The DNA sequence encoding PR0533 (SEQ ID NO:1) is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli*; see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO533 coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO533 protein can then be purified using a metal chelating column under conditions that allow tight binding of the protein.

### EXAMPLE 8

### Expression of PRO533 in mammalian cells

This example illustrates preparation of a potentially glycosylated form of PRO533 by recombinant expression in mammalian cells.

The vector, pRK5 (see EP 307,247, published March 15, 1989), is employed as the expression vector. Optionally, the PR0533 DNA is ligated into pRK5 with selected restriction enzymes to allow insertion of the PRO533 DNA using ligation methods such as described in Sambrook et al., supra. The resulting vector is called pRK5-PRO533.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO533 DNA is mixed with about 1 µg DNA encoding the VA RNA gene [Thimmappaya et al., Cell, 31:543 (1982)] and dissolved in 500 µl of 1 mM Tris-HCI. 0.1 mM EDTA, 0.227 M CaCl₂. To this mixture is added, dropwise, 500 µl of 50 mM HEPES (pH 7.35), 280 mM NaCl. 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml "S-methionine. After a 12 hour incubation, the conditioned medium is collected, concentrated on a spin filter, and loaded onto a 15% SDS gel. The processed gel may be dried and exposed to film for a selected period of time to reveal the presence of PRO533 polypeptide. The cultures containing transfected cells may undergo further incubation (in serum free medium) and the medium is tested in selected bioassays.

In an alternative technique, PRO533 may be introduced into 293 cells transiently using the dextran sulfate method described by Somparyrac et al., Proc. Natl. Acad, Sci., 12: 7575 (1981). 293 cells are grown to maximal density in a spinner flask and 700 µg pRK5-PRO533 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium. 5 µg/ml bovine insulin and 0.1 µg/ml bovine transferrin. After about four days, the conditioned media is centrifuged and filtered to remove cells and debris. The sample containing expressed PRO533 can then be concentrated and purified by any selected method, such as dialysis and/or column chromatography.

The various FGFR-Fc fusion proteins described herein were expressed transiently in 293 cells in serum free medium and purfied over protein G column. DNA49435 was expressed transiently in 293 cells in serum free medium with the expression vector pRK-gD-FGF-19 as a fusion protein with the gD signal sequence and epitope tage and a genenase cleavage site
(MGGAAARLGAVILFVVIVGLHGVRGKYALADASLKMADPNRFRGKDL
PVLDQLLEGGAAHYALLPG) fused to the N-terminus.

In another embodiment, PRO533 can be expressed in CHO cells. The pRK5-PRO533 can be transfected into CHO cells using known reagents such as CaPO₄ or DEAE-dextran. As described above, the cell cultures can be incubated, and the medium replaced with culture medium (alone) or medium containing a radiolabel such as "S-methionine. After determining the presence of PRO533 polypeptide, the culture medium may be replaced with serum free medium. Preferably, the cultures are incubated for about 6 days, and then the conditioned medium is harvested. The medium containing the expressed PRO533 can then be concentrated and purified by any selected method.

Epitope-tagged PR0533 may also be expressed in host CHO cells. The PRO533 may be subcloned out of the pRK5 vector. The subclone insert can undergo PCR to fuse in frame with a selected epitope tag such as a poly-his tag into a Baculovirus expression vector. The poly-his tagged PRO533 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PR0533 can then be concentrated and purified by any selected method, such as by Ni²⁺-chelate affinity chromatography.

### EXAMPLE 9

### Expression of PRO533 in Yeast

The following method describes recombinant expression of PRO533 in yeast.

First, yeast expression vectors are constructed for intracellular production or secretion of PRO533 from the ADH2/GAPDH promoter. DNA encoding PR0533 and the promoter is inserted into suitable restriction enzyme sites in the selected plasmid to direct intracellular expression of PR0533. For secretion, DNA encoding PR0533 can be cloned into the selected plasmid, together with DNA encoding the ADH2/GAPDH promoter, a native PRO533 signal peptide or other mammalian signal peptide, or, for example, a yeast alpha-factor or invertase secretory signal/leader sequence, and linker sequences (if needed) for expression of PRO533.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PR0533 can subsequently be isolated and purified by removing the yeast cells from the fermentation medium by centrifugation and then concentrating the medium using selected cartridge filters. The concentrate containing PRO533 may further be purified using selected column chromatography resins.

### EXAMPLE 10

### Expression of PRO533 in Baculovirus-Infected Insect Cells

The following method describes recombinant expression of PR0533 in Baculovirus-infected insect cells.

The sequence coding for PRO533 is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO533 or the desired portion of the coding sequence of PRO533 [such as the sequence encoding the extracellular domain of a transmembrane protein or the sequence encoding the mature protein if the protein is extracellular] is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PR0533 can then be purified, for example, by Ni²⁺-chelate affinity chromatography as follows. Extracts are prepared from recombinant virus-infected Sf9 cells as described by Rupert et al., Nature, 362:175-179(1993). Briefly, Sf9 cells are washed, resuspended in sonication buffer (25 mL Hepes, pH 7.9; 12.5 mM MgCl₂; 0.1 mM EDTA; 10% glycerol: 0.1% NP-40: 0.4 M KCI), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PR0533 are pooled and dialyzed against loading buffer.

Alternatively, purification of the IgG tagged (or Fc tagged) PRO533 can be performed using known chromatography techniques, including for instance, Protein A or protein G column chromatography.

PR0533 (UNQ334) were expressed in baculovirus infected Sf9 insect cells. While the expression was actually performed in a 0.5-2 L scale, it can be readily scaled up for larger (e.g. 8 L) preparations. PRO533 may expressed as an IgG construct (immunoadhesin), in which the protein extracellular region was fused to an IgGI constant region sequence containing the hinge, CH2 and CH3 domains and/or in poly-His tagged forms. DNA49435 was expressed in His-tagged form by inclusion of the C terminal extension GHHHHHHHH.

Following PCR amplification, the coding sequence was subcloned into a baculovirus expression vector (pb.PH.His.c), and the vector and Baculogold^{®} baculovirus DNA (Pharmingen) were co-transfected into 105 *Spodoptera frugiperda* ("Sf9") cells (ATCC CRL 1711), using Lipofectin (Gibco BRL), pb.PH.His is a modification of the commercially available baculovirus expression vector pVL1393 (Pharmingen), with modified polylinker regions to include the His tag sequence. The cells were grown in Hink's TNM-FH medium supplemented with 10% FBS (Hyclone). Cells were incubated for 5 days at 28°C. The supernatant was harvested and subsequently used for the first viral amplification by infecting Sf9 cells in Hink's TNM-FH medium supplemented with 10% FBS at an approximate multiplicity of infection (MOI) of 10. Cells were incubated for 3 days at 28°C. The supernatant was harvested and the expression of the constructs in the baculovirus expression vector was determined by batch binding of I ml of supernatant to 25 mL of Ni-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The first viral amplification supernatant was used to infect a spinner culture (500 ml) of Sf9 cells grown in ESF-921 medium (Expression Systems LLC) at an approximate MOl of 0.1. Cells were incubated for 3 days at 28°C. The supernatant was harvested and filtered. Batch binding and SDS-PAGE analysis was repeated, as necessary, until expression of the spinner culture was confirmed.

The conditioned medium from the transfected cells (0.5 to 3 L) was harvested by centrifugation to remove the cells and filtered through 0.22 micron filters. For the poly-His tagged constructs, the protein construct were purified using a Ni-NTA column (Qiagen). Before purification, imidazole was added to the conditioned media to a concentration of 5 mM. The conditioned media were pumped onto a 6 ml Ni-NTA column equilibrated in 20 mM Hepes, pH 7.4, buffer containing 0.3 M NaCl and 5 mM imidazole at a flow rate of 4-5 ml/min, at 4°C. After loading, the column was washed with additional equilibration buffer and the protein eluted with equilibration buffer containing 0.25 M imidazole. The highly purified protein was subsequently desalted into a storage buffer containing 10 mM Hepes, 0.14 M NaCl and 4% mannitol, pH 6.8, with a 25 ml G25 Superfine (Pharmacia) column and stored at -80°C.

### EXAMPLE 11

### Demonstration of binding of PRO533 (UNQ334) to FGF Receptor 4

PR0533 was expressed in baculovirus in a C-terminal His8 epitope tagged form as described in Example 8, as was a control C-terminal His8 epitope protein. The extracellular domains of FGF receptors 1-4 and TIE1 receptor were expressed as Fc fusion proteins. Proteins were allowed to interact in binding buffer (DMEM media + 10mM Hepes pH 7.4 + 0.1% albumin + 200 ng/ml heparin) at room temperature for one hour. Protein A Sepharose (Pharmacia) was added (0.01 ml) and binding continued for 30 minutes. Protein A Sepharose beads were collected and washed twice in binding buffer. Samples were then resolved by SDS PAGE under reducing conditions. Western blot analysis was conducted with anti-His antibody (Qiagen) as recommended by manufacturer. The results are shown in Figure 9. The specific binding components are as indicated above lanes 1-8 in Figure 9. Lane 9 contains PR0533-His (UNQ334-His) loaded directly onto gel for comparison. The position of the molecular weight markers is indicated on the left side of the gel for comparison.

The results demonstrate a high specificity binding to FGF Receptor 4 (FGFR4-Fc). This is very significant, since most FGF ligands bind more than one FGF receptor.

### EXAMPLE 12

### Preparation of Antibodies that Bind PRO533

This example illustrates preparation of monoclonal antibodies which can specifically bind PRO533.

Techniques for producing the monoclonal antibodies are known in the art and are described, for instance, in Goding, supra. Immunogens that may be employed include purified PRO533, fusion proteins containing PRO533, and cells expressing recombinant PRO533 on the cell surface. Selection of the immunogen can be made by the skilled artisan without undue experimentation.

Mice, such as Balb/c, are immunized with the PR0533 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PR0533 antibodies.

After a suitable antibody titer has been detected, the animals "positive" for antibodies can be injected with a final intravenous injection of PRO533. Three to four days later, the mice are sacrificed and the spleen cells are harvested. The spleen.cells are then fused (using 35% polyethylene glycol) to a selected murine myeloma cell line such as P3X63AgU.I. available from ATCC. No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO533. Determination of "positive" hybridoma cells secreting the desired monoclonal antibodies against PRO533 is within the skill in the art.

The positive hybridoma cells can be injected intraperitoneally into syngeneic Balb/c mice to produce ascites containing the anti-PRO533 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### Deposit of Material

The following materials have been deposited with the American Type Culture Collection, 10801 University Blvd., Manassas, VA 20110-2209, USA (ATCC):

| Material | ATCC Dep. No. | Deposit Date |
|---|---|---|
| DNA49435-1219 | 209480 | November 21, 1997 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and other constructs that are functionally equivalent may be within the scope of this invention as defined in the claims. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications in addition to those shown and described herein will become apparent to those skilled in the art from the foregoing description and fall within the scope of the appended claims.

## Claims

1. An isolated nucleic acid molecule comprising
(a) a DNA molecule which encodes either:
(i) a polypeptide which has the sequence of amino acid residues from 1 or 23 to 216 of Figure 1 (SEQ ID NO:1), or
(ii) a polypeptide which is a variant of the polypeptide of (i), has at least an 80% sequence identity to a FGF-19 polypeptide having the sequence of amino acid residues from 23 to 216 of Figure 1 (SEQ ID NO: 1), and binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms); or
(b) the complement of the DNA molecule of (a).

2. The isolated nucleic acid molecule of claim 1 comprising the sequence of nucleotide positions from 464-466 to 1109-1111 of Figure 2 (SEQ ID NO: 2).

3. The isolated nucleic acid molecule of claim 1 comprising a DNA molecule encoding (a) a FGF 19 polypeptide having the sequence of amino acid residues from 1 to 216 of Figure 1 (SEQ ID NO: 1), or (b) the complement of the DNA molecule of (a).

4. An isolated nucleic acid molecule encoding a FGF-19 polypeptide, comprising DNA hybridizing under stringent conditions to the complement of the nucleic acid having the sequence of nucleotide positions from 464-466 to 1109-1111 of Figure 2 (SEQ ID NO: 2), which DNA encodes a polypeptide which binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms).

5. An isolated nucleic acid molecule comprising
(a) a DNA molecule which encodes either:
(i) the same mature polypeptide encoded by the human protein cDNA in ATCC Deposit No.209480 (DNA49435-1219, or
(ii) a polypeptide which is a variant of the polypeptide of (i), which has at least an 80% sequence identity to the amino acid sequence of the mature polypeptide encoded by the human protein cDNA in ATCC Deposit No.209480 (Designation: DNA49435-1219), and binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms); or
(b) a DNA molecule which is the complement of the DNA molecule of (a).

6. The isolated nucleic acid molecule of claim 5 comprising DNA encoding the same mature polypeptide encoded by the human protein cDNA in ATCC Deposit No.209480 (DNA49435-1219).

7. The isolated nucleic acid molecule of claim 1 comprising (a) DNA encoding a polypeptide having the sequence of amino acid residues from 23 to 216 of Figure 1 (SEQ ID NO: 1), or (b) the complement of the DNA of (a).

8. An isolated nucleic acid molecule comprising (a) DNA encoding a polypeptide other than the polypeptide of claim 1(a) (i), which scores at least 80% positives according to the BLOSUM62 matrix when compared to the sequence of amino acid residues from about 23 to about 216 of Figure 1 (SEQ ID NO: 1), and binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms), or (b) the complement of the DNA of (a).

9. An isolated nucleic acid molecule having at least 20-80 nucleotides which encodes a polypeptide other than the polypeptide of claim 1(a)(i), which binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms), and is produced by hybridizing a test DNA molecule under stringent conditions with (a) a FGF-19 encoding DNA molecule having the sequence of nucleic acid residues from 1 to 826 and 1199 to of Figure 2 (SEQ ID NO: 2), or (b) the complement of the DNA molecule of (a), and, if the test DNA molecule has at least an 80% sequence identity to (a) or (b), isolating the test DNA molecule.

10. A vector comprising the nucleic acid of any one of claims 1 to 9.

11. The vector of Claim 10 operably linked to control sequences recognized by a host cell transformed with the vector.

12. A host cell comprising the vector of Claim 11.

13. The host cell of Claim 12, wherein said cell is a CHO cell.

14. The host cell of Claim 12, wherein said cell is an *E. coli.*

15. The host cell of Claim 12, wherein said cell is a yeast cell.

16. A process for producing a FGF-19 polypeptide comprising culturing the host cell of any one of claims 12 to 15 under conditions suitable for expression of said FGF-19 polypeptide and recovering said FGF-19 polypeptide from the cell culture.

17. An isolated FGF-19 polypeptide encoded by the DNA of any one of claims 1 to 9.

18. The isolated polypeptide of claim 17 comprising amino acid residues from 23 to 216 of Figure 1 (SEQ ID NO: 1).

19. An isolated FGF-19 polypeptide of claim 17 scoring at least 80% positives according to the BLOSUM62 matrix when compared to the sequence of amino acid residues from 23 to 216 of Figure 1 (SEQ ID NO: 1) and which binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms).

20. An isolated FGF-19 polypeptide comprising the sequence of amino acid residues from 23 to 216 of Figure 1 (SEQ ID NO: 1), or a fragment thereof sufficient to provide a binding site for an anti-FGF-19 antibody and which binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms).

21. An isolated FGF-19 polypeptide encoded by the cDNA insert of the vector deposited as ATCC Deposit No.209480 (DNA49435-1219).

22. An isolated polypeptide which binds to the extracellular domain (ECD) of FGFR4 in the presence of heparin, but not to the ECD of FGFR1 (IIIc splice form), FGFR2 (IIIb or IIIc splice forms), or FGFR3 (IIIb or IIIc splice forms), produced by (i) hybridizing a test DNA molecule under stringent conditions with (a) a DNA molecule encoding FGF-19 polypeptides having the sequence of amino acid residues from 23 to 216 of Figure 1 (SEQ ID NO: 1), or (b) the complement of the DNA molecule of (a), and, if said test DNA molecule has at least an 80% sequence identity to (a) or (b), (ii) culturing a host cell comprising said test DNA molecule under conditions suitable for the expression of said polypeptide, and (iii) recovering said polypeptide from the cell culture.

23. A chimeric molecule comprising a FGF-19 polypeptide according to any one of claims 17 to 22 fused to a heterologous amino acid sequence.

24. The chimeric molecule of Claim 23, wherein said heterologous amino acid sequence is an epitope tag sequence.

25. The chimeric molecule of Claim 23, wherein said heterologous amino acid sequence is a Fc region of an immunoglobulin.

26. An antibody which specifically binds to a FGF-19 polypeptide or a fragment thereof as defined in any one of claims 18 and 20 to 22.

27. The antibody of Claim 26, wherein said antibody is a monoclonal antibody.

28. The antibody of claim 26 or claim 27, which antibody is an antibody fragment.

29. The antibody of any one of claims 26 to 28, which antibody is a human or humanised antibody.

30. A method of determining the presence of a FGF-19 polypeptide as defined in any one of claims 17 to 22 comprising exposing a cell suspected of containing a FGF-19 polypeptide to an anti-FGF-19 antibody as defined in any one of claims 26 to 29 and determining binding of antibody to the cell.

31. An anti-FGF-19 antibody as defined in any one of claims 26 to 29 for use in a method of medical treatment.

## Patentansprüche

1. Isoliertes Nucleinsäuremolekül, umfassend
(a) ein DNA-Molekül, das für:
(i) ein Polypeptid, das die Sequenz von Aminosäureresten von 1 oder 23 bis 216 aus Fig. 1 (Seq.-ID Nr. 1) aufweist, oder
(ii) ein Polypeptid, das eine Variante des Polypeptids aus (i) ist, zumindest 80 % Sequenzidentität mit einem FGF-19-Polypeptid mit der Sequenz der Aminosäurereste 23 bis 216 aus Fig. 1 (Seq.-ID Nr. 1) aufweist und sich an die extrazelluläre Domäne (ECD) von FGFR4 in Gegenwart von Heparin bindet, aber nicht an die ECD von FGFR1 (IIIc-Spleißform), FGFR2 (IIIb- oder IIIc-Spleißformen) oder FGFR3 (IIIb- oder IIIc-Spleißformen),
kodiert; oder
(b) das Komplement des DNA-Moleküls aus (a).

2. Isoliertes Nucleinsäuremolekül nach Anspruch 1, umfassend die Sequenz der Nucleotidpositionen von 464-466 bis 1109-1111 aus Fig. 2 (Seq.-ID Nr. 2).

3. Isoliertes Nucleinsäuremolekül nach Anspruch 1, umfassend ein DNA-Molekül, das für (a) ein FGF-19-Polypeptid mit der Sequenz der Aminosäurereste von 1 bis 216 aus Fig. 1 (Seq.-ID Nr. 1) kodiert, oder (b) das Komplement des DNA-Moleküls aus (a).

4. Isoliertes Nucleinsäuremolekül, das für ein FGF-19-Polypeptid kodiert, umfassend DNA, die unter stringenten Bedingungen an das Komplement der Nucleinsäure mit der Sequenz der Nucleotidpositionen von 464-466 bis 1109-1111 aus Fig. 2 (Seq.-ID Nr. 2) hybridisiert, wobei die DNA für ein Polypeptid kodiert, das sich an die extrazelluläre Domäne (ECD) von FGFR4 in Gegenwart von Heparin bindet, aber nicht an die ECD von FGFR1 (IIIc-Spleißform), FGFR2 (IIIb- oder IIIc- Spleißformen) oder FGFR3 (IIIb- oder IIIc-Spleißformen).

5. Isoliertes Nucleinsäuremolekül, umfassend
(a) ein DNA-Molekül, das für:
(i) dasselbe reife Polypeptid, für welches die menschliche Protein-cDNA in ATCC-Hinterlegungsnummer 209480 (DNA-49435-1219) kodiert, oder
(ii) ein Polypeptid, das eine Variante des Polypeptids aus (i) ist, die zumindest 80 % Sequenzidentität mit der Aminosäuresequenz des reifen Polypeptids aufweist, für welches die menschliche Protein-cDNA in ATCC-Hinterlegungsnummer 209480 (Benennung: DNA49435-1219) kodiert, und sich an die extrazelluläre Domäne (ECD) von FGFR4 in der Gegenwart von Heparin bindet, aber nicht an die ECD von FGFR1 (IIIc-Spleißform), FGFR2 (IIIb- oder IIIc-Spleißformen) oder FGFR3 (IIIb- oder IIIc-Spleißformen),
kodiert; oder
(b) ein DNA-Molekül, das das Komplement des DNA-Moleküls aus (a) ist.

6. Isoliertes Nucleinsäuremolekül nach Anspruch 5, umfassend DNA, die für dasselbe reife Polypeptid kodiert, für welches die reife Protein-cDNA in ATCC-Hinterlegungsnummer 209480 (DNA49435-1219) kodiert.

7. Isoliertes Nucleinsäuremolekül nach Anspruch 1, umfassend (a) DNA, die für ein Polypeptid mit der Sequenz von Aminosäureresten 23 bis 216 aus Fig. 1 (Seq.-ID Nr. 1) kodiert, oder (b) das Komplement der DNA von (a).

8. Isoliertes Nucleinsäuremolekül, umfassend (a) DNA, die für ein Polypeptid kodiert, das ein anderes ist als das Polypeptid aus Anspruch 1(a)(i), das zumindest 80 % Positive gemäß der BLOSUM62-Matrix verzeichnet, wenn es mit der Sequenz der Aminosäurereste aus etwa 23 bis etwa 216 von Fig. 1 (Seq.-ID Nr.1) verglichen wird, und sich an die extrazelluläre Domäne (ECD) von FGFR4 in Gegenwart von Heparin bindet, aber nicht an die ECD von FGFR1 (IIIc-Spleißform), FGFR2 (IIIb- oder IIIc-Spleißformen) oder FGFR3 (IIIb- oder IIIc-Spleißformen), oder (b) das Komplement der DNA von (a).

9. Isoliertes Nucleinsäuremolekül mit zumindest 20-80 Nucleotiden, das für ein Polypeptid kodiert, das kein Polypeptid nach Anspruch 1(a)(i) ist, das sich an die extrazelluläre Domäne (ECD) von FGFR4 in Gegenwart von Heparin bindet, aber nicht an die ECD von FGFR1 (IIIc-Spleißform), FGFR2 (IIIb- oder IIIc-Spleißformen) oder FGFR3 (IIIb- oder IIIc-Spleißformen), und durch Hybridisierung eines Test-DNA-Moleküls unter stringenten Bedingungen mit (a) einem für FGF-19 kodierenden DNA-Molekül mit der Sequenz der Nucleinsäurereste von 1 bis 826 und 1199 bis aus Fig. 2 (Seq.-ID Nr. 2) oder (b) dem Komplement des DNA-Moleküls von (a) und, wenn das Test-DNA-Molekül zumindest 80 % Sequenzidentität mit (a) oder (b) aufweist, Isolation des Test-DNA-Moleküls hergestellt wird.

10. Vektor, der die Nucleinsäure nach einem der Ansprüche 1 bis 9 umfasst.

11. Vektor nach Anspruch 10, der operabel an die Kontrollsequenzen gebunden ist, die von einer Wirtszelle erkannt werden, die mit dem Vektor transformiert ist.

12. Wirtszelle, die den Vektor nach Anspruch 11 umfasst.

13. Wirtszelle nach Anspruch 12, worin die Wirtszelle eine CHO-Zelle ist.

14. Wirtszelle nach Anspruch 12, worin die Zelle eine E.-coli-Zelle ist.

15. Wirtszelle nach Anspruch 12, worin die Zelle eine Hefezelle ist.

16. Verfahren zur Herstellung eines FGF-19-Polypeptids, das die Kultivierung der Wirtszelle nach einem der Ansprüche 12 bis 15 unter zur Expression des FGF-19-Polypeptids geeigneten Bedingungen und die Gewinnung des FGF-19-Polypeptids aus der Zellkultur umfasst.

17. Isoliertes FGF-19-Polypeptid, für welches die DNA nach einem der Ansprüche 1 bis 9 kodiert.

18. Isoliertes Polypeptid nach Anspruch 17, umfassend die Aminosäurereste von 23 bis 216 aus Fig. 1 (Seq.-ID Nr. 1).

19. Isoliertes FGF-19-Polypeptid nach Anspruch 17, das zumindest 80 % Positive gemäß der BLOSUM62-Matrix verzeichnet, wenn es mit der Sequenz der Aminosäurereste von 23 bis 216 aus Fig. 1 (Seq.-ID Nr. 1) verglichen wird, und sich an die extrazelluläre Domäne (ECD) von FGFR4 in Gegenwart von Heparin bindet, aber nicht an die ECD von FGFR1 (IIIc-Spleißform), FGFR2 (IIIb- oder IIIc-Spleißformen) oder FGFR3 (IIIb- oder IIIc-Spleißformen).

20. Isoliertes FGF19-Polypeptid, umfassend die Sequenz von Aminosäureresten 23 bis 216 aus Fig. 1 (Seq.-ID Nr. 1), oder ein Fragment davon, das ausreicht, um eine Bindungsstelle für einen Anti-FGF-19-Antikörper bereitzustellen, und sich an die extrazelluläre Domäne (ECD) von FGFR4 in Gegenwart von Heparin bindet, aber nicht an die ECD von FGFR1 (IIIC-Spleißform), FGFR2 (IIIb- oder IIIc-Spleißformen) oder FGFR3 (IIIb- oder IIIc-Spleißformen).

21. Isoliertes FGF-19-Polypeptid, für welches das cDNA-Insert des Vektors kodiert, das als ATCC-Hinterlegungs-Nr. 209480 (DNA49435-1219) hinterlegt ist.

22. Isoliertes Polypeptid, das sich an die extrazelluläre Domäne (ECD) von FGFR4 in Gegenwart von Heparin bindet, aber nicht an die ECD von FGFR1 (IIIc-Spleißform), FGFR2 (IIIb- oder IIIc-Spleißformen) oder FGFR3 (IIIb- oder IIIc-Spleißformen), produziert durch (i) Hybridisierung eines Test-DNA-Moleküls unter stringenten Bedingungen mit (a) einem DNA-Molekül, das für FGF-19-Polypeptid mit der Sequenz von Aminosäureresten 23 bis 216 aus Fig. 1 (Seq.-ID Nr. 1) kodiert, oder (b) dem Komplement des DNA-Moleküls aus (a) und, wenn das Test-DNA-Molekül zumindest 80 % Sequenzidentität mit (a) oder (b) aufweist, (ii) Kultivierung einer Wirtszelle, die das DNA-Testmolekül umfasst, unter Bedingungen, die für die Expression des Polypeptids geeignet sind, und (iii) Gewinnung des Polypeptids aus der Zellkultur.

23. Chimäres Molekül, das ein FGF-19-Polypeptid nach einem der Ansprüche 17 bis 22, fusioniert an eine heterologe Aminosäuresequenz, umfasst.

24. Chimäres Molekül nach Anspruch 23, worin die heterologe Aminosäuresequenz eine Epitopmarkierungssequenz ist.

25. Chimäres Molekül nach Anspruch 23, worin die heterologe Aminosäuresequenz eine Fc-Region eines Immunglobulins ist.

26. Antikörper, der sich spezifisch an ein FGF-19-Polypeptid oder ein Fragment davon, wie in einem der Ansprüche 18 und 20 bis 22 definiert, bindet.

27. Antikörper nach Anspruch 26, worin der Antikörper ein monoklonaler Antikörper ist.

28. Antikörper nach Anspruch 26 oder 27, wobei der Antikörper ein Antikörperfragment ist.

29. Antikörper nach einem der Ansprüche 26 bis 28, wobei der Antikörper ein menschlicher oder humanisierter Antikörper ist.

30. Verfahren zur Bestimmung der Gegenwart eines FGF-19-Polypeptids wie in einem der Ansprüche 17 bis 22 definiert, umfassend das Aussetzen einer Zelle, von der vermutet wird, ein FGF-19-Polypeptid zu enthalten, gegenüber einem Anti-FGF-19-Antikörper, wie in einem der Ansprüche 26 bis 29 definiert, und Bestimmung der Bindung des Antikörpers an die Zelle.

31. Anti-FGF-19-Antikörper, wie in einem der Ansprüche 26 bis 29 definiert, zur Verwendung in einem Verfahren zur medizinischen Behandlung.

## Revendications

1. Molécule d'acide nucléique isolée, comprenant
(a) une molécule d'ADN qui code pour soit :
(i) un polypeptide qui a la séquence de résidus d'aminoacides de 1 ou 23 à 216 de la figure 1 (SEQ ID N° 1), soit
(ii) un polypeptide qui est un variant du polypeptide de (i), qui présente une identité de séquence d'au moins 80 % avec un polypeptide FGF-19 ayant la séquence de résidus d'aminoacides de 23 à 216 de la figure 1 (SEQ ID N° 1), et qui se lie au domaine extracellulaire (ECD) de FGFR4 en présence d'héparine, mais non au ECD de FGFR1 (forme à épissage IIIc), de FGFR2 (formes à épissage IIIb ou IIIc) ou de FGFR3 (formes à épissage IIIb ou IIIc) ; ou
(b) le complément de la molécule d'ADN de (a).

2. Molécule d'acide nucléique isolée suivant la revendication 1, comprenant la séquence des positions de nucléotides 464-466 à 1109-1111 de la figure 2 (SEQ ID N° 2).

3. Molécule d'acide nucléique isolée suivant la revendication 1, comprenant une molécule d'ADN codant pour (a) un polypeptide FGF-19 ayant la séquence de résidus d'aminoacides de 1 à 216 de la figure 1 (SEQ ID N° 1), ou (b) le complément de la molécule d'ADN de (a).

4. Molécule d'acide nucléique isolée codant pour un polypeptide FGF-19, comprenant un ADN s'hybridant dans des conditions stringentes au complément de l'acide nucléique ayant la séquence des positions de nucléotides 464-466 à 1109-1111 de la figure 2 (SEQ ID N° 2), l'ADN qui code pour un polypeptide qui se lie au domaine extracellulaire (ECD) de FGFR4 en présence d'héparine, mais non au ECD de FGFR1 (forme à épissage IIIc), de FGFR2 (formes à épissage IIIb ou IIIc) ou FGFR3 (formes à épissage IIIb ou IIIc).

5. Molécule d'acide nucléique isolée, comprenant
(a) une molécule d'ADN qui code pour soit :
(i) le même polypeptide mature codé par l'ADNc de protéine humaine dans le dépôt ATCC N° 209480 (DNA49435-1219), soit
(ii) un polypeptide qui est un variant du polypeptide de (i), qui présente une identité de séquence d'au moins 80 % avec la séquence d'aminoacides du polypeptide mature codé par l'ADNc de protéine humaine dans le dépôt ATCC N° 209480 (désignation : DNA49435-1219), et qui se lie au domaine extracellulaire (ECD) de FGFR4 en présence d'héparine, mais non au ECD de FGFR1 (forme à épissage IIIc), de FGFR2 (formes à épissage IIIb ou IIIc) ou de FGFR3 (formes à épissage IIIb ou IIIc), ou
(b) une molécule d'ADN qui est le complément de la molécule d'ADN de (a).

6. Molécule d'acide nucléique isolée suivant la revendication 5, comprenant un ADN codant pour le même polypeptide mature codé par l'ADNc de protéine humaine dans le dépôt ATCC N° 209480 (DNA49435-1219).

7. Molécule d'acide nucléique isolée suivant la revendication 1, comprenant (a) l'ADN codant pour un polypeptide ayant la séquence de résidus d'aminoacides de 23 à 216 de la figure 1 (SEQ ID N° 1), ou (b) le complément de l'ADN de (a).

8. Molécule d'acide nucléique isolée comprenant (a) un ADN codant pour un polypeptide autre que le polypeptide de la revendication 1(a)(i), qui a une valeur numérique d'évaluation d'au moins 80 % de positifs d'après la matrice BLOSUM62 lors de la comparaison avec la séquence de résidus d'aminoacides d'environ 23 à environ 216 de la figure (SEQ ID N° 1), et qui se lie au domaine extracellulaire (ECD) de FGFR4 en présence d'héparine, mais non au ECD de FGFR1 (forme à épissage IIIc), de FGFR2 (formes à épissage IIIb ou IIIc) ou de FGFR3 (formes à épissage IIIb ou IIIc), ou (b) le complément de l'ADN de (a).

9. Molécule d'acide nucléique isolée comprenant au moins 20 à 80 nucléotides qui code pour un polypeptide autre que le polypeptide de la revendication 1(a)(i), qui se lie au domaine extracellulaire (ECD) de FGFR4 en présence d'héparine, mais non au ECD de FGFR1 (forme à épissage IIIc), de FGFR2 (formes à épissage IIIb ou IIIc) ou de FGFR3 (formes à épissage IIIb ou IIIc), et qui est produit par hybridation d'une molécule d'ADN d'essai dans des conditions stringentes avec (a) une molécule d'ADN codant pour FGF-19 ayant la séquence de résidus d'acide nucléique de 1 à 826 et 1199 de la figure 2 (SEQ ID N° 2) ou (b) le complément de la molécule d'ADN de (a) et, si la molécule d'ADN d'essai présente une identité de séquence d'au moins 80 % avec (a) ou (b), par isolement de la molécule d'ADN d'essai.

10. Vecteur comprenant l'acide nucléique de l'une quelconque des revendications 1 à 9.

11. Vecteur suivant la revendication 10, lié de manière fonctionnelle à des séquences de régulation reconnues par une cellule hôte transformée avec le vecteur.

12. Cellule hôte comprenant le vecteur de la revendication 11.

13. Cellule hôte suivant la revendication 12, ladite cellule étant une cellule CHO.

14. Cellule hôte suivant la revendication 12, ladite cellule étant une cellule de *E. coli*.

15. Cellule hôte suivant la revendication 12, ladite cellule étant une cellule de levure.

16. Procédé pour la production d'un polypeptide FGF-19, comprenant la culture de la cellule hôte de l'une quelconque des revendications 12 à 15, dans des conditions convenables pour l'expression dudit polypeptide FGF-19 et la récupération dudit polypeptide FGF-19 à partir de la culture cellulaire.

17. Polypeptide FGF-19 isolé codé par l'ADN de l'une quelconque des revendications 1 à 9.

18. Polypeptide isolé suivant la revendication 17, comprenant les résidus d'aminoacides 23 à 216 de la figure 1 (SEQ ID N° 1).

19. Polypeptide FGF-19 isolé suivant la revendication 17, ayant une valeur numérique d'évaluation d'au moins 80 % de positifs d'après la matrice BLOSUM62 lors de la comparaison avec la séquence de résidus d'aminoacides 23 à 216 de la figure 1 (SEQ ID N° 1) et qui se lie au domaine extracellulaire (ECD) de FGFR4 en présence d'héparine, mais non au ECD de FGFR1 (forme à épissage IIIc), de FGFR2 (formes à épissage IIIb ou IIIc) ou de FGFR3 (formes à épissage IIIb ou IIIc).

20. Polypeptide FGF-19 isolé comprenant la séquence de résidus d'aminoacides 23 à 216 de la figure 1 (SEQ ID N° 1), ou un de ses fragments suffisants pour fournir un site de liaison pour un anticorps anti-FGF-19 et qui se lie au domaine extracellulaire (ECD) de FGFR4 en présence d'héparine, mais non au ECD de FGFR1 (forme à épissage IIIc), de FGFR2 (formes à épissage IIIb ou IIIc) ou de FGFR3 (formes à épissage IIIb ou IIIc).

21. Polypeptide FGF-19 isolé codé par le segment d'insertion d'ADNc du vecteur déposé en tant que dépôt ATCC N° 209480 (DNA49435-1219).

22. Polypeptide isolé qui se lie au domaine extracellulaire (ECD) de FGFR4 en présence d'héparine, mais non au ECD de FGFR1 (forme à épissage IIIc), de FGFR2 (formes à épissage IIIb ou IIIc) ou de FGFR3 (formes à épissage IIIb ou IIIc), produit (i) en hybridant une molécule d'ADN d'essai dans des conditions stringentes avec (a) une molécule d'ADN codant pour des polypeptides FGF-19 ayant la séquence de résidus d'aminoacides 23 à 216 de la figure 1 (SEQ ID N° 1), ou (b) le complément de la molécule d'ADN de (a), et, si ladite molécule d'ADN d'essai présente une identité de séquence d'au moins 80 % avec (a) ou (b), (ii) en cultivant une cellule hôte comprenant ladite molécule d'ADN d'essai dans des conditions convenables pour l'expression dudit polypeptide, et (iii) en recueillant ledit polypeptide à partir de la culture cellulaire.

23. Molécule chimère comprenant un polypeptide FGF-19 suivant l'une quelconque des revendications 17 à 22, fusionné à une séquence d'aminoacides hétérologue.

24. Molécule chimère suivant la revendication 23, dans laquelle ladite séquence d'aminoacides hétérologue est une séquence de marqueur épitopique.

25. Molécule chimère suivant la revendication 23, dans laquelle ladite séquence d'aminoacides hétérologue est une région Fc d'une immunoglobuline.

26. Anticorps qui se lie spécifiquement à un polypeptide FGF-19 ou à un de ses fragments tels que définis dans l'une quelconque des revendications 18 et 20 à 22.

27. Anticorps suivant la revendication 26, ledit anticorps étant un anticorps monoclonal.

28. Anticorps suivant la revendication 26 ou la revendication 27, anticorps qui est un fragment d'anticorps.

29. Anticorps suivant l'une quelconque des revendications 26 à 28, anticorps qui est un anticorps humain ou humanisé.

30. Méthode pour déterminer la présence d'un polypeptide FGF-19 tel que défini dans l'une quelconque des revendications 17 à 22, comprenant l'exposition d'une cellule supposée contenir un polypeptide FGF-19 à un anticorps anti-FGF-19 tel que défini dans l'une quelconque des revendications 26 à 29, et la détermination de la liaison de l'anticorps à la cellule.

31. Anticorps anti-FGF-19 tel que défini dans l'une quelconque des revendications 26 à 29, destiné à être utilisé dans une méthode de traitement médical.
